# EUROPEAN PATENT APPLICATION

(11) **EP 4 786 497 A1**
(43) Date of publication of application: **05.08.2026**
(21) Application number: 24872128.4
(22) Date of filing: 24.09.2024
(51) Int. Cl.: C07K 16/28, A61K 39/395, A61P 3/00, A61P 25/00, A61P 27/02, A61P 43/00, C07K 16/46, C12N 1/15, C12N 1/19, C12N 1/21, C12N 5/10, C12N 15/13, C12N 15/63, C12P 21/08

(54) **ANTI-MERTK AGONIST ANTIBODY, ANTI-MERTK BISPECIFIC ANTIBODY, AND USE THEREOF**

(30) Priority: 25.09.2023 JP 2023160157; 22.12.2023 JP 2023216438
(71) Applicant: Astellas Pharma Inc., Tokyo 103-8411 (JP)
(72) Inventor: NAKAMURA, Naoto, Tokyo 103-8411 (JP); KUBO, Satoshi, Tokyo 103-8411 (JP); SAKAMOTO, Koji, Tokyo 103-8411 (JP); TOMINAGA, Hiroaki, Tokyo 103-8411 (JP); FUJITA, Hirotada, Tokyo 103-8411 (JP); IWASAKI, Masashi, Tokyo 103-8411 (JP); SUZUKI, Kozue, Tokyo 103-8411 (JP); NAGASHIMA, Akira, Tokyo 103-8411 (JP)
(74) Representative: Hoffmann Eitle
(86) International application number: PCT/JP2024/033807
(87) International publication number: WO 2025/070337

(57) **Abstract**

An object of the present invention is to provide an anti-MerTK antibody and an anti-MerTK bispecific antibody that can be used in the prevention or the treatment of diseases related to phagocytosis. The present inventors have obtained a novel anti-MerTK agonist antibody by searching for an anti-MerTK agonist antibody that binds to MerTK and activates downstream signals. This anti-MerTK agonist antibody activated the phagocytic clearance function of RPE cells by activating downstream signals via MerTK expressed on RPE cell surface. Furthermore, an anti-MerTK-anti-misfolded protein bispecific antibody prepared on the basis of the sequence of the anti-MerTK agonist antibody activated the phagocytic clearance function of macrophages for a misfolded protein. Thus, the anti-MerTK antibody or the anti-MerTK-anti-misfolded protein bispecific antibody is expected to be used in the prevention or the treatment of diseases related to phagocytosis.

## Description

### TECHNICAL FIELD

The present invention relates to a novel anti-MerTK agonist antibody, an anti-MerTK bispecific antibody, and use thereof.

### BACKGROUND ART

The mechanism of rapid phagocytic clearance of apoptotic cells, cell-derived debris, and the like by phagocytes such as macrophages is called efferocytosis and recognized as a very important mechanism for the maintenance of tissue or immune homeostasis (Nat. Rev. Mol. Cell Biol., 2020, 21: 398-414). For example, at a lesion of arteriosclerosis, macrophages contribute to the suppression of inflammatory reaction or the suppression of progression of the disease by recognizing and rapidly clearing apoptotic cells of the self (Nature, 2016, 536: 86-90).

Mer tyrosine kinase (MerTK), a member of TAM (Tyro3, Axl and MerTK) receptor tyrosine kinase family, is expressed by phagocytes such as macrophages and known to function as a major molecule of efferocytosis. MerTK is a single-pass transmembrane protein extracellularly having two immunoglobulin-like domains and two fibronectin III-type domains and intracellularly having one tyrosine kinase domain, and growth arrest specific gene 6 (Gas6), protein S (ProS), tubby and tubby-like protein 1 (TULP-1), and the like have been identified as its ligands. Among them, Gas6 and ProS are ligands that play a role in binding so as to bridge phosphatidylserine (PtdSer) exposed on the surface of apoptotic cells and the immunoglobulin-like domains of MerTK on phagocytes. MerTK binds to the apoptotic cells via these ligands and transduces activation signals (tyrosine autophosphorylation) necessary for efferocytosis or the like to downstream sites (Nat. Rev. Mol. Cell Biol., 2020, 21: 398-414; Cold Spring Harb. Perspect. Biol., 2013, 5: a009076; and EMBO J., 2010, 29: 3898-3910). It has been further suggested that MerTK promotes an anti-inflammatory function through the suppression of Toll-like receptor-mediated inflammatory cytokine production of dendritic cells (Cell, 2007, 131: 1124-1136) or the induction of synthesis of specialized pro-resolving mediators from macrophages (Sci. Signal., 2018, 11: eaar3721).

MerTK is also expressed on retinal pigment epithelium (RPE) cells. Owing to MerTK-mediated activation of intracellular downstream signals, the RPE cells phagocytize photoreceptor outer segment (POS) debris desquamated as a waste. The RPE cells are positioned so as to come in contact with POS. PtdSer is exposed on the end of POS, and the RPE cells phagocytically clear POS by recognizing PtdSer via MerTK and its ligands. This phagocytic function is very important for the homeostatic and functional maintenance of photoreceptor cells and the retina (Front. Immunol., 2020, 11: 604205).

Retinitis pigmentosa is a genetic disease that is caused by a mutation in a gene influencing the function or homeostasis of photoreceptor cells or RPE, and 80 or more causative genes have been identified so far. A majority of the causative genes are attributed to photoreceptor cells, and mutations therein induce cell death, leading to the degeneration of retinal tissues. Furthermore, dead cells or their debris induces an inflammatory condition and aggravates symptoms. The possibility is suggested that rapid phagocytic clearance of dead cells or debris by the functional activation of MerTK can treat retinitis pigmentosa (Prog. Retin. Eye Res., 2017, 63: 107-131; Adv. Exp. Med. Biol., 2023, 1415: 365-370; and PNAS Nexus, 2022, 1: pgac003).

Gene therapy for causative genes and treatment methods for suppressing retinal disorder by use of pharmacological preparations such as anti-angiogenic agents and complement inhibitors are under development as methods for treating debris accumulation-derived retinal degradation (e.g., retinitis pigmentosa or age-related macular degeneration) (Prog. Retin. Eye Res., 2018, 63: 107-130). Nonetheless, no satisfactory treatment method has yet been attained, and there is a further demand for drug discovery research and development.

For MerTK to be activated, tyrosine autophosphorylation of the intracellular kinase domain needs to be induced. The possibility has been suggested that an anti-MerTK antibody screened for by using the phosphorylation of the intracellular kinase domain of MerTK and its downstream molecule AKT as an indicator could treat retinal degradation by activating MerTK-mediated intracellular downstream signal transduction and removing debris (PTL 4). Although the presence of an anti-MerTK antibody that activates phagocytosis via MerTK on macrophages has been suggested so far (PTLs 1 to 4 and NPL 1), no anti-MerTK antibody that actually exhibits the activation of phagocytosis of RPE cells has been reported.

A bispecific antibody that binds to MerTK and a material to be phagocytized has been studied in recent years for the purpose of activating efficient phagocytic clearance of the material to be phagocytized by putting phagocytes and the material to be phagocytized physically close to each other. For example, a bispecific antibody that binds to MerTK and CD20, a bispecific antibody that binds to MerTK and amyloid β (Aβ) (NPL 2), and a bispecific antibody that binds to MerTK and epidermal growth factor receptor (EGFR) serving as a tumor associated antigen (TAA) on cancer cells (NPL 3) have been reported so far, and the bispecific antibodies have been reported to be capable of activating a MerTK-mediated phagocytic clearance function.

Transthyretin (TTR) is a protein with a molecular weight of 14 kDa composed of 127 amino acids. Although a main production location of TTR is the liver, it is also produced from the choroid plexus of the ventricle, retinal pigment epithelium cells of the retina, pancreatic α cells, and the like. TTR assumes a stable tetramer structure in blood and functions as a transport carrier for a vitamin A-retinol binding protein complex and thyroid hormone T4, mainly, in blood and spinal fluid (PTL 5). TTR is one of the causative proteins in systemic amyloidosis caused by genetic mutations or aging, and deposition of misfolded TTR in organs throughout the body is known to cause dysfunction. The possibility has been suggested that an antibody against misfolded TTR activates the phagocytosis of misfolded TTR by phagocytes via an Fc region and inhibits the deposition of the misfolded TTR (Amyloid, 2016, 23: 86-97). However, in the phase I clinical trial on NI006, a monoclonal anti-TTR IgG1 antibody that binds to misfolded TTR, in addition to the inhibitory action on the deposition of misfolded TTR, the event of cytokine release syndrome has been reported in several test patients (NPL 4). The development of additional drugs for treating systemic amyloidosis such as TTR amyloidosis is required.

### CITATION LIST

### PATENT LITERATURE

PTL 1: International Publication No. WO 2016/106221
PTL 2: International Publication No. WO 2019/005756
PTL 3: International Publication No. WO 2020/176497
PTL 4: International Publication No. WO 2021/202590
PTL 5: International Publication No. WO 2015/115332

### NON PATENT LITERATURE

NPL 1: PLoS ONE, 2015, 10: e0145078
NPL 2: mAbs, 2020, 12: 1685832
NPL 3: Int. J. Mol. Sci., 2022, 23: 15673
NPL 4: N. Engl. J. Med., 2023, 389: 239-250

### SUMMARY OF INVENTION

### TECHNICAL PROBLEM

An object of the present invention is to provide an anti-MerTK agonist antibody and an anti-MerTK bispecific antibody that can be used in the prevention or the treatment of diseases related to phagocytosis.

### SOLUTION TO PROBLEM

The present inventors have conducted considerable diligent studies in the preparation of an anti-MerTK antibody that binds to MerTK and activates the phagocytic clearance function of phagocytes via MerTK, and consequently obtained a novel anti-MerTK agonist antibody tA3-20 (Example 1). tA3-20 activated the phagocytic clearance function of RPE cells *in vitro* (Example 2) and further activated the phosphorylation of the intracellular kinase domain of MerTK and phosphorylation signals for its downstream AKT in RPE *in vivo* (Example 3). Furthermore, an anti-MerTK-anti-TTR bispecific antibody containing a one-armed antibody containing the heavy chain variable region and the light chain variable region of tA3-20 activated MerTK-mediated phagocytosis of misfolded TTR (Example 6) and further markedly activated the phagocytic clearance function for the target in senile mice (Example 7). An anti-MerTK-anti-immunoglobulin light chain (IgL) bispecific antibody containing a one-armed antibody containing the heavy chain variable region and the light chain variable region of tA3-20 bound to human MerTK and misfolded IgL in an antibody concentration-dependent manner and activated the phagocytic clearance function (Example 10). Thus, the present inventors have completed the present invention by providing an anti-MerTK agonist antibody and an anti-MerTK bispecific antibody that bind to MerTK and activate a MerTK-mediated phagocytic clearance function of phagocytes.

Specifically, the present invention relates to, but not limited to, the following [1] to [86].
[1] An anti-MerTK antibody containing
   a heavy chain variable region containing CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
   a light chain variable region containing CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6,
   or an antigen binding fragment thereof.
[2] The anti-MerTK antibody according to [1], containing a heavy chain variable region and a light chain variable region selected from the group consisting of the following (1) to (6):
   (1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
   (2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
   (3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
   (4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
   (5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
   (6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20,
   or an antigen binding fragment thereof.
[3] The anti-MerTK antibody according to [1] or [2], containing a heavy chain containing the heavy chain variable region and a light chain containing the light chain variable region.
[4] The anti-MerTK antibody according to [3], containing L234A and L235A amino acid mutations (LALA mutations) in the heavy chain (wherein positions of the mutations are amino acid positions that are numbered according to the EU index in a human Igγ1 constant region).
[5] The anti-MerTK antibody according to [3], containing a P331G mutation in the heavy chain (wherein a position of the mutation is an amino acid position that is numbered according to the EU index in a human Igγ1 constant region).
[6] The anti-MerTK antibody according to [3], containing L234A and L235A amino acid mutations (LALA mutations) and a P331G mutation in the heavy chain (wherein positions of the mutations are amino acid positions that are numbered according to the EU index in a human Igγ1 constant region).
[7] The anti-MerTK antibody according to [6] selected from the group consisting of the following (1) to (6):
   (1) an anti-MerTK antibody containing a heavy chain consisting of the amino acid sequence of SEQ ID NO: 8, and a light chain consisting of the amino acid sequence of SEQ ID NO: 10;
   (2) an anti-MerTK antibody containing a heavy chain consisting of the amino acid sequence of SEQ ID NO: 8, and a light chain consisting of the amino acid sequence of SEQ ID NO: 12;
   (3) an anti-MerTK antibody containing a heavy chain consisting of the amino acid sequence of SEQ ID NO: 8, and a light chain consisting of the amino acid sequence of SEQ ID NO: 14;
   (4) an anti-MerTK antibody containing a heavy chain consisting of the amino acid sequence of SEQ ID NO: 8, and a light chain consisting of the amino acid sequence of SEQ ID NO: 16;
   (5) an anti-MerTK antibody containing a heavy chain consisting of the amino acid sequence of SEQ ID NO: 8, and a light chain consisting of the amino acid sequence of SEQ ID NO: 18; and
   (6) an anti-MerTK antibody containing a heavy chain consisting of the amino acid sequence of SEQ ID NO: 8, and a light chain consisting of the amino acid sequence of SEQ ID NO: 20.
[8] The anti-MerTK antibody according to any of [1] to [7] which is an IgG antibody (anti-MerTK IgG antibody).
[9] The anti-MerTK antibody or the antigen binding fragment thereof according to [1] or [2], wherein the antigen binding fragment is a single-chain variable fragment (scFv), a Fab fragment, a Fab' fragment, or a F(ab')₂ fragment.
[10] The anti-MerTK antibody or the antigen binding fragment thereof according to any of [1] to [9], wherein the anti-MerTK antibody or an antigen-binding fragment thereof is modified post-translationally.
[11] A fusion product or a complex of the anti-MerTK antibody or the antigen binding fragment thereof according to any of [1] to [10], or a cell allowed to express the anti-MerTK antibody or the antigen binding fragment thereof according to any of [1] to [10] on the cell surface.
[12] A polynucleotide containing a nucleotide sequence encoding a heavy chain variable region or a light chain variable region of an anti-MerTK antibody or an antigen binding fragment thereof, the polynucleotide being selected from the group consisting of the following (1) to (7):
   (1) a polynucleotide containing a nucleotide sequence encoding a heavy chain variable region consisting of an amino acid sequence from amino acid positions 1 to 119 of SEQ ID NO: 8;
   (2) a polynucleotide containing a nucleotide sequence encoding a light chain variable region consisting of an amino acid sequence from amino acid positions 1 to 109 of SEQ ID NO: 10;
   (3) a polynucleotide containing a nucleotide sequence encoding a light chain variable region consisting of an amino acid sequence from amino acid positions 1 to 109 of SEQ ID NO: 12;
   (4) a polynucleotide containing a nucleotide sequence encoding a light chain variable region consisting of an amino acid sequence from amino acid positions 1 to 109 of SEQ ID NO: 14;
   (5) a polynucleotide containing a nucleotide sequence encoding a light chain variable region consisting of an amino acid sequence from amino acid positions 1 to 110 of SEQ ID NO: 16;
   (6) a polynucleotide containing a nucleotide sequence encoding a light chain variable region consisting of an amino acid sequence from amino acid positions 1 to 110 of SEQ ID NO: 18; and
   (7) a polynucleotide containing a nucleotide sequence encoding a light chain variable region consisting of an amino acid sequence from amino acid positions 1 to 110 of SEQ ID NO: 20.
[13] A polynucleotide containing a nucleotide sequence encoding a heavy chain or a light chain of an anti-MerTK antibody, the polynucleotide being selected from the group consisting of the following (1) to (7):
   (1) a polynucleotide containing a nucleotide sequence encoding a heavy chain consisting of the amino acid sequence of SEQ ID NO: 8;
   (2) a polynucleotide containing a nucleotide sequence encoding a light chain consisting of the amino acid sequence of SEQ ID NO: 10;
   (3) a polynucleotide containing a nucleotide sequence encoding a light chain consisting of the amino acid sequence of SEQ ID NO: 12;
   (4) a polynucleotide containing a nucleotide sequence encoding a light chain consisting of the amino acid sequence of SEQ ID NO: 14;
   (5) a polynucleotide containing a nucleotide sequence encoding a light chain consisting of the amino acid sequence of SEQ ID NO: 16;
   (6) a polynucleotide containing a nucleotide sequence encoding a light chain consisting of the amino acid sequence of SEQ ID NO: 18; and
   (7) a polynucleotide containing a nucleotide sequence encoding a light chain consisting of the amino acid sequence of SEQ ID NO: 20.
[14] An expression vector containing the polynucleotide according to [12] or [13].
[15] A host cell transformed with the expression vector according to [14].
[16] A host cell containing a polynucleotide selected from the group consisting of the following (1) to (13):
   (1) a polynucleotide containing a nucleotide sequence encoding a heavy chain variable region consisting of an amino acid sequence from amino acid positions 1 to 119 of SEQ ID NO: 8;
   (2) a polynucleotide containing a nucleotide sequence encoding a light chain variable region consisting of an amino acid sequence from amino acid positions 1 to 109 of SEQ ID NO: 10;
   (3) a polynucleotide containing a nucleotide sequence encoding a light chain variable region consisting of an amino acid sequence from amino acid positions 1 to 109 of SEQ ID NO: 12;
   (4) a polynucleotide containing a nucleotide sequence encoding a light chain variable region consisting of an amino acid sequence from amino acid positions 1 to 109 of SEQ ID NO: 14;
   (5) a polynucleotide containing a nucleotide sequence encoding a light chain variable region consisting of an amino acid sequence from amino acid positions 1 to 110 of SEQ ID NO: 16;
   (6) a polynucleotide containing a nucleotide sequence encoding a light chain variable region consisting of an amino acid sequence from amino acid positions 1 to 110 of SEQ ID NO: 18;
   (7) a polynucleotide containing a nucleotide sequence encoding a light chain variable region consisting of an amino acid sequence from amino acid positions 1 to 110 of SEQ ID NO: 20;
   (8) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 109 of SEQ ID NO: 10;
   (9) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 109 of SEQ ID NO: 12;
   (10) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 109 of SEQ ID NO: 14;
   (11) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 110 of SEQ ID NO: 16;
   (12) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 110 of SEQ ID NO: 18; and
   (13) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 110 of SEQ ID NO: 20.
[17] A method for producing an anti-MerTK antibody or an antigen binding fragment thereof, containing the step of culturing the host cell according to [15] or [16] so that the anti-MerTK antibody or the antigen binding fragment thereof is expressed.
[18] A pharmaceutical composition containing the anti-MerTK antibody or the antigen binding fragment thereof according to any of [1] to [10], and a pharmaceutically acceptable excipient.
[19] The pharmaceutical composition according to [18] for use in the prevention and/or the treatment of an eye disease.
[20] The pharmaceutical composition according to [19], wherein the eye disease is a retinal disease.
[21] The pharmaceutical composition according to [20], wherein the retinal disease is a retinal degenerative disease.
[22] The pharmaceutical composition according to [21], wherein the retinal degenerative disease is retinitis pigmentosa or age-related macular degeneration.
[23] The anti-MerTK antibody or the antigen binding fragment thereof according to any of [1] to [10] for use in the prevention and/or the treatment of an eye disease.
[24] A method for preventing and/or treating an eye disease, containing the step of administering a therapeutically effective amount of the anti-MerTK antibody or the antigen binding fragment thereof according to any of [1] to [10] to a subject.
[25] Use of the anti-MerTK antibody or the antigen binding fragment thereof according to any of [1] to [10] in the production of a pharmaceutical composition for the prevention and/or the treatment of an eye disease.
[26] A bispecific antibody that binds to MerTK and a misfolded protein and/or amyloid, containing a heavy chain variable region and a light chain variable region of an anti-MerTK antibody, and a heavy chain variable region and a light chain variable region of an antibody that binds to the misfolded protein and/or the amyloid, wherein
   the heavy chain variable region of the anti-MerTK antibody contains CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
   the light chain variable region of the anti-MerTK antibody contains CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6.
[27] The bispecific antibody according to [26], wherein the heavy chain variable region and the light chain variable region of the anti-MerTK antibody are selected from the group consisting of the following (1) to (6):
   (1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
   (2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
   (3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
   (4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
   (5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
   (6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20.
[28] The bispecific antibody according to [27], containing a one-armed antibody (one-armed anti-MerTK antibody) containing a heavy chain fragment containing the heavy chain variable region of the anti-MerTK antibody and a light chain containing the light chain variable region of the anti-MerTK antibody, and one Fc region consisting of a first Fc polypeptide and a second Fc polypeptide.
[29] The bispecific antibody according to [28], wherein the heavy chain fragment and the light chain of the one-armed anti-MerTK antibody are selected from the group consisting of the following (1) to (6):
   (1) a heavy chain fragment consisting of an amino acid sequence from amino acid positions 1 to 217 of SEQ ID NO: 8, and a light chain consisting of the amino acid sequence of SEQ ID NO: 10;
   (2) a heavy chain fragment consisting of an amino acid sequence from amino acid positions 1 to 217 of SEQ ID NO: 8, and a light chain consisting of the amino acid sequence of SEQ ID NO: 12;
   (3) a heavy chain fragment consisting of an amino acid sequence from amino acid positions 1 to 217 of SEQ ID NO: 8, and a light chain consisting of the amino acid sequence of SEQ ID NO: 14;
   (4) a heavy chain fragment consisting of an amino acid sequence from amino acid positions 1 to 217 of SEQ ID NO: 8, and a light chain consisting of the amino acid sequence of SEQ ID NO: 16;
   (5) a heavy chain fragment consisting of an amino acid sequence from amino acid positions 1 to 217 of SEQ ID NO: 8 and a light chain consisting of the amino acid sequence of SEQ ID NO: 18; and
   (6) a heavy chain fragment consisting of an amino acid sequence from amino acid positions 1 to 217 of SEQ ID NO: 8, and a light chain consisting of the amino acid sequence of SEQ ID NO: 20.
[30] The bispecific antibody according to [29], wherein the C terminus of scFv or a Fab region containing the heavy chain variable region and the light chain variable region of the antibody that binds to the misfolded protein and/or the amyloid is linked via a hinge region to the N terminus of the second Fc polypeptide of the one-armed anti-MerTK antibody.
[31] The bispecific antibody according to any of [26] to [30], wherein the antibody that binds to the misfolded protein and/or the amyloid is an anti-TTR antibody that binds to misfolded TTR.
[32] The bispecific antibody according to [31], wherein
   the heavy chain variable region of the anti-TTR antibody contains CDR1 consisting of the amino acid sequence of SEQ ID NO: 27, CDR2 consisting of the amino acid sequence of SEQ ID NO: 28, and CDR3 consisting of the amino acid sequence of SEQ ID NO: 29, and
   the light chain variable region of the anti-TTR antibody contains CDR1 consisting of the amino acid sequence of SEQ ID NO: 30, CDR2 consisting of the amino acid sequence of SEQ ID NO: 31, and CDR3 consisting of the amino acid sequence of SEQ ID NO: 32.
[33] The bispecific antibody according to [32], wherein
   the heavy chain variable region of the anti-TTR antibody is a heavy chain variable region consisting of an amino acid sequence from amino acid positions 1 to 119 of SEQ ID NO: 43, and
   the light chain variable region of the anti-TTR antibody is a light chain variable region consisting of an amino acid sequence from amino acid positions 135 to 245 of SEQ ID NO: 43.
[34] The bispecific antibody according to [33], containing scFv containing the heavy chain variable region and the light chain variable region of the anti-TTR antibody (anti-TTR-scFv).
[35] The bispecific antibody according to [34], wherein the anti-TTR-scFv consists of the amino acid sequence of SEQ ID NO: 43.
[36] The bispecific antibody according to [34] or [35], containing a one-armed antibody (one-armed anti-MerTK antibody) containing a heavy chain fragment containing the heavy chain variable region of the anti-MerTK antibody and a light chain containing the light chain variable region of the anti-MerTK antibody, and one Fc region consisting of a first Fc polypeptide and a second Fc polypeptide, wherein the C terminus of the anti-TTR-scFv is linked via a hinge region to the N terminus of the second Fc polypeptide of the one-armed anti-MerTK antibody.
[37] The bispecific antibody according to any of [28] to [36], containing an Fc region containing L234A and L235A amino acid mutations (LALA mutations) and a P331G mutation (wherein positions of the mutations are amino acid positions that are numbered according to the EU index in a human Igγ1 constant region).
[38] The bispecific antibody according to [37], wherein the first Fc polypeptide and the second Fc polypeptide contain an amino acid sequence having at least 90% identity to the amino acid sequence of SEQ ID NO: 39 or an amino acid sequence differing from the amino acid sequence of SEQ ID NO: 39 by one to ten substitutions.
[39] The bispecific antibody according to [38], containing an Fc region containing knobs-into-holes mutations.
[40] The bispecific antibody according to [38], containing an Fc region containing LALA mutations, a P331G mutation, and knobs-into-holes mutations.
[41] The bispecific antibody according to [39] or [40], wherein the knobs-into-holes mutations are a T366W mutation in one of the Fc polypeptides constituting the Fc region, and T366S, L368A, and Y407V mutations in the other Fc polypeptide constituting the Fc region (wherein positions of the mutations are amino acid positions that are numbered according to the EU index in a human Igγ1 constant region).
[42] The bispecific antibody according to [38], wherein the sequences of the first Fc polypeptide and the second Fc polypeptide are polypeptides consisting of any sequence of the following (1) or (2):
   (1) a first Fc polypeptide consisting of the amino acid sequence of SEQ ID NO: 40, and a second Fc polypeptide consisting of the amino acid sequence of SEQ ID NO: 41; or
   (2) a first Fc polypeptide consisting of the amino acid sequence of SEQ ID NO: 41, and a second Fc polypeptide consisting of the amino acid sequence of SEQ ID NO: 40.
[43] The bispecific antibody according to [42], containing a hinge region consisting of the amino acid sequence represented by SEQ ID NO: 42 and/or a hinge region consisting of the amino acid sequence represented by SEQ ID NO: 70.
[44] The bispecific antibody according to any of [26] to [43], wherein the bispecific antibody is modified post-translationally.
[45] A fusion product or a complex of the bispecific antibody according to any of [26] to [44], or a cell allowed to express the bispecific antibody according to any of [26] to [44] on the cell surface.
[46] A polynucleotide for use in the production of the bispecific antibody according to any of [26] to [44], the polynucleotide being selected from the group consisting of the following (1) to (9):
   (1) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8;
   (2) a polynucleotide containing a nucleotide sequence encoding a light chain variable region of an anti-MerTK antibody, the light chain variable region consisting of an amino acid sequence from amino acid positions 1 to 109 of SEQ ID NO: 10;
   (3) a polynucleotide containing a nucleotide sequence encoding a light chain variable region of an anti-MerTK antibody, the light chain variable region consisting of an amino acid sequence from amino acid positions 1 to 109 of SEQ ID NO: 12;
   (4) a polynucleotide containing a nucleotide sequence encoding a light chain variable region of an anti-MerTK antibody, the light chain variable region consisting of an amino acid sequence from amino acid positions 1 to 109 of SEQ ID NO: 14;
   (5) a polynucleotide containing a nucleotide sequence encoding a light chain variable region of an anti-MerTK antibody, the light chain variable region consisting of an amino acid sequence from amino acid positions 1 to 110 of SEQ ID NO: 16;
   (6) a polynucleotide containing a nucleotide sequence encoding a light chain variable region of an anti-MerTK antibody, the light chain variable region consisting of an amino acid sequence from amino acid positions 1 to 110 of SEQ ID NO: 18;
   (7) a polynucleotide containing a nucleotide sequence encoding a light chain variable region of an anti-MerTK antibody, the light chain variable region consisting of an amino acid sequence from amino acid positions 1 to 110 of SEQ ID NO: 20;
   (8) a polynucleotide containing a nucleotide sequence encoding a heavy chain variable region of an anti-TTR antibody, the heavy chain variable region consisting of an amino acid sequence from amino acid positions 1 to 119 of SEQ ID NO: 43; and
   (9) a polynucleotide containing a nucleotide sequence encoding a light chain variable region of an anti-TTR antibody, the light chain variable region consisting of an amino acid sequence from amino acid positions 135 to 245 of SEQ ID NO: 43.
[47] An expression vector containing the polynucleotide according to [46].
[48] A host cell transformed with the expression vector according to [47].
[49] A host cell for use in the production of the bispecific antibody according to any of [27] to [44], the host cell containing polynucleotides selected from the group consisting of the following (1) to (3):
   (1) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8, a polynucleotide containing a nucleotide sequence encoding a light chain variable region of an anti-MerTK antibody, the light chain variable region consisting of an amino acid sequence from amino acid positions 1 to 110 of SEQ ID NO: 16, a polynucleotide containing a nucleotide sequence encoding a heavy chain variable region of an anti-TTR antibody, the heavy chain variable region consisting of an amino acid sequence from amino acid positions 1 to 119 of SEQ ID NO: 43, and a polynucleotide containing a nucleotide sequence encoding a light chain variable region of an anti-TTR antibody, the light chain variable region consisting of an amino acid sequence from amino acid positions 135 to 245 of SEQ ID NO: 43;
   (2) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8, a polynucleotide containing a nucleotide sequence encoding a light chain variable region of an anti-MerTK antibody, the light chain variable region consisting of an amino acid sequence from amino acid positions 1 to 110 of SEQ ID NO: 18, a polynucleotide containing a nucleotide sequence encoding a heavy chain variable region of an anti-TTR antibody, the heavy chain variable region consisting of an amino acid sequence from amino acid positions 1 to 119 of SEQ ID NO: 43, and a polynucleotide containing a nucleotide sequence encoding a light chain variable region of an anti-TTR antibody, the light chain variable region consisting of an amino acid sequence from amino acid positions 135 to 245 of SEQ ID NO: 43; and
   (3) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8, a polynucleotide containing a nucleotide sequence encoding a light chain variable region of an anti-MerTK antibody, the light chain variable region consisting of an amino acid sequence from amino acid positions 1 to 110 of SEQ ID NO: 20, a polynucleotide containing a nucleotide sequence encoding a heavy chain variable region of an anti-TTR antibody, the heavy chain variable region consisting of an amino acid sequence from amino acid positions 1 to 119 of SEQ ID NO: 43, and a polynucleotide containing a nucleotide sequence encoding a light chain variable region of an anti-TTR antibody, the light chain variable region consisting of an amino acid sequence from amino acid positions 135 to 245 of SEQ ID NO: 43.
[50] A method for producing a bispecific antibody that binds to MerTK and TTR, containing the step of culturing the host cell according to [48] or [49] so that the bispecific antibody is expressed.
[51] A pharmaceutical composition containing the bispecific antibody according to any of [26] to [44], and a pharmaceutically acceptable excipient.
[52] The pharmaceutical composition according to [51] for use in the prevention and/or the treatment of diseases derived from the accumulation of a misfolded protein.
[53] The pharmaceutical composition according to [52], wherein the disease derived from the accumulation of a misfolded protein is amyloidosis.
[54] The pharmaceutical composition according to [53], wherein the amyloidosis is systemic amyloidosis.
[55] The pharmaceutical composition according to [54], wherein the systemic amyloidosis is TTR amyloidosis or amyloid polyneuropathy.
[56] The bispecific antibody according to any of [26] to [44] for use in the prevention and/or the treatment of diseases derived from the accumulation of a misfolded protein.
[57] A method for preventing and/or treating a disease derived from the accumulation of a misfolded protein, containing the step of administering a therapeutically effective amount of the bispecific antibody according to any of [26] to [44] to a subject.
[58] Use of the bispecific antibody according to any of [26] to [44] in the production of a pharmaceutical composition for the prevention and/or the treatment of diseases derived from the accumulation of a misfolded protein.
[59] The bispecific antibody according to any of [26] to [30], wherein the antibody that binds to the misfolded protein and/or the amyloid is an anti-IgL antibody that binds to a misfolded IgL.
[60] The bispecific antibody according to [59], wherein
   the heavy chain variable region of the anti-IgL antibody contains CDR1 consisting of an amino acid sequence from amino acid positions 26 to 35 of SEQ ID NO: 57, CDR2 consisting of an amino acid sequence from amino acid positions 50 to 68 of SEQ ID NO: 57, and CDR3 consisting of an amino acid sequence from amino acid positions 101 to 108 of SEQ ID NO: 57, and
   the light chain variable region of the anti-IgL antibody contains CDR1 consisting of an amino acid sequence from amino acid positions 24 to 39 of SEQ ID NO: 59, CDR2 consisting of an amino acid sequence from amino acid positions 55 to 61 of SEQ ID NO: 59, and CDR3 consisting of an amino acid sequence from amino acid positions 94 to 103 of SEQ ID NO: 59.
[61] The bispecific antibody according to [60], wherein
   the heavy chain variable region of the anti-IgL antibody is a heavy chain variable region consisting of an amino acid sequence from amino acid positions 1 to 119 of SEQ ID NO: 57, and
   the light chain variable region of the anti-IgL antibody is a light chain variable region consisting of an amino acid sequence from amino acid positions 1 to 112 of SEQ ID NO: 59.
[62] The bispecific antibody according to [61], containing a Fab fragment containing the heavy chain variable region and the light chain variable region of the anti-IgL antibody (anti-IgL-Fab).
[63] The bispecific antibody according to [62], wherein the anti-IgL-Fab contains a fragment that consists of a heavy chain variable region (VH) consisting of an amino acid sequence from amino acid positions 1 to 226 of SEQ ID NO: 57 and a light chain constant region (CL), and a fragment that consists of a light chain variable region (VL) consisting of the amino acid sequence of SEQ ID NO: 59 and a CH1 domain.
[64] The bispecific antibody according to [62] or [63], containing a one-armed antibody (one-armed anti-MerTK antibody) containing a heavy chain fragment containing the heavy chain variable region of the anti-MerTK antibody and a light chain containing the light chain variable region of the anti-MerTK antibody, and one Fc region consisting of a first Fc polypeptide and a second Fc polypeptide, wherein the C terminus of the anti-IgL-Fab is linked via a hinge region to the N terminus of the second Fc polypeptide of the one-armed anti-MerTK antibody.
[65] The bispecific antibody according to any of [59] to [64], containing an Fc region containing L234A and L235A amino acid mutations (LALA mutations) and a P331G mutation (wherein positions of the mutations are amino acid positions that are numbered according to the EU index in a human Igγ1 constant region).
[66] The bispecific antibody according to [65], wherein the first Fc polypeptide and the second Fc polypeptide contain an amino acid sequence having at least 90% identity to the amino acid sequence of SEQ ID NO: 39 or an amino acid sequence differing from the amino acid sequence of SEQ ID NO: 39 by one to ten substitutions.
[67] The bispecific antibody according to [66], containing an Fc region containing knobs-into-holes mutations.
[68] The bispecific antibody according to [66], containing an Fc region containing LALA mutations, a P331G mutation, and knobs-into-holes mutations.
[69] The bispecific antibody according to [67] or [68], wherein the knobs-into-holes mutations are a T366W mutation in one of the Fc polypeptides constituting the Fc region, and T366S, L368A, and Y407V mutations in the other Fc polypeptide constituting the Fc region (wherein positions of the mutations are amino acid positions that are numbered according to the EU index in a human Igγ1 constant region).
[70] The bispecific antibody according to [66], wherein the sequences of the first Fc polypeptide and the second Fc polypeptide are polypeptides consisting of any sequence of the following (1) or (2):
   (1) a first Fc polypeptide consisting of the amino acid sequence of SEQ ID NO: 40, and a second Fc polypeptide consisting of the amino acid sequence of SEQ ID NO: 41; or
   (2) a first Fc polypeptide consisting of the amino acid sequence of SEQ ID NO: 41, and a second Fc polypeptide consisting of the amino acid sequence of SEQ ID NO: 40.
[71] The bispecific antibody according to [70], containing a hinge region consisting of the amino acid sequence represented by SEQ ID NO: 42.
[72] The bispecific antibody according to any of [59] to [71], wherein the bispecific antibody is modified post-translationally.
[73] A fusion product or a complex of the bispecific antibody according to any of [59] to [72], or a cell allowed to express the bispecific antibody according to any of [59] to [72] on the cell surface.
[74] A polynucleotide for use in the production of the bispecific antibody according to any of [59] to [72], the polynucleotide being selected from the group consisting of the following (1) to (9):
   (1) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8;
   (2) a polynucleotide containing a nucleotide sequence encoding a light chain variable region of an anti-MerTK antibody, the light chain variable region consisting of an amino acid sequence from amino acid positions 1 to 109 of SEQ ID NO: 10;
   (3) a polynucleotide containing a nucleotide sequence encoding a light chain variable region of an anti-MerTK antibody, the light chain variable region consisting of an amino acid sequence from amino acid positions 1 to 109 of SEQ ID NO: 12;
   (4) a polynucleotide containing a nucleotide sequence encoding a light chain variable region of an anti-MerTK antibody, the light chain variable region consisting of an amino acid sequence from amino acid positions 1 to 109 of SEQ ID NO: 14;
   (5) a polynucleotide containing a nucleotide sequence encoding a light chain variable region of an anti-MerTK antibody, the light chain variable region consisting of an amino acid sequence from amino acid positions 1 to 110 of SEQ ID NO: 16;
   (6) a polynucleotide containing a nucleotide sequence encoding a light chain variable region of an anti-MerTK antibody, the light chain variable region consisting of an amino acid sequence from amino acid positions 1 to 110 of SEQ ID NO: 18;
   (7) a polynucleotide containing a nucleotide sequence encoding a light chain variable region of an anti-MerTK antibody, the light chain variable region consisting of an amino acid sequence from amino acid positions 1 to 110 of SEQ ID NO: 20;
   (8) a polynucleotide containing a nucleotide sequence encoding a heavy chain variable region of an anti-IgL antibody, the heavy chain variable region consisting of an amino acid sequence from amino acid positions 1 to 119 of SEQ ID NO: 57; and
   (9) a polynucleotide containing a nucleotide sequence encoding a light chain variable region of an anti-IgL antibody, the light chain variable region consisting of an amino acid sequence from amino acid positions 1 to 112 of SEQ ID NO: 59.
[75] An expression vector containing the polynucleotide according to [74].
[76] A host cell transformed with the expression vector according to [75].
[77] A host cell for use in the production of the bispecific antibody according to any of [59] to [72], the host cell containing polynucleotides selected from the group consisting of the following (1) to (4):
   (1) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8, a polynucleotide containing a nucleotide sequence encoding a light chain variable region of an anti-MerTK antibody, the light chain variable region consisting of an amino acid sequence from amino acid positions 1 to 109 of SEQ ID NO: 12, a polynucleotide containing a nucleotide sequence encoding a heavy chain variable region of an anti-IgL antibody, the heavy chain variable region consisting of an amino acid sequence from amino acid positions 1 to 119 of SEQ ID NO: 57, and a polynucleotide containing a nucleotide sequence encoding a light chain variable region of an anti-IgL antibody, the light chain variable region consisting of an amino acid sequence from amino acid positions 1 to 112 of SEQ ID NO: 59;
   (2) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8, a polynucleotide containing a nucleotide sequence encoding a light chain variable region of an anti-MerTK antibody, the light chain variable region consisting of an amino acid sequence from amino acid positions 1 to 110 of SEQ ID NO: 16, a polynucleotide containing a nucleotide sequence encoding a heavy chain variable region of an anti-IgL antibody, the heavy chain variable region consisting of an amino acid sequence from amino acid positions 1 to 119 of SEQ ID NO: 57, and a polynucleotide containing a nucleotide sequence encoding a light chain variable region of an anti-IgL antibody, the light chain variable region consisting of an amino acid sequence from amino acid positions 1 to 112 of SEQ ID NO: 59;
   (3) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8, a polynucleotide containing a nucleotide sequence encoding a light chain variable region of an anti-MerTK antibody, the light chain variable region consisting of an amino acid sequence from amino acid positions 1 to 110 of SEQ ID NO: 18, a polynucleotide containing a nucleotide sequence encoding a heavy chain variable region of an anti-IgL antibody, the heavy chain variable region consisting of an amino acid sequence from amino acid positions 1 to 119 of SEQ ID NO: 57, and a polynucleotide containing a nucleotide sequence encoding a light chain variable region of an anti-IgL antibody, the light chain variable region consisting of an amino acid sequence from amino acid positions 1 to 112 of SEQ ID NO: 59; and
   (4) a polynucleotide containing a nucleotide sequence encoding a heavy chain variable region of an anti-MerTK antibody, the heavy chain variable region consisting of an amino acid sequence from amino acid positions 1 to 119 of SEQ ID NO: 8, a polynucleotide containing a nucleotide sequence encoding a light chain variable region of an anti-MerTK antibody, the light chain variable region consisting of an amino acid sequence from amino acid positions 1 to 110 of SEQ ID NO: 20, a polynucleotide containing a nucleotide sequence encoding a heavy chain variable region of an anti-IgL antibody, the heavy chain variable region consisting of an amino acid sequence from amino acid positions 1 to 119 of SEQ ID NO: 57, and a polynucleotide containing a nucleotide sequence encoding a light chain variable region of an anti-IgL antibody, the light chain variable region consisting of an amino acid sequence from amino acid positions 1 to 112 of SEQ ID NO: 59.
[78] A method for producing a bispecific antibody that binds to MerTK and IgL, containing the step of culturing the host cell according to [76] or [77] so that the bispecific antibody is expressed.
[79] A pharmaceutical composition containing the bispecific antibody according to any of [59] to [72], and a pharmaceutically acceptable excipient.
[80] The pharmaceutical composition according to [79] for use in the prevention and/or the treatment of diseases derived from the accumulation of a misfolded protein.
[81] The pharmaceutical composition according to [80], wherein the disease derived from the accumulation of a misfolded protein is amyloidosis.
[82] The pharmaceutical composition according to [81], wherein the amyloidosis is systemic amyloidosis.
[83] The pharmaceutical composition according to [82], wherein the systemic amyloidosis is AL amyloidosis.
[84] The bispecific antibody according to any of [59] to [72] for use in the prevention and/or the treatment of diseases derived from the accumulation of a misfolded protein.
[85] A method for preventing and/or treating a disease derived from the accumulation of a misfolded protein, containing the step of administering a therapeutically effective amount of the bispecific antibody according to any of [59] to [72] to a subject.
[86] Use of the bispecific antibody according to any of [59] to [72] in the production of a pharmaceutical composition for the prevention and/or the treatment of diseases derived from the accumulation of a misfolded protein.

### ADVANTAGEOUS EFFECTS OF INVENTION

The anti-MerTK antibody or the antigen binding fragment thereof and the anti-MerTK bispecific antibody according to the present invention activate the phagocytic clearance function of phagocytes by binding to MerTK expressed on the phagocytes and activating MerTK-mediated intracellular downstream signals. The anti-MerTK antibody or the antigen binding fragment thereof and the anti-MerTK bispecific antibody according to the present invention, or pharmaceutical compositions containing them can be used for the prevention or the treatment of diseases (e.g., an eye disease) that can be prevented or treated by the activation of MerTK-mediated intracellular downstream signals.

### BRIEF DESCRIPTION OF DRAWINGS

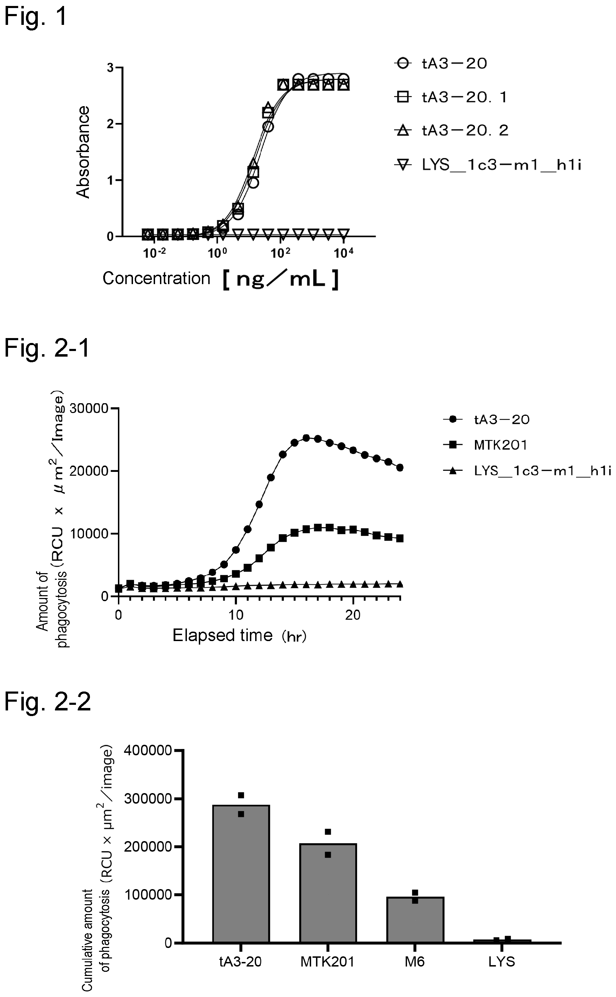

[Fig. 1] Fig. 1 shows the binding activity of a test antibody against human MerTK-His protein, as measured by ELISA. The ordinate of the drawing depicts the binding activity (value obtained by subtracting a background value absorbance at 570 nm from absorbance at 450 nm), and the abscissa depicts the concentration of the test antibody. The symbols depict a mean absorbance value of each test antibody.
[Fig. 2-1] Fig. 2-1 shows the action of a test antibody on the phagocytosis of apoptotic cells by iPS-RPE cells. The ordinate depicts fluorescence intensity serving as an indicator for the amount of phagocytosis of apoptotic cells. The abscissa depicts an elapsed time from the start of evaluation. Each dot depicts a mean value from four wells.
[Fig. 2-2] Fig. 2-2 shows the activity of a test antibody on the phagocytosis of pig POS by iPS-RPE cells. The ordinate depicts a cumulative value from the start of measurement to 60 hours later of fluorescence intensity serving as an indicator for the amount of phagocytosis of POS. Each dot depicts a numeric value from each well. The values of the bar graph depict mean values.

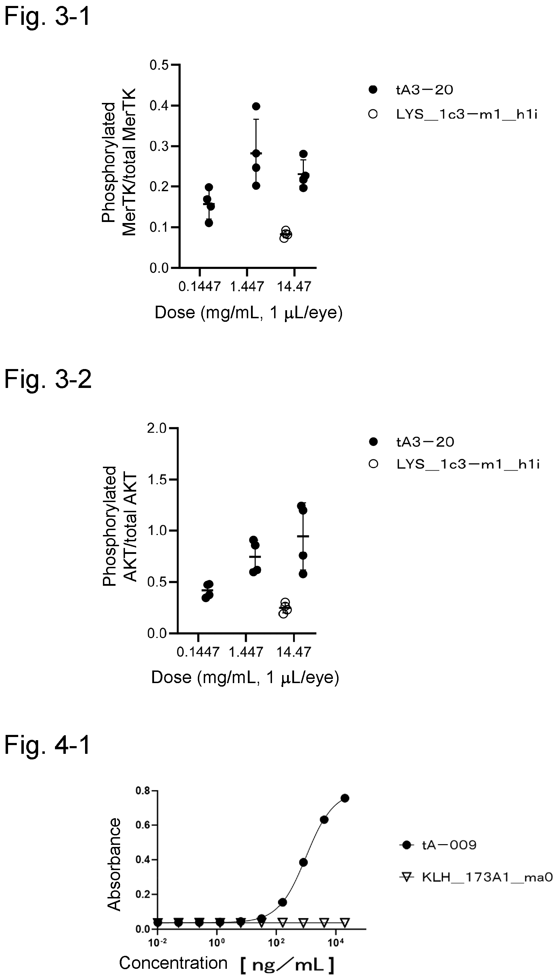

[Fig. 3-1] Fig. 3-1 shows a phosphorylated MerTK protein level in RPE 1 hour after administration of tA3-20 into the mouse vitreous body. The ordinate of the drawing depicts the amount of the phosphorylated MerTK protein normalized with the total amount of MerTK protein, and the abscissa depicts the dose of the test antibody. Each dot depicts a numeric value from each individual. The bars depict mean values. The error bars depict standard deviations.
[Fig. 3-2] Fig. 3-2 shows a phosphorylated AKT protein level in RPE 1 hour after administration of tA3-20 into the mouse vitreous body. The ordinate of the drawing depicts the amount of the phosphorylated AKT protein normalized with the total amount of AKT protein, and the abscissa depicts the dose of the test antibody. Each dot depicts a numeric value from each individual. The bars depict mean values. The error bars depict standard deviations.
[Fig. 4-1] Fig. 4-1 shows the binding activity of a test antibody against misTTR measured by ELISA. The ordinate of the drawing depicts the binding activity (value obtained by subtracting a background value absorbance at 570 nm from absorbance at 450 nm), and the abscissa depicts the concentration of the test antibody. The symbols depict a mean absorbance value of each test antibody.

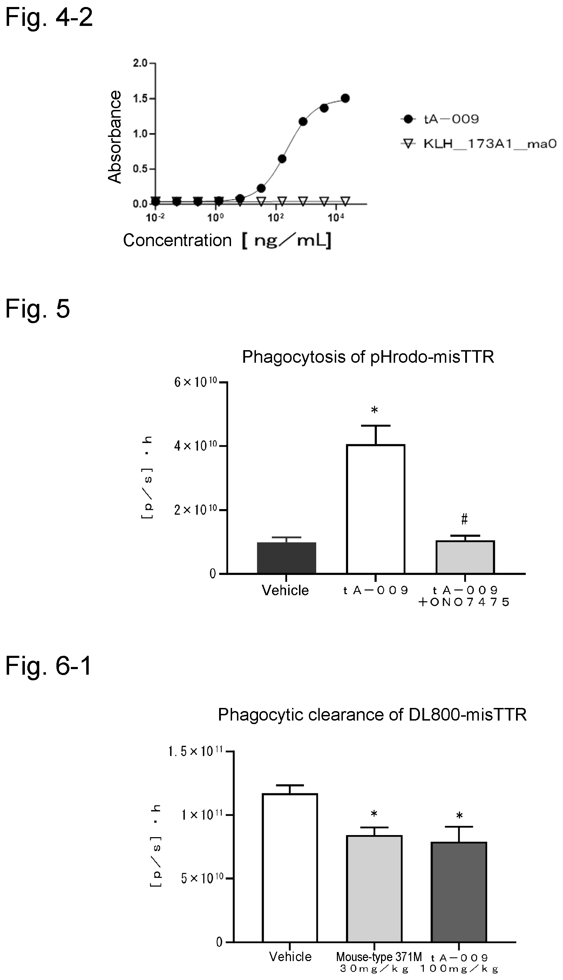

[Fig. 4-2] Fig. 4-2 shows the binding activity of a test antibody against human MerTK-Fc protein measured by ELISA. The ordinate of the drawing depicts the binding activity (value obtained by subtracting a background value absorbance at 570 nm from absorbance at 450 nm), and the abscissa depicts the concentration of the test antibody. The symbols depict a mean absorbance value of each test antibody.
[Fig. 5] Fig. 5 shows MerTK-dependent phagocytosis of pHrodo-misTTR by tA-009. The ordinate of the drawing depicts the total amount of phagocytosis ([p/s] h) for an observation period. The values depict mean + standard error. The comparison between a vehicle group as a control group and a tA-009 group and the comparison between the tA-009 group as a control group and a tA-009 + ONO7475 group were performed using Student's t-test, and statistical significance was confirmed when a P value was less than 0.05. The asterisk (*) and the sharp (#) in the drawing depict significant differences in the comparison between the vehicle group and the tA-009 group and the comparison between the tA-009 group and the tA-009 + ONO7475 group, respectively.
[Fig. 6-1] Fig. 6-1 shows the activation of a phagocytic clearance function for DL800-misTTR by tA-009 or mouse-type 371M. The ordinate of the drawing depicts the total amount of residual misTTR ([p/s] h) for an observation period. The values depict mean + standard error. The comparison between a vehicle group as a control group and a drug (mouse-type 371M or tA-009) group was performed using Dunnett's test, and statistical significance was confirmed when a P value was less than 0.05. * in the drawing depicts a significant difference in the comparison between the vehicle group and the drug group.

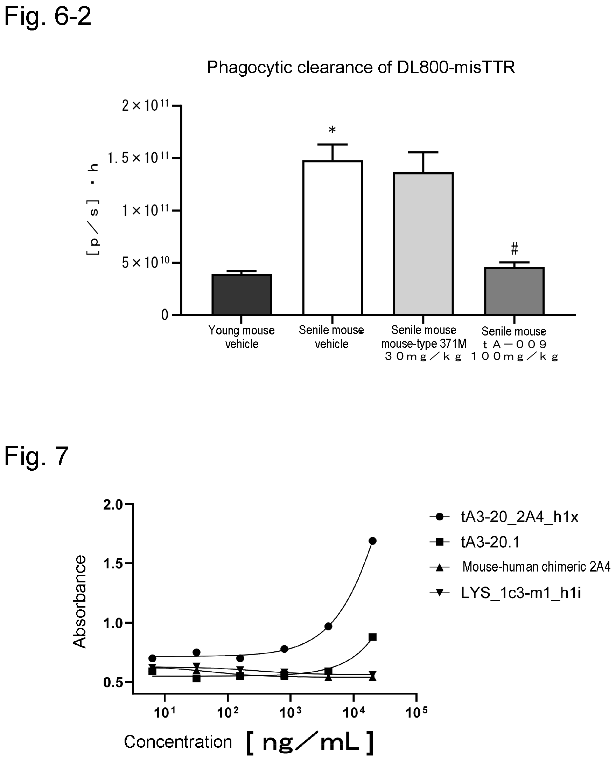

[Fig. 6-2] Fig. 6-2 shows the activation of a phagocytic clearance function by tA-009 or mouse-type 371M against DL800-misTTR subcutaneously administered to senile mice. The ordinate of the drawing depicts the total amount of residual misTTR ([p/s] h) for an observation period. The values depict mean + standard error. The comparison between a young mouse/vehicle group as a control group and a senile mouse/vehicle group was performed using Student's t-test, and the comparison between the senile mouse/vehicle group as a control group and a senile mouse/drug (mouse-type 371M or tA-009) group was performed using Dunnett's test. Statistical significance was confirmed when each P value was less than 0.05. * and # in the drawing depict significant differences in the comparison between the young mouse/vehicle group and the senile mouse/vehicle group and the comparison between the senile mouse/vehicle group and the senile mouse/drug group, respectively.
[Fig. 7] Fig. 7 shows the binding activity of a test antibody against human MerTK and misfolded IgL protein, as measured by ELISA. The ordinate of the drawing depicts the binding activity (value obtained by subtracting a background value absorbance at 570 nm from absorbance at 450 nm), and the abscissa depicts the concentration of the test antibody. The symbols depict the absorbance of each test antibody.

### DESCRIPTION OF EMBODIMENTS

Hereinafter, the present invention will be described in detail.

### <Definition>

Terms of the present specification are used in meanings that are generally used by those skilled in the art unless otherwise specified below.

An antibody (or an immunoglobulin) refers to a glycoprotein, the basic structure of which is a four-chain structure having a bilaterally symmetrical Y-shaped structure composed of two heavy chains having a single sequence and two light chains having a single sequence. The antibody has five classes: IgG, IgM, IgA, IgD, and IgE. The basic structure of the antibody molecule is common among these classes, and two heavy chains having a molecular weight of 50,000 to 70,000 and two light chains having a molecular weight of 20,000 to 30,000 are bound through a disulfide bond and a noncovalent bond to form an antibody molecule having a Y-shaped four-chain structure with a molecular weight of 150,000 to 190,000. The heavy chain consists of a polypeptide chain usually containing approximately 440 amino acids, has a structure characteristic of each class, and is designated as Igγ, Igµ, Igα, Igδ, and Igε correspondingly to IgG, IgM, IgA, IgD, and IgE, respectively. IgG further has subclasses IgG1, IgG2, IgG3, and IgG4, and heavy chains corresponding thereto are designated as Igγ1, Igγ2, Igγ3, and Igγ4, respectively. The light chain consists of a polypeptide chain usually containing approximately 220 amino acids, and is known to have two types, λ type and κ type, which are designated as Igλ and Igκ, respectively. Each of these two types of light chains can be paired with any type of heavy chain.

In an antibody molecule, the heavy chain has four intrachain disulfide bonds (five in Igµ and Igε) while the light chain has two intrachain disulfide bonds, and one loop is formed every 100 to 110 amino acid residues. Their three-dimensional structures are similar among the loops and are designated as structural units or domains. In both the heavy chain and the light chain, a domain positioned at the amino terminus (in the present specification, also referred to as the "N terminus") is designated as a variable region, which has diverse amino acid sequences even among antibodies produced from the same class (or subclass) of the same species of animals, and is known to contribute to antibody-antigen binding specificity. A domain on the carboxy-terminal (in the present specification, also referred to as the "C terminus") side, downstream of the variable region, on the C-terminal side has an amino acid sequence substantially constant in each class or subclass, and is designated as a constant region. The heavy chain has, in the direction from the N terminus toward the C terminus, a heavy chain variable region (VH) and a heavy chain constant region (CH). CH is further divided, from the N-terminal side, into three domains: a CH1 domain, a CH2 domain, and a CH3 domain. The light chain has, in the direction from the N terminus toward the C terminus, a light chain variable region (VL) and a light chain constant region (CL).

The amino acid sequences of three complementarity-determining regions (CDRs) present in VH and VL vary very largely and contribute to the variability of the variable regions. CDRs reside in the order of CDR1, CDR2, and CDR3 at the N terminus of each of the heavy chain and the light chain, and these regions are composed of approximately 5 to 10 amino acid residues and form an antigen binding site. On the other hand, a portion except for CDRs of the variable region is designated as framework regions (FRs), which consist of FR1 to FR4 and comparatively little vary in amino acid sequence.

When an antibody is treated with protease papain, three antibody fragments are obtained. Two N-terminal fragments are designated as Fab (fragment, antigen binding) regions. As used herein, the "Fab region" refers to a region composed VH and CH1 domain of the heavy chain and the light chain (VL and CL) and binds to an antigen at an antigen binding site located at an end portion constituted by the Fab region. As used herein, the "heavy chain fragment" refers to a fragment composed of VH and CH1 domain of the heavy chain, that constitute the Fab region. The C-terminal fragment is designated as an Fc (fragment, crystallizable) region. As used herein, the "Fc polypeptide" refers to a polypeptide composed of CH2 domain and CH3 domain of the heavy chain, and the "Fc region" refers to a complex of two Fc polypeptides, a first Fc polypeptide and a second Fc polypeptide. In this context, the term "first" or "second" used for the Fc polypeptide is used for convenient discrimination and does not intend to confer a particular order or meaning.

As used herein, the "hinge region" refers to a highly mobile peptide region disposed between CH1 domain and CH2 domain of the heavy chain. The heavy chain fragment and the Fc polypeptide are linked via the hinge region. Two antibody heavy chains are linked via a disulfide bond in the hinge region.

As used herein, the "antigen" is used in a meaning usually used, and is used as a term that refers to a molecule , or a portion thereof, to which an antibody or an antigen binding fragment is capable of specifically binding. The antigen can be a molecule such as a protein or a nucleic acid. One antigen may have one or more epitopes that can interact with different antibodies or the like.

As used herein, the "IgG antibody" refers to an antibody having a Y-shaped structure composed of two Fab regions and an Fc region. Specifically, the IgG antibody has a structure composed of two heavy chains and two light chains. In one embodiment, the two Fab regions of the IgG antibody contain the same VH and VL sequences.

As used herein, the "antigen binding fragment" refers to a molecule containing at least one polypeptide chain that contains a heavy chain variable region and a light chain variable region of an immunoglobulin molecule and has antigen binding activity. Representative examples of the antigen binding fragment include a single-chain variable fragment (scFv), a Fab fragment, a Fab' fragment, and a F(ab')₂ fragment. The scFv is a monovalent antigen binding fragment constituted by VH and VL linked via a linker. The Fab fragment is a monovalent antigen binding fragment constituted by a fragment containing a light chain and VH and CH1 domain of heavy chain. The Fab fragment contains a Fab region. The Fab' fragment is a monovalent antigen binding fragment constituted by a fragment containing a light chain, VH and CH1 domain of heavy chain, and a portion of a hinge region, and this portion of the hinge region contains a cysteine residue contained in the S-S bond between the heavy chains. The F(ab')₂ fragment is a bivalent molecule of Fab' fragments linked through a disulfide bond. "Monovalent" means that one antigen binding site is contained, and "bivalent" means that two antigen binding sites are contained. The Fab fragment, the Fab' fragment, and the F(ab')₂ fragment may also include a fragment having whole or partial crossover of VH and CH1 domain of heavy chain and VL and CL of light chain (mAbs, 2016, 8 (6): 1010-1020). The "antigen binding fragment" may also include a heavy chain antibody such as a VHH antibody (mAbs, 2017, 9 (2): 182-212).

In the present specification, the "Half-Ig" is a monovalent antibody containing one VH, one VL, and one Fc polypeptide. In one embodiment, the Half-Ig contains one VH, one CH1 domain, one VL, and one Fc polypeptide. In one embodiment, the Half-Ig contains one heavy chain fragment (VH and CH1 domain), one Fc polypeptide (CH2 domain and CH3 domain), and one light chain (VL and CL). In one embodiment, the Half-Ig contains one heavy chain and one light chain.

In the present specification, the "one-armed antibody" is a monovalent antibody containing one VH, one CH1 domain, one VL, one CL, and one Fc region consisting of a first Fc polypeptide and a second Fc polypeptide. In one embodiment, the one-armed antibody contains one heavy chain fragment (VH and CH1 domain), one Fc region (first and second Fc polypeptides (CH2 domain and CH3 domain)), and one light chain (VL and CL). In one embodiment, the one-armed antibody contains one heavy chain fragment, a hinge region, one Fc region, and one light chain. In one embodiment, the one-armed antibody contains one Fab region, hinge region and one Fc region. In one embodiment, the one-armed antibody contains one Fab region, a hinge region, and one Fc region and has a structure where the heavy chain fragment of the Fab region is linked via the hinge region to the first Fc polypeptide of the Fc region. In one embodiment, the one-armed antibody contains one Fab region, a hinge region, and one Fc region and has a structure where the heavy chain fragment of the Fab region is linked via the hinge region to the N terminus of the first Fc polypeptide of the Fc region (one heavy chain).

As used herein, the "bispecific antibody" refers to an antibody that can specifically bind to two different antigens. The bispecific antibody is constituted by two antibodies or antigen binding fragments that can bind to different antigens, respectively. Bispecific antibodies having various structures are known to those skilled in the art (mAbs, 2017, 9 (2): 182-212).

As used herein, the "multispecific antibody" refers to an antibody that can specifically bind to three or more different antigens, and is designated as, for example, a trispecific antibody or a tetraspecific antibody, depending on the number of antigens to be bound. The multispecific antibody is constituted by three or more antibodies and/or antigen binding fragments that can bind to different antigens, respectively.

In the present specification, the "antibody" is used as a term including an IgG antibody, Half-Ig, a one-armed antibody, a bispecific antibody, and a multispecific antibody unless particularly limited contextually.

As used herein, the "human antibody" refers to an antibody having a human immunoglobulin amino acid sequence. As used herein, the "fully human antibody" refers to an antibody consisting of only a human immunoglobulin amino acid sequence. As used herein, the "mouse-human chimeric antibody" refers to an antibody having antigen binding variable regions of a mouse immunoglobulin amino acid sequence and constant regions of a human immunoglobulin amino acid sequence.

An amino acid residue number of an antibody used in the present specification can be prescribed by specifying the Kabat numbering or EU index (Kabat et al., Sequences of Proteins of Immunological Interest, 5th Ed., 1991, NIH Publication: No. 91-3242) in accordance with the numbering system.

As used herein, the "subject" means a human or other animals in need of prevention or treatment of diseases. In one embodiment, the subject is a human in need of prevention or treatment of diseases. In one embodiment, the subject is a human in need of the prevention or treatment of various eye diseases, lung diseases, autoimmune diseases, neuropathic diseases, heart diseases, endocrine/metabolic diseases, cancers, or the like.

As used herein, the "diagnosis" encompasses every diagnosis such as not only the determination of the presence or absence of affection with a disease but the determination of the severity (degree of progression) after definitive diagnosis or whether or not the future possibility of affection with or onset of a disease is high.

As used herein, the "prevention" means some intervention that is performed for preventing or delaying the development of a disease.

As used herein, the "treatment" means some intervention, procedure, or administration of an active ingredient to a subject that is performed for the subject for the purpose of recovering, alleviating, improving, suppressing, or delaying the progression, onset, aggravation, or recurrence of symptoms of a disease, a pathogenic condition, or a biochemical sign related to a disease.

As used herein, the "active ingredient" means a substance that is contained in a pharmaceutical composition, a medicament, or the like for use in the prevention or the treatment of diseases and exhibits some physiological activity. In one embodiment, the active ingredient is an antibody or an antigen binding fragment. In one embodiment, the active ingredient is a fusion product or a complex of the antibody or the antigen binding fragment. In one embodiment, the active ingredient is a bispecific antibody.

In the present specification, the "pharmaceutical composition" is a drug that contains an active ingredient and a pharmaceutically acceptable excipient (including, for example, but not limited to, a pharmaceutical excipient or a pharmaceutical carrier) and is prescribed for the purpose of preventing or treating a subject.

As used herein, the "effective amount" of a drug refers to an amount of the drug necessary for bringing about physiological change in a subject by acting on tissues or cells of the subject when the drug is administered to the subject.

### <Anti-MerTK antibody of present invention>

As used herein, the anti-MerTK antibody or the antigen binding fragment thereof refers to an antibody capable of binding to human MerTK, or an antigen binding fragment thereof. Whether it binds to human MerTK can be confirmed by use of a known binding activity measurement method. For example, enzyme-linked immunosorbent assay (ELISA) described in Example 1-2 or 1-3 can be used as the method for measuring binding activity.

The present invention provides the following anti-MerTK antibody or antigen binding fragment thereof:
an anti-MerTK antibody, or an antigen binding fragment thereof, containing a heavy chain variable region containing CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and a light chain variable region containing CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6.

In the present specification, this anti-MerTK antibody is also referred to as the "anti-MerTK antibody of the present invention", and this anti-MerTK antibody or antigen binding fragment thereof is also referred to as the "anti-MerTK antibody or antigen binding fragment thereof of the present invention".

In one embodiment, the anti-MerTK antibody or the antigen binding fragment thereof of the present invention is an anti-MerTK antibody or an antigen binding fragment thereof containing a heavy chain variable region and a light chain variable region selected from the group consisting of the following (1) to (6):
(1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
(2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
(3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20
   .

In one embodiment, the anti-MerTK antibody of the present invention is an antibody containing a heavy chain and a light chain. The antibody containing a heavy chain and a light chain includes an IgG antibody, Half-Ig, and a one-armed antibody. In one embodiment, the anti-MerTK antibody of the present invention is an IgG antibody (anti-MerTK IgG antibody).

In one embodiment, the antigen binding fragment of the present invention is scFv, a Fab fragment, a Fab' fragment, a F(ab')₂ fragment, or a VHH antibody.

Any constant region of Igγ, Igµ, Igα, Igδ, or Igε may be selected as a heavy chain constant region contained in the anti-MerTK antibody or the antigen binding fragment thereof of the present invention. The Igγ may be selected from, for example, Igγ1, Igγ2, Igγ3, and Igγ4. Any constant region of Igλ or Igκ may be selected as a light chain constant region contained in the anti-MerTK antibody or the antigen binding fragment thereof of the present invention. In one embodiment, the heavy chain constant region and the light chain constant region of the anti-MerTK antibody or the antigen binding fragment thereof are human Igγ1 and Igλ constant regions, respectively.

The anti-MerTK antibody of the present invention may contain a mutation that reduces antibody-dependent cellular cytotoxicity (ADCC), antibody-dependent cellular phagocytosis (ADCP), or complement-dependent cytotoxicity (CDC). L234A is a substitution of leucine at amino acid position 234 of a human Igγ1 constant region by alanine. L235A is a substitution of leucine at amino acid position 235 of a human Igγ1 constant region by alanine. The L234A and L235A amino acid mutations in the human Igγ1 constant region are referred to as "LALA mutations". These mutations are known to reduce the binding activity of an antibody Fc region against Fcγ receptor and the binding activity against C1q and thereby reduce ADCC, ADCP, or CDC of the antibody (Mol. Immunol., 1992, 29: 633-639; J. Immunol., 2000, 164: 4178-4184; and Front. Immunol., 2019, 10: 1296). P329A is a substitution of proline at amino acid position 329 of a human Igγ1 constant region by alanine, and P331G or P331S is a substitution of proline at amino acid position 331 of a human Igγ1 constant region by glycine or serine. These mutations are known to reduce CDC of the antibody (J. Immunol., 2000, 164: 4178-4184).

In one embodiment, the anti-MerTK antibody of the present invention contains L234A and L235A amino acid mutations (LALA mutations) in the heavy chain. In one embodiment, the anti-MerTK antibody of the present invention contains any of P329A, P331G, or P331S mutations in the heavy chain. In one embodiment, the anti-MerTK antibody of the present invention contains LALA mutations and any of P329A, P331G, or P331S mutations in the heavy chain. In one embodiment, the anti-MerTK antibody of the present invention contains LALA mutations and a P329A mutation in the heavy chain. In one embodiment, the anti-MerTK antibody of the present invention contains LALA mutations and a P331G mutation in the heavy chain.

In the present specification, the amino acid mutations such as LALA mutations or a P329A, P331G or P331S mutation are described on the basis of amino acid positions that are numbered according to the EU index in a human Igγ1 constant region. For example, L234A is, as mentioned above, a substitution of leucine at amino acid position 234 that is numbered according to the EU index in a human Igγ1 constant region by alanine.

The anti-MerTK antibody of the present invention may further contain other mutations based on known techniques. The anti-MerTK antibody of the present invention may contain, for example, an N297G mutation (Protein cell, 2018, 9: 63-73), mutations based on a technique of knobs into holes (kih) (hereinafter, also referred to as "knobs-into-holes mutations"), or electrostatic steering mutations.

The kih technique is a technique that can efficiently obtain a heterodimeric antibody molecule of interest by replacing amino acid side chains present in a CH3 region in one of the heavy chains with larger side chains (knobs), replacing amino acid side chains present in a CH3 region of the other heavy chain with smaller side chains (holes), and placing the knobs into the holes to promote the heterodimerization of the heavy chains (Nature, 1994, 372: 379-383; Nature Biotech., 1998, 16: 677-681; J. Mol. Biol., 1997, 270: 26-35; Proc. Natl. Acad. Sci. U.S.A., 2013, 110: E2987-E2996, and International Publication No. WO 1998/050431).

The technique using electrostatic steering is a technique that can efficiently obtain a heterodimeric antibody molecule of interest by replacing amino acid side chains present in a CH3 region in one of the heavy chains with negatively charged amino acid side chains, and replacing amino acid side chains present in a CH3 region of the other heavy chain with positively charged amino acid side chains (in the present specification, referred to as "electrostatic steering mutations") to promote the heterodimerization of the heavy chains through electrostatic force (J. Biol. Chem., 2010, 285: 19637-19646). Mutations including E356K, T364S, M368L, T370K, D399K, and R411T in a CH3 domain in one of the heavy chains and T364S, M368L, T370K, K409E, R411T, and K439D in a CH3 region of the other heavy chain are known as electrostatic steering mutations in mouse-derived antibodies and mutations for stabilizing heterodimeric antibody molecules (mAbs, 2019, 12: 1-12).

In one embodiment, the anti-MerTK antibody of the present invention is an anti-MerTK antibody selected from the group consisting of the following (1) to (6):
(1) an anti-MerTK antibody containing a heavy chain consisting of the amino acid sequence of SEQ ID NO: 8, and a light chain consisting of the amino acid sequence of SEQ ID NO: 10;
(2) an anti-MerTK antibody containing a heavy chain consisting of the amino acid sequence of SEQ ID NO: 8, and a light chain consisting of the amino acid sequence of SEQ ID NO: 12;
(3) an anti-MerTK antibody containing a heavy chain consisting of the amino acid sequence of SEQ ID NO: 8, and a light chain consisting of the amino acid sequence of SEQ ID NO: 14;
(4) an anti-MerTK antibody containing a heavy chain consisting of the amino acid sequence of SEQ ID NO: 8, and a light chain consisting of the amino acid sequence of SEQ ID NO: 16;
(5) an anti-MerTK antibody containing a heavy chain consisting of the amino acid sequence of SEQ ID NO: 8, and a light chain consisting of the amino acid sequence of SEQ ID NO: 18; and
(6) an anti-MerTK antibody containing a heavy chain consisting of the amino acid sequence of SEQ ID NO: 8, and a light chain consisting of the amino acid sequence of SEQ ID NO: 20.

The anti-MerTK antibody or the antigen binding fragment thereof of the present invention also includes an antibody that binds to human MerTK as well as to mouse-, monkey-, and/or rat-derived MerTK, or an antigen binding fragment thereof. In one embodiment, the anti-MerTK antibody or the antigen binding fragment thereof of the present invention is an antibody that binds to human-, mouse-, monkey-, and/or rat-derived MerTK, or an antigen binding fragment thereof. In one embodiment, the anti-MerTK antibody or the antigen binding fragment thereof of the present invention binds to human MerTK (gene No: NM_006343). The binding to each MerTK can be confirmed by use of a known binding activity measurement method. Examples of the method for measuring binding activity include methods such as ELISA and flow cytometry. In the case of using ELISA or flow cytometry, a method described in, for example, Example 3 or 5 of International Publication No. WO 2022/224997 can be used.

In a method for evaluating the agonistic activity of the anti-MerTK antibody or the antigen binding fragment thereof of the present invention, activity of inducing AKT phosphorylation as downstream signals of MerTK may be evaluated. For example, a known method can be used as a method for evaluating the activity of inducing AKT phosphorylation. In the present specification, the anti-MerTK antibody having the activity of inducing AKT phosphorylation is also referred to as an "anti-MerTK agonist antibody".

As used herein, "post-translational modification" means that an antibody, when expressed intracellularly, undergoes a modification after translation. Examples of the post-translational modification include N-linked or O-linked glycosylation, N-terminal or C-terminal processing, deamidation, aspartic acid isomerization, and methionine oxidation. Such post-translational modifications are known to occur in various antibodies (J. Pharm. Sci., 2008, 97: 2426-2447).

In one embodiment, the anti-MerTK antibody or the antigen binding fragment thereof of the present invention may be modified post-translationally. In one embodiment, the post-translational modification is N-linked or O-linked glycosylation, N-terminal or C-terminal processing, deamidation, aspartic acid isomerization, and/or methionine oxidation.

Those skilled in the art are capable of readily preparing the anti-MerTK antibody or the antigen binding fragment thereof of the present invention by using a method known in the art on the basis of sequence information on the heavy chain variable region and the light chain variable region of the anti-MerTK antibody of the present invention disclosed in the present specification. The anti-MerTK antibody or the antigen binding fragment thereof of the present invention can be produced in accordance with, for example, but not particularly limited to, a method described in the after-mentioned section <Method for producing anti-MerTK antibody of present invention>.

### <Polynucleotide of present invention>

The present invention also provides a polynucleotide containing a nucleotide sequence encoding the heavy chain variable region of the anti-MerTK antibody or the antigen binding fragment thereof of the present invention, and a polynucleotide containing a nucleotide sequence encoding the light chain variable region of the anti-MerTK antibody or the antigen binding fragment thereof of the present invention (also referred to as the "polynucleotide of the present invention").

In one embodiment, the polynucleotide containing a nucleotide sequence encoding the heavy chain variable region of the anti-MerTK antibody or the antigen binding fragment thereof of the present invention is a polynucleotide containing a nucleotide sequence encoding a heavy chain variable region consisting of an amino acid sequence from amino acid positions 1 to 119 of SEQ ID NO: 8.

In one embodiment, a polynucleotide containing a nucleotide sequence encoding the heavy chain of the anti-MerTK antibody of the present invention is a polynucleotide containing a nucleotide sequence encoding a heavy chain consisting of the amino acid sequence represented by SEQ ID NO: 8.

In one embodiment, the polynucleotide containing a nucleotide sequence encoding the light chain variable region of the anti-MerTK antibody or the antigen binding fragment thereof of the present invention is a polynucleotide selected from the group consisting of the following (a) to (f):
(a) a polynucleotide containing a nucleotide sequence encoding a light chain variable region consisting of an amino acid sequence from amino acid positions 1 to 109 of SEQ ID NO: 10;
(b) a polynucleotide containing a nucleotide sequence encoding a light chain variable region consisting of an amino acid sequence from amino acid positions 1 to 109 of SEQ ID NO: 12;
(c) a polynucleotide containing a nucleotide sequence encoding a light chain variable region consisting of an amino acid sequence from amino acid positions 1 to 109 of SEQ ID NO: 14;
(d) a polynucleotide containing a nucleotide sequence encoding a light chain variable region consisting of an amino acid sequence from amino acid positions 1 to 110 of SEQ ID NO: 16;
(e) a polynucleotide containing a nucleotide sequence encoding a light chain variable region consisting of an amino acid sequence from amino acid positions 1 to 110 of SEQ ID NO: 18; and
(f) a polynucleotide containing a nucleotide sequence encoding a light chain variable region consisting of an amino acid sequence from amino acid positions 1 to 110 of SEQ ID NO: 20.

In one embodiment, a polynucleotide containing a nucleotide sequence encoding the light chain of the anti-MerTK antibody of the present invention is a polynucleotide selected from the group consisting of the following (a) to (f):
(a) a polynucleotide containing a nucleotide sequence encoding a light chain consisting of the amino acid sequence of SEQ ID NO: 10;
(b) a polynucleotide containing a nucleotide sequence encoding a light chain consisting of the amino acid sequence of SEQ ID NO: 12;
(c) a polynucleotide containing a nucleotide sequence encoding a light chain consisting of the amino acid sequence of SEQ ID NO: 14;
(d) a polynucleotide containing a nucleotide sequence encoding a light chain consisting of the amino acid sequence of SEQ ID NO: 16;
(e) a polynucleotide containing a nucleotide sequence encoding a light chain consisting of the amino acid sequence of SEQ ID NO: 18; and
(f) a polynucleotide containing a nucleotide sequence encoding a light chain consisting of the amino acid sequence of SEQ ID NO: 20.

Those skilled in the art are capable of readily preparing the polynucleotide of the present invention by using a method known in the art on the basis of the nucleotide sequence thereof. The polynucleotide of the present invention may be synthesized by use of, for example, a gene synthesis method known in the art. Various methods known to those skilled in the art, such as an antibody gene synthesis method described in International Publication No. WO 90/07861, may be used as such gene synthesis methods.

### <Expression vector of present invention>

The present invention also provides an expression vector containing a polynucleotide containing a nucleotide sequence encoding the heavy chain variable region of the anti-MerTK antibody or the antigen binding fragment thereof of the present invention and/or a polynucleotide containing a nucleotide sequence encoding the light chain variable region of the anti-MerTK antibody or the antigen binding fragment thereof of the present invention (also referred to as the "expression vector of the present invention").

In one embodiment, the expression vector of the present invention can be an expression vector containing a polynucleotide containing a nucleotide sequence encoding the heavy chain variable region of the anti-MerTK antibody of the present invention, an expression vector containing a polynucleotide containing a nucleotide sequence encoding the light chain variable region of the anti-MerTK antibody of the present invention, or an expression vector containing a polynucleotide containing a nucleotide sequence encoding the heavy chain variable region of the anti-MerTK antibody of the present invention and a polynucleotide containing a nucleotide sequence encoding the light chain variable region.

In one embodiment, the expression vector of the present invention can be an expression vector containing a polynucleotide containing a nucleotide sequence encoding the heavy chain of the anti-MerTK antibody of the present invention, an expression vector containing a polynucleotide containing a nucleotide sequence encoding the light chain of the anti-MerTK antibody of the present invention, or an expression vector containing a polynucleotide containing a nucleotide sequence encoding the heavy chain of the anti-MerTK antibody of the present invention and a polynucleotide containing a nucleotide sequence encoding the light chain.

In one embodiment, the expression vector of the present invention contains a polynucleotide selected from the group consisting of the following (1) to (7):
(1) a polynucleotide containing a nucleotide sequence encoding a heavy chain variable region consisting of an amino acid sequence from amino acid positions 1 to 119 of SEQ ID NO: 8;
(2) a polynucleotide containing a nucleotide sequence encoding a light chain variable region consisting of an amino acid sequence from amino acid positions 1 to 109 of SEQ ID NO: 10;
(3) a polynucleotide containing a nucleotide sequence encoding a light chain variable region consisting of an amino acid sequence from amino acid positions 1 to 109 of SEQ ID NO: 12;
(4) a polynucleotide containing a nucleotide sequence encoding a light chain variable region consisting of an amino acid sequence from amino acid positions 1 to 109 of SEQ ID NO: 14;
(5) a polynucleotide containing a nucleotide sequence encoding a light chain variable region consisting of an amino acid sequence from amino acid positions 1 to 110 of SEQ ID NO: 16;
(6) a polynucleotide containing a nucleotide sequence encoding a light chain variable region consisting of an amino acid sequence from amino acid positions 1 to 110 of SEQ ID NO: 18; and
(7) a polynucleotide containing a nucleotide sequence encoding a light chain variable region consisting of an amino acid sequence from amino acid positions 1 to 110 of SEQ ID NO: 20.

In one embodiment, the expression vector of the present invention contains a polynucleotide selected from the group consisting of the following (1) to (7):
(1) a polynucleotide containing a nucleotide sequence encoding a heavy chain consisting of the amino acid sequence of SEQ ID NO: 8;
(2) a polynucleotide containing a nucleotide sequence encoding a light chain consisting of the amino acid sequence of SEQ ID NO: 10;
(3) a polynucleotide containing a nucleotide sequence encoding a light chain consisting of the amino acid sequence of SEQ ID NO: 12;
(4) a polynucleotide containing a nucleotide sequence encoding a light chain consisting of the amino acid sequence of SEQ ID NO: 14;
(5) a polynucleotide containing a nucleotide sequence encoding a light chain consisting of the amino acid sequence of SEQ ID NO: 16;
(6) a polynucleotide containing a nucleotide sequence encoding a light chain consisting of the amino acid sequence of SEQ ID NO: 18; and
(7) polynucleotide containing a nucleotide sequence encoding a light chain consisting of the amino acid sequence of SEQ ID NO: 20.

The expression vector of the present invention is not particularly limited as long as various host cells of eukaryotic cells (e.g., animal cells, insect cells, plant cells, or yeast) and/or prokaryotic cells (e.g., *E. coli*) can express the polynucleotide containing a nucleotide sequence encoding the heavy chain variable region of the anti-MerTK antibody or the antigen binding fragment thereof of the present invention and/or the polynucleotide containing a nucleotide sequence encoding the light chain variable region of the anti-MerTK antibody or the antigen binding fragment thereof of the present invention, and produce polypeptide(s) encoded thereby. Examples of such an expression vector include plasmid vectors and virus vectors (e.g., adenovirus and retrovirus). For example, pcDNA3.4 TOPO vector, pEE6.4, or pEE12.4 can be used. Alternatively, an antibody gene may be expressed by introducing variable region gene fragments to an expression vector having human Ig constant region genes in advance, such as AG-γ1 or AG-κ (see, for example, International Publication No. WO 94/20632).

The expression vector of the present invention may contain a promoter operably linked to the polynucleotide of the present invention. Examples of the promoter for expressing the polynucleotide of the present invention in animal cells include promoters derived from viruses such as CMV, RSV, and SV40, actin promoter, EF (elongation factor) 1α promoter, and heat shock promoter. Examples of the promoter for expression in a bacterium (e.g., a bacterium of the genus *Escherichia*) include trp promoter, lac promoter, λPL promoter, and tac promoter. Examples of the promoter for expression in a yeast include GAL1 promoter, GAL10 promoter, PH05 promoter, PGK promoter, GAP promoter, and ADH promoter.

In the case of using animal cells, insect cells, or a yeast as host cells, the expression vector of the present invention may contain a start codon and a stop codon. In this case, the expression vector of the present invention may contain, for example, an enhancer sequence, 5' and 3' untranslated regions of a gene encoding the antibody of the present invention or its heavy chain variable region or light chain variable region, a secretory signal sequence, a splicing junction, a polyadenylation site, or a replicable unit. In the case of using *E. coli* as host cells, the expression vector of the present invention may contain a start codon, a stop codon, a terminator region, and a replicable unit. In this case, the expression vector of the present invention may contain a selective marker (e.g., tetracycline resistance gene, ampicillin resistance gene, kanamycin resistance gene, neomycin resistance gene, or dihydrofolate reductase gene) usually used depending on a purpose.

### <Transformed host cell of present invention>

The present invention also provides a host cell transformed with the polynucleotide of the present invention or the expression vector of the present invention (also collectively referred to as the "polynucleotide, etc. of the present invention") (also referred to as the "host cell of the present invention"). The host cell of the present invention intracellularly contains the polynucleotide, etc. of the present invention. The introduced polynucleotide, etc. of the present invention may or may not be integrated into the genomic DNA of the host cell. Cells for use as the host cell may be any of cells culturable *in vitro* or *in vivo* cells. When the host cell is cells culturable *in vitro*, the host cell of the present invention can be prepared by introducing the polynucleotide, etc. of the present invention to the cells *in vitro*. A method for transforming the host cell is not particularly limited. For example, a method generally used by those skilled in the art, such as a calcium phosphate method, electroporation, or lipofection can be used. When the host cell is *in vivo* cells, a method for introducing the polynucleotide, etc. of the present invention to the host cell is not particularly limited. A carrier for nucleic acid delivery (cationic carrier or noncationic carrier (including, for example, but not limited to, liposome and lipid nanoparticles (LNP)), etc.) can be used.

The cells culturable *in vitro* are not particularly limited as long as they can be transformed with the expression vector used or by a method such as electroporation to express an antibody or a polypeptide. Examples of the cells culturable *in vitro* include various cells such as conventional cells usually used in the technical field of the present invention and artificially established cells (e.g., animal cells (e.g., CHO-K1 cells, ExpiCHO-S(R) cells, CHOK1SV cells, CHO-DG44 cells, HEK293 cells, Expi293F cells, and NS0 cells), insect cells (e.g., Sf9), bacteria (bacteria of the genus *Escherichia*, etc.), and yeasts (the genus *Saccharomyces* and the genus *Pichia*)). In one embodiment, the host cell of the present invention is Expi293F cells, CHO-K1 cells, or ExpiCHO-S cells.

The host cell transformed *in vitro* can be selected by a method generally used by those skilled in the art. For example, a cell isolation method such as a drug selection method using a drug selection marker gene and a drug such as tetracycline, ampicillin, neomycin, or hygromycin, a limiting dilution method, a single cell sorting method, or a colony pickup method can be used as the selection method.

In one embodiment, the host cell of the present invention contains polynucleotide(s) selected from the group consisting of the following (a) to (c):
(a) a polynucleotide containing a nucleotide sequence encoding the heavy chain variable region of the anti-MerTK antibody or the antigen binding fragment thereof of the present invention, and a polynucleotide containing a nucleotide sequence encoding the light chain variable region of the antibody or the antigen binding fragment thereof;
(b) a polynucleotide containing a nucleotide sequence encoding the heavy chain variable region of the anti-MerTK antibody or the antigen binding fragment thereof of the present invention; and
(c) a polynucleotide containing a nucleotide sequence encoding the light chain variable region of the anti-MerTK antibody or the antigen binding fragment thereof of the present invention.

In one embodiment, the host cell of the present invention contains polynucleotide(s) selected from the group consisting of the following (a) to (c):
(a) a polynucleotide containing a nucleotide sequence encoding the heavy chain of the anti-MerTK antibody of the present invention, and a polynucleotide containing a nucleotide sequence encoding the light chain of the antibody;
(b) a polynucleotide containing a nucleotide sequence encoding the heavy chain of the anti-MerTK antibody of the present invention; and
(c) a polynucleotide containing a nucleotide sequence encoding the light chain of the anti-MerTK antibody of the present invention.

In one embodiment, the host cell of the present invention contains a polynucleotide(s) selected from the group consisting of the following (1) to (13):
(1) a polynucleotide containing a nucleotide sequence encoding a heavy chain variable region consisting of an amino acid sequence from amino acid positions 1 to 119 of SEQ ID NO: 8;
(2) a polynucleotide containing a nucleotide sequence encoding a light chain variable region consisting of an amino acid sequence from amino acid positions 1 to 109 of SEQ ID NO: 10;
(3) a polynucleotide containing a nucleotide sequence encoding a light chain variable region consisting of an amino acid sequence from amino acid positions 1 to 109 of SEQ ID NO: 12;
(4) a polynucleotide containing a nucleotide sequence encoding a light chain variable region consisting of an amino acid sequence from amino acid positions 1 to 109 of SEQ ID NO: 14;
(5) a polynucleotide containing a nucleotide sequence encoding a light chain variable region consisting of an amino acid sequence from amino acid positions 1 to 110 of SEQ ID NO: 16;
(6) a polynucleotide containing a nucleotide sequence encoding a light chain variable region consisting of an amino acid sequence from amino acid positions 1 to 110 of SEQ ID NO: 18;
(7) a polynucleotide containing a nucleotide sequence encoding a light chain variable region consisting of an amino acid sequence from amino acid positions 1 to 110 of SEQ ID NO: 20;
(8) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 109 of SEQ ID NO: 10;
(9) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 109 of SEQ ID NO: 12;
(10) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 109 of SEQ ID NO: 14;
(11) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 110 of SEQ ID NO: 16;
(12) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 110 of SEQ ID NO: 18; and
(13) a polynucleotide containing a nucleotide sequence encoding a heavy chain variable region consisting of an amino acid sequence from amino acid positions 1 to 119 of SEQ ID NO: 8, and a polynucleotide containing a nucleotide sequence encoding a light chain variable region consisting of an amino acid sequence from amino acid positions 1 to 110 of SEQ ID NO: 20.

### <Method for producing anti-MerTK antibody of present invention>

The present invention also provide a method for producing the anti-MerTK antibody or the antigen binding fragment thereof of the present invention (also referred to as the "production method of the present invention"). The production method of the present invention may include a method for producing the polynucleotide of the present invention mentioned above, a method for producing the expression vector of the present invention, and a method for producing the host cell of the present invention. The production method of the present invention may also include the step of culturing the host cell described in the aforementioned section <Transformed host cell of present invention> so that the antibody is expressed into the cell or the culture supernatant, a method for recovering, isolating, and purifying the antibody, and the like. However, the production method of the present invention is not limited to these methods as long as the anti-MerTK antibody or the antigen binding fragment thereof of the present invention is produced.

In one embodiment, the production method of the present invention includes the step of culturing the host cell containing the polynucleotide containing a nucleotide sequence encoding the heavy chain variable region of the anti-MerTK antibody or the antigen binding fragment thereof of the present invention, and the polynucleotide containing a nucleotide sequence encoding the light chain variable region of the antibody or the antigen binding fragment thereof so that the anti-MerTK antibody is expressed.

The transformed host cell can be cultured by a known method. Culture conditions, for example, temperature, pH of a medium, and a culture time, are appropriately selected. When the host cell is animal cells, for example, MEM medium (Science, 1959, 130: 432-437), DMEM medium (Virology, 1959, 8: 396-397), RPMI1640 medium (J. Am. Med. Assoc., 1967, 199: 519), or 199 medium (Proc. Soc. Exp. Biol. Med., 1950, 73: 1-8) containing approximately 5 to 20% fetal bovine serum can be used as the medium. The pH of the medium is preferably approximately 6 to 8. The culture is usually performed at approximately 30 to 40°C for approximately 15 to 336 hours, if necessary, with aeration or stirring. When the host cell is insect cells, for example, Grace's medium (Proc. Natl. Acad. Sci. U.S.A., 1985, 82: 8404-8408) containing fetal bovine serum can be used as the medium. The pH of the medium is preferably approximately 5 to 8. The culture is usually performed at approximately 20 to 40°C for approximately 15 to 100 hours, if necessary, with aeration or stirring. When the host cell is *E. coli* or a yeast, for example, a liquid medium containing a nutrient is appropriate as the medium. The nutritive medium preferably contains a carbon source and an inorganic nitrogen source or an organic nitrogen source necessary for the growth of the transformed host cell. Examples of the carbon source include glucose, dextran, soluble starch, and sucrose. Examples of the inorganic nitrogen source or the organic nitrogen source include ammonium salts, nitrates, amino acids, corn steep liquor, peptone, casein, meat extracts, soybean cake, and potato extracts. Other nutrients (e.g., inorganic salts (e.g., calcium chloride, sodium dihydrogen phosphate, and magnesium chloride) and vitamins) and antibiotics (e.g., tetracycline, neomycin, ampicillin, and kanamycin) may be contained, if desired. The pH of the medium is preferably approximately 5 to 8. When the host cell is *E. coli*, for example, LB medium or M9 medium (Cold Spring Harb. Laboratory, 2001, 3: A2.2) can be used as a preferred medium. The culture is usually performed at approximately 14 to 39°C for approximately 3 to 24 hours, if necessary, with aeration or stirring. When the host cell is a yeast, for example, Burkholder minimum medium (Proc. Natl. Acad. Sci. U.S.A., 1980, 77: 4504-4508) can be used as the medium. The culture is usually performed at approximately 20 to 35°C for approximately 14 to 144 hours, if necessary, with aeration or stirring. The anti-MerTK antibody or the antigen binding fragment thereof of the present invention can be expressed by the culture as mentioned above.

The production method of the present invention can further include the step of recovering, for example, isolating or purifying the anti-MerTK antibody or the antigen binding fragment thereof from the transformed host cell, in addition to the step of culturing the transformed host cell of the present invention so that the anti-MerTK antibody or the antigen binding fragment thereof is expressed. Examples of the isolation or purification method include methods that exploit solubility, such as salting-out and solvent precipitation, methods that exploit difference in molecular weight, such as dialysis, ultrafiltration, and gel filtration, methods that exploit charge, such as ion exchange chromatography and hydroxyapatite chromatography, methods that exploit specific affinity, such as affinity chromatography, methods that exploit difference in hydrophobicity, such as reverse-phase high-performance liquid chromatography, and methods that exploit difference in isoelectric point, such as isoelectric focusing electrophoresis. For example, the antibody accumulated in the culture supernatant can be purified by each chromatography, for example, column chromatography using a protein A column or a protein G column.

The anti-MerTK antibody or the antigen binding fragment thereof of the present invention also includes the anti-MerTK antibody or the antigen binding fragment thereof produced by the production method of the present invention.

### <Fusion product of present invention, complex of present invention, and cell allowed to express anti-MerTK antibody or antigen binding fragment thereof of present invention on the cell surface>

The present invention also provides the anti-MerTK antibody or the antigen binding fragment thereof of the present invention linked to a protein (including an antibody) or a polypeptide other than MerTK (also referred to as the "fusion product of the present invention"). Specifically, as used herein, the "fusion product of the present invention" means the anti-MerTK antibody or the antigen binding fragment thereof of the present invention and a protein (including an antibody) or a polypeptide other than MerTK linked to each other. The protein or the polypeptide for use in the fusion product of the present invention is not particularly limited. For example, an antibody, an antigen binding fragment, a ligand, a receptor, a cytokine, a chemokine, a growth factor, a physiologically active substance, human serum albumin, various tag peptides, an artificial helix motif peptide, a maltose binding protein, glutathione S transferase, or other peptides or proteins capable of promoting multimerization may be used. In one embodiment, the fusion product of the present invention can be the protein or the polypeptide linked via a linker (e.g., a peptide linker) to the anti-MerTK antibody or the antigen binding fragment thereof of the present invention. In one embodiment, the protein or the polypeptide for use in the fusion product of the present invention can be, for example, an antibody or an antigen binding fragment thereof against a cell surface antigen of immunocytes such as T cells, B cells, natural killer (NK) cells, dendritic cells, granulocytes, macrophages, or mast cells, epithelial cells, fibroblasts, or RPE cells, or a polypeptide that activates immunocytes, such as each interleukin (e.g., IL-2, IL-7, IL-12, or IL-15). In this case, the protein or the polypeptide for use in the fusion product of the present invention may be linked directly or via an arbitrary linker (e.g., a peptide linker) to the anti-MerTK antibody or the antigen binding fragment thereof of the present invention.

The present invention also provides the anti-MerTK antibody or the antigen binding fragment thereof of the present invention bound to a carbohydrate, a lipid, a metal (including a radioisotope), an organic compound (including toxin, near-infrared fluorescent dye, and a chelating agent), or the like (also referred to as a "modifier") (also referred to as the "complex of the present invention"). Specifically, as used herein, the "complex of the present invention" means the anti-MerTK antibody or the antigen binding fragment thereof of the present invention and a substance other than the protein or the polypeptide linked to each other. As used herein, the "modifier" refers to a nonpeptidic substance bound directly or via a linker or the like to the antibody or the antigen binding fragment thereof. Examples of the modifier for use in the complex of the present invention include, but are not particularly limited to, polyethylene glycol, sugar chains, phospholipids, radioisotopes (e.g., zirconium-89 (89Zr), yttrium-90 (90Y), indium-111 (111In), astatine-211 (211At), and actinium-225 (225Ac)), organic compounds, toxins, near-infrared fluorescent dyes (e.g., IRDye(R)), and chelating agents. The substance for use in the complex may be bound directly or via an arbitrary linker to the anti-MerTK antibody or the antigen binding fragment thereof of the present invention. In one embodiment, the complex of the present invention is a drug conjugate of the anti-MerTK antibody or the antigen binding fragment thereof (antibody drug conjugate: ADC). The drug and the linker for use in ADC may be selected from among drugs and linkers generally used by those skilled in the art. In one embodiment, the complex of the present invention is a radioisotope-labeled antibody in which the anti-MerTK antibody or the antigen binding fragment thereof is bound to a radioisotope.

The complex of the present invention can also be used for the detection of a soluble or membrane-bound form of MerTK. As used herein, the "detection" encompasses quantitative or qualitative detection.

The present invention also provides a cell (e.g., a chimeric antigen receptor-T cell: CAR-T cell) allowed to express the anti-MerTK antibody or the antigen binding fragment thereof of the present invention on surface of the cell (e.g., an immunocompetent cell or an immune effector cells) (hereinafter, also referred to as the "presenting cell of the present invention"). A method for preparing the presenting cell of the present invention is not particularly limited. It can be prepared, for example, by using a polynucleotide encoding the anti-MerTK antibody or the antigen binding fragment thereof of the present invention so that the anti-MerTK antibody or the antigen binding fragment thereof is expressed on cell surface. Various cells (macrophages, alveolar macrophages, T cells, and NK cells, etc.) can be used as the cell allowed to express the anti-MerTK antibody or the antigen binding fragment thereof of the present invention.

The fusion product of the present invention, the complex of the present invention, and the presenting cell of the present invention are also collectively referred to as the "fusion product, etc. of the present invention".

In one embodiment, the antibody or the antigen binding fragment for use in the fusion product, etc. of the present invention may be modified post-translationally. In one embodiment, the post-translational modification is N-linked or O-linked glycosylation, N-terminal or C-terminal processing, deamidation, aspartic acid isomerization, and/or methionine oxidation.

Those skilled in the art are capable of readily preparing the anti-MerTK antibody or the antigen binding fragment thereof of the present invention or the fusion product, etc. of the present invention by using a method known in the art on the basis of sequence information on VH and VL of the anti-MerTK antibody or the antigen binding fragment thereof of the present invention and information on another peptide or protein (e.g., an antibody) for use in the fusion product of the present invention or the modifier for use in the complex of the present invention disclosed in the present specification. The anti-MerTK antibody or the antigen binding fragment thereof of the present invention can be produced in accordance with, for example, but not particularly limited to, a method described in the section <Method for producing anti-MerTK antibody of present invention>.

### <Pharmaceutical composition of present invention>

The present invention also provides a pharmaceutical composition comprising the anti-MerTK antibodyor the antigen binding fragment thereof of the present invention, or the fusion product, etc. of the present invention, and a pharmaceutically acceptable excipient (also referred to as the "pharmaceutical composition of the present invention"). The pharmaceutical composition of the present invention can be prepared by a method usually used with an excipient usually used in the art, i.e., a pharmaceutical excipient, a pharmaceutical carrier, or the like. Examples of the formulation of such a pharmaceutical composition include parenteral formulations such as injections, formulations for intravenous drips, and eye drops. Administration such as intraocular administration, intravitreal administration, retrobulbar administration, subconjunctival administration, sub-Tenon administration, subretinal administration, inhalation administration, intrathecal administration, intraperitoneal administration, intravenous administration, subcutaneous administration, intra-articular administration, administration by nasal drops, and intramuscular administration can be performed. For the production of preparations, excipients, carriers, additives, and the like appropriate for these formulations can be used in a pharmaceutically acceptable range.

The pharmaceutical composition of the present invention may contain a plurality of anti-MerTK antibodies or antigen binding fragments thereof of the present invention, or fusion products, etc. of the present invention, and post-translationally modified forms thereof. The present invention also includes a pharmaceutical composition containing, for example, an antibody that has undergone N-linked or O-linked glycosylation, N-terminal or C-terminal processing, deamidation, aspartic acid isomerization, and/or methionine oxidation.

In one embodiment, the pharmaceutical composition of the present invention is a pharmaceutical composition containing the anti-MerTK antibody or the antigen binding fragment thereof of the present invention, or the fusion product, etc. of the present invention, and/or a post-translationally modified form of the antibody or the antigen binding fragment thereof or the fusion product, etc. of the present invention containing any heavy chain variable region and light chain variable region of the following (a) to (f):
(a) a heavy chain variable region consisting of an amino acid sequence from amino acid positions 1 to 119 of SEQ ID NO: 8 and a light chain variable region consisting of an amino acid sequence from amino acid positions 1 to 109 of SEQ ID NO: 10;
(b) a heavy chain variable region consisting of an amino acid sequence from amino acid positions 1 to 119 of SEQ ID NO: 8 and a light chain variable region consisting of an amino acid sequence from amino acid positions 1 to 109 of SEQ ID NO: 12;
(c) a heavy chain variable region consisting of an amino acid sequence from amino acid positions 1 to 119 of SEQ ID NO: 8 and a light chain variable region consisting of an amino acid sequence from amino acid positions 1 to 109 of SEQ ID NO: 14;
(d) a heavy chain variable region consisting of an amino acid sequence from amino acid positions 1 to 119 of SEQ ID NO: 8 and a light chain variable region consisting of an amino acid sequence from amino acid positions 1 to 110 of SEQ ID NO: 16;
(e) a heavy chain variable region consisting of an amino acid sequence from amino acid positions 1 to 119 of SEQ ID NO: 8 and a light chain variable region consisting of an amino acid sequence from amino acid positions 1 to 110 of SEQ ID NO: 18; and
(f) a heavy chain variable region consisting of an amino acid sequence from amino acid positions 1 to 119 of SEQ ID NO: 8 and a light chain variable region consisting of an amino acid sequence from amino acid positions 1 to 110 of SEQ ID NO: 20.

In one embodiment, the pharmaceutical composition of the present invention is a pharmaceutical composition containing the anti-MerTK antibody of the present invention, or the fusion product, etc. of the present invention, and/or a post-translationally modified form of the antibody or the fusion product, etc. of the present invention containing any heavy chain and light chain of the following (a) to (f):
(a) a heavy chain consisting of the amino acid sequence of SEQ ID NO: 8 and a light chain consisting of the amino acid sequence represented by SEQ ID NO: 10;
(b) a heavy chain consisting of the amino acid sequence of SEQ ID NO: 8 and a light chain consisting of the amino acid sequence represented by SEQ ID NO: 12;
(c) a heavy chain consisting of the amino acid sequence of SEQ ID NO: 8 and a light chain consisting of the amino acid sequence represented by SEQ ID NO: 14;
(d) a heavy chain consisting of the amino acid sequence of SEQ ID NO: 8 and a light chain consisting of the amino acid sequence represented by SEQ ID NO: 16;
(e) a heavy chain consisting of the amino acid sequence of SEQ ID NO: 8 and a light chain consisting of the amino acid sequence represented by SEQ ID NO: 18; and
(f) a heavy chain consisting of the amino acid sequence of SEQ ID NO: 8 and a light chain consisting of the amino acid sequence represented by SEQ ID NO: 20.

The amount of the anti-MerTK antibody or the antigen binding fragment thereof of the present invention added for the production of preparations described above differs depending on the severity of symptoms or the age of a patient, the formulation of the preparation used, or the binding titer of the antibody, etc. For example, approximately 0.0001 mg/kg to 100 mg/kg can be used.

### <Pharmaceutical use of anti-MerTK antibody of present invention>

The anti-MerTK antibody or the antigen binding fragment thereof of the present invention, or the fusion product, etc. of the present invention, and the pharmaceutical compositions containing them can be used for the prevention or the treatment of diseases caused by the accumulation of apoptotic cells, debris derived from apoptotic cells, and/or cells, tissues, or debris highly expressing PtdSer. As used herein, the "debris" is conceptually discriminated from intact cells and refers to a mixture containing substances that have become unnecessary *in vivo*, such as cell fragments or/and body constituents. As used herein, the "debris derived from cells" refers to a mixture containing substances that have become unnecessary *in vivo*, such as cell fragments resulting from the fragmentation of intact cells, or/and body constituents. As used herein, the "cells, tissues, or debris highly expressing PtdSer" refers to cells, tissues, or debris containing PtdSer, except for apoptotic cells and debris derived from apoptotic cells.

In one embodiment, the anti-MerTK antibody or the antigen binding fragment thereof of the present invention, or the fusion product, etc. of the present invention, or the pharmaceutical compositions containing them can activate phagocytes via the activation of MerTK so that a material to be phagocytized can be phagocytized thereby.

As used herein, the "phagocytes" mean cells having a phagocytic clearance function; the "phagocytosis" means action by which a material to be phagocytized is taken up into the phagocytes; and the "phagocytic clearance function" means a function from the phagocytosis to the subsequent digestion and degradation of the taken-up material to be phagocytized. Examples of the phagocytes, the phagocytosis or/and phagocytic clearance function of which is enhanced or induced by the anti-MerTK antibody or the antigen binding fragment thereof of the present invention, or the fusion product, etc. of the present invention, or the pharmaceutical compositions containing them include macrophages, alveolar macrophages, dendritic cells, microglia, oligodendrocytes, epithelial cells, RPE cells, respiratory epithelial cells, Kupffer cells, hepatocytes, endothelial cells, neutrophils, monocytes, megakaryocytes, astrocytic cells, Langerhans cells, satellite cells, astrocytes, neural progenitor cells, podocytes, mesangium cells, CD103+ dendritic cells, CD11b+ macrophages, CD11b+ CD103+ macrophages, intestinal epithelial cells, and Sertoli cells (Nat. Rev. Mol. Cell Biol., 2020, 23: 398-414; and Cells, 2021, 10: 1443). In the present specification, the enhancement or induction of the phagocytosis or the phagocytic clearance function is also collectively referred to as the "activation" of the phagocytosis or the phagocytic clearance function. In one embodiment, the phagocytes that are activated by the anti-MerTK antibody or the antigen binding fragment thereof of the present invention, or the fusion product, etc. of the present invention, or the pharmaceutical compositions containing them are epithelial cells (e.g., RPE cells, respiratory epithelial cells, or intestinal epithelial cells). In one embodiment, the phagocytes that are activated by the anti-MerTK antibody or the antigen binding fragment thereof of the present invention, or the fusion product, etc. of the present invention, or the pharmaceutical compositions containing them are RPE cells. In the present specification, the "RPE cells" are cells that constitute retinal pigment epithelium (RPE). In one embodiment, the phagocytes that are activated by the anti-MerTK antibody or the antigen binding fragment thereof of the present invention, or the fusion product, etc. of the present invention, or the pharmaceutical compositions containing them are macrophages or tissue-resident macrophages (e.g., alveolar macrophages, microglia, or Kupffer cells). In one embodiment, the phagocytes that are activated by the anti-MerTK antibody or the antigen binding fragment thereof of the present invention, or the fusion product, etc. of the present invention, or the pharmaceutical compositions containing them are alveolar macrophages.

As used herein, the "material to be phagocytized" means a material that is subject to phagocytosis by phagocytes. The anti-MerTK antibody or the antigen binding fragment thereof of the present invention, or the fusion product, etc. of the present invention, or the pharmaceutical compositions containing them are capable of enhancing the clearance of the material to be phagocytized by activating the phagocytic clearance function of phagocytes. Examples of the material to be phagocytized include, but are not limited to, apoptotic cells, cancer cells, neutrophils, mast cells, myofibroblasts, adipose cells, debris derived from these cells, POS, and myelin debris.

In one embodiment, the material to be phagocytized is apoptotic cells. In one embodiment, the material to be phagocytized is debris derived from apoptotic cells. In one embodiment, the material to be phagocytized is cells, tissues, or debris highly expressing PtdSer. In one embodiment, the material to be phagocytized is POS. In one embodiment, the material to be phagocytized is myelin debris. In one embodiment, the material to be phagocytized is neutrophils. In one embodiment, the material to be phagocytized is debris derived from neutrophils.

The disease to be prevented or treated according to the present invention includes a disease caused by the accumulation of apoptotic cells, debris derived from apoptotic cells, or cells, tissues, or debris highly expressing PtdSer, or a disease derived from a decline in phagocytic clearance function. Examples of the disease caused by the accumulation of apoptotic cells, debris derived from apoptotic cells, or cells, tissues, or debris highly expressing PtdSer, or the disease derived from a decline in phagocytic clearance function include, but are not particularly limited to, various eye diseases, lung diseases, autoimmune diseases, neuropathic diseases, fibrotic diseases, circulatory organ diseases, and cancers.

In one embodiment, the disease to be prevented or treated according to the present invention is an eye disease. In one embodiment, the eye disease to be prevented or treated according to the present invention is a retinal disease. In one embodiment, the retinal disease to be prevented or treated according to the present invention is a retinal degenerative disease. In one embodiment, the retinal degenerative disease to be prevented or treated according to the present invention is retinitis pigmentosa, age-related macular degeneration, and/or diabetic retinopathy.

In one embodiment, the disease to be prevented or treated according to the present invention is a lung disease. In one embodiment, the lung disease to be prevented or treated according to the present invention is an acute lung disease. In one embodiment, the acute lung disease to be prevented or treated according to the present invention is acute respiratory distress syndrome and/or acute lung injury.

In one embodiment, the disease to be prevented or treated according to the present invention is an autoimmune disease. In one embodiment, the autoimmune disease to be prevented or treated according to the present invention is systemic lupus erythematosus. In one embodiment, the autoimmune disease to be prevented or treated according to the present invention is multiple sclerosis.

In one embodiment, the disease to be prevented or treated according to the present invention is rejection at the time of transplantation of various organs.

In one embodiment, the disease to be prevented or treated according to the present invention is a circulatory organ disease. In one embodiment, the circulatory organ disease to be prevented or treated according to the present invention is atherosclerosis. In one embodiment, the circulatory organ disease to be prevented or treated according to the present invention is an ischemic disease.

In one embodiment, the disease to be prevented or treated according to the present invention is a cancer. Examples of the cancer to be prevented or treated include, but are not particularly limited to, peritoneal dissemination of various cancer cells, stomach cancer, lung cancer, acute lymphoblastic leukemia (ALL), acute myeloid leukemia (AML), Hodgkin's lymphoma, non-Hodgkin's lymphoma, B cell lymphoma, multiple myeloma, T cell lymphoma, and other blood cancers, myelodysplastic syndrome, adenocarcinoma, squamous cell carcinoma, adenosquamous carcinoma, undifferentiated cancer, large-cell cancer, non-small cell lung cancer, small-cell lung cancer, mesothelioma, skin cancer, cutaneous T cell lymphoma, breast cancer, prostatic cancer, bladder cancer, vaginal cancer, neck cancer, head and neck cancer, uterine cancer, uterine cervical cancer, liver cancer, gallbladder cancer, bile duct cancer, kidney cancer, pancreatic cancer, colon cancer, large intestinal cancer, rectal cancer, small intestinal cancer, stomach cancer, esophageal cancer, testicle cancer, ovary cancer, brain tumor, and other solid cancers, and cancers of bone tissues, cartilage tissues, adipose tissues, muscular tissues, vascular tissues, and hematopoietic cancers as well as sarcoma such as chondrosarcoma, Ewing's sarcoma, malignant hemangioendothelioma, malignant schwannoma, osteosarcoma, and soft tissue sarcoma, and granuloma such as glioblastoma, glioblastoma multiforme, hepatoblastoma, medulloblastoma, nephroblastoma, neuroblastoma, pancreatoblastoma, pleuropulmonary blastoma, and retinoblastoma.

### <Diagnostic use of anti-MerTK antibody of present invention>

The anti-MerTK antibody or the antigen binding fragment thereof of the present invention, or the fusion product, etc. of the present invention, or a test kit for diagnosis containing it (hereinafter, also referred to as the "test kit of the present invention") can be used in the detection of MerTK. The test kit of the present invention may, if necessary, contain a detectable label, for example, an indicator enzyme, a radiolabel, a fluorescent dye molecule, or paramagnetic particles. The test kit of the present invention may contain a booklet for provision of information, for example, a booklet that shows procedures of carrying out the method described in the present specification using a reagent. In one embodiment, the anti-MerTK antibody of the present invention enables an inflammatory state to be diagnosed by detecting a soluble form of MerTK in a body fluid (blood, etc.). In one embodiment, the complex of the present invention enables an inflammatory state or a glycosylated state of MerTK to be diagnosed by detecting or analyzing a soluble form of MerTK in blood.

The present invention also includes a method for diagnosing, preventing, or treating a disease derived from the accumulation of apoptotic cells, debris derived from apoptotic cells, or cells, tissues, or debris highly expressing PtdSer or a disease derived from a decline in phagocytic clearance function, including the step of administering a diagnostically, prophylactically, or therapeutically effective amount of the anti-MerTK antibody or the antigen binding fragment thereof of the present invention, and/or the fusion product, etc. of the present invention to a subject. The present invention also includes the anti-MerTK antibody or the antigen binding fragment thereof of the present invention, or the fusion product, etc. of the present invention for use in the diagnosis, prevention, or treatment of diseases derived from the accumulation of apoptotic cells, debris derived from apoptotic cells, or cells, tissues, or debris highly expressing PtdSer or a disease derived from a decline in phagocytic clearance function. The present invention also includes use of the anti-MerTK antibody or the antigen binding fragment thereof of the present invention, or the fusion product, etc. of the present invention in the production of a pharmaceutical composition for the diagnosis, prevention, or treatment of diseases derived from the accumulation of apoptotic cells, debris derived from apoptotic cells, or cells, tissues, or debris highly expressing PtdSer or a disease derived from a decline in phagocytic clearance function.

### <Anti-MerTK bispecific antibody of present invention>

The present invention also provides a bispecific antibody (hereinafter, also referred to as the "anti-MerTK bispecific antibody of the present invention") containing the anti-MerTK antibody or the antigen binding fragment thereof of the present invention by linking the anti-MerTK antibody or the antigen binding fragment thereof to an antibody or an antigen binding fragment thereof against an arbitrary antigen other than MerTK (hereinafter, also collectively referred to as "another antibody"). The anti-MerTK bispecific antibody of the present invention contains a heavy chain variable region and a light chain variable region of the anti-MerTK antibody of the present invention and a heavy chain variable region and a light chain variable region of another antibody.

The anti-MerTK bispecific antibody of the present invention contains the following heavy chain variable region and light chain variable region of the anti-MerTK antibody:
a heavy chain variable region containing CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
a light chain variable region containing CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6.

In one embodiment, the anti-MerTK bispecific antibody of the present invention contains a heavy chain variable region and a light chain variable region of the anti-MerTK antibody selected from the group consisting of the following (1) to (6):
(1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
(2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
(3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20.

The anti-MerTK bispecific antibody of the present invention can have an arbitrary structure described in, for example, the literature (mAbs, 2017, 9: 182-212, Fig. 2). In one embodiment, the anti-MerTK bispecific antibody of the present invention may assume a symmetric or asymmetric structure. In one embodiment, the anti-MerTK bispecific antibody of the present invention is of antibody fusion type (e.g., IgG₂ type), variable region-alone type (e.g., DART type or Diabody type), CH1/CL fusion protein type (e.g., scFv₂-CH1/CL type), Fab fusion protein type (e.g., Fab-scFv type), non-immunoglobulin fusion protein type (e.g., scFv₂-albumin type), Fc region modified IgG type (e.g., IgG (kih) type, CrossMab (IgG-kih) type, or DuetMab type), added and Fc region modified IgG type (e.g., IgG (kih)-scFab type or CrossMab-Fab type), modified Fc region and CH3 fusion protein type (e.g., scFv-Fc (kih) type or Fab-scFv-Fc (kih) type), added IgGs-heavy chain fusion type (e.g., IgG-HC-scFv type), added IgGs-light chain fusion type, added IgGs-heavy chain and light chain fusion type, Fc region fusion type, CH3 fusion type, IgE/IgM and CH2 fusion type, F(ab')₂ fusion type, CH1/CL fusion protein type, modified IgG type, or non-immunoglobulin fusion type. In one embodiment, the anti-MerTK bispecific antibody of the present invention has an asymmetric structure.

In one embodiment, the anti-MerTK bispecific antibody of the present invention can have a structure where another antibody or an antigen binding fragment thereof is further linked to the N terminus or the C terminus of the heavy chain or the light chain of the anti-MerTK antibody of the present invention which is an IgG antibody. In one embodiment, the anti-MerTK bispecific antibody of the present invention has a structure where another antibody or an antigen binding fragment thereof is further linked to the N terminus of the heavy chain, the N-terminus of a second Fc polypeptide, the C-terminus of an Fc region, or the N terminus or the C terminus of the light chain in the anti-MerTK antibody of the present invention which is a one-armed antibody. In one embodiment, the anti-MerTK bispecific antibody of the present invention has a structure where another antibody or an antigen binding fragment thereof is further linked via a hinge region to the N terminus of the second Fc polypeptide of the anti-MerTK antibody of the present invention which is a one-armed antibody.

In one embodiment, the anti-MerTK bispecific antibody of the present invention can be of DuetMab type, CrossMab type (IgG-kih) type, Fab-scFv-Fc (kih) type, IgG(kih)-scFab type, CrossMab-Fab type, or IgG-HC-scFv type.

In one embodiment, the anti-MerTK bispecific antibody of the present invention can be of F(ab')₂ fusion type, Diabody type, DART type, or Fab-scFv type.

In one embodiment, the antigen binding fragment of the anti-MerTK antibody of the present invention contained in the anti-MerTK bispecific antibody of the present invention can be scFv, a Fab fragment, a Fab' fragment, a F(ab')₂ fragment, or a VHH antibody.

In one embodiment, the antigen binding fragment of another antibody contained in the anti-MerTK bispecific antibody of the present invention can be scFv, a Fab fragment, a Fab' fragment, a F(ab')₂ fragment, or a VHH antibody.

In one embodiment, the anti-MerTK bispecific antibody of the present invention contains a heavy chain fragment containing the heavy chain variable region of the anti-MerTK antibody, and a light chain containing the light chain variable region of the anti-MerTK antibody. In one embodiment, the anti-MerTK bispecific antibody of the present invention contains a one-armed antibody containing a heavy chain fragment containing the heavy chain variable region and a light chain containing the light chain variable region of the anti-MerTK antibody, and one Fc region consisting of a first Fc polypeptide and a second Fc polypeptide (hereinafter, also referred to as a "one-armed anti-MerTK antibody"), and a heavy chain variable region and a light chain variable region of another antibody. In one embodiment, the anti-MerTK bispecific antibody of the present invention contains the one-armed anti-MerTK antibody, and a scFv or a Fab region containing the heavy chain variable region and the light chain variable region of another antibody. In one embodiment, the anti-MerTK bispecific antibody of the present invention is a bispecific antibody in which the C terminus of the scFv or the Fab region containing the heavy chain variable region and the light chain variable region of another antibody is linked via a hinge region to the N terminus of the second Fc polypeptide of the one-armed anti-MerTK antibody. In one embodiment, the anti-MerTK bispecific antibody of the present invention is of Fab-scFv-Fc (kih) type. In one embodiment, the anti-MerTK bispecific antibody of the present invention is of CrossMab (IgG-kih) type.

In one embodiment, the anti-MerTK bispecific antibody of the present invention contains a one-armed anti-MerTK antibody containing a heavy chain fragment and a light chain selected from the group consisting of the following (1) to (6):
(1) a heavy chain fragment consisting of an amino acid sequence from amino acid positions 1 to 217 of SEQ ID NO: 8, and a light chain consisting of the amino acid sequence of SEQ ID NO: 10;
(2) a heavy chain fragment consisting of an amino acid sequence from amino acid positions 1 to 217 of SEQ ID NO: 8, and a light chain consisting of the amino acid sequence of SEQ ID NO: 12;
(3) a heavy chain fragment consisting of an amino acid sequence from amino acid positions 1 to 217 of SEQ ID NO: 8, and a light chain consisting of the amino acid sequence of SEQ ID NO: 14;
(4) a heavy chain fragment consisting of an amino acid sequence from amino acid positions 1 to 217 of SEQ ID NO: 8, and a light chain consisting of the amino acid sequence of SEQ ID NO: 16;
(5) a heavy chain fragment consisting of an amino acid sequence from amino acid positions 1 to 217 of SEQ ID NO: 8, and a light chain consisting of the amino acid sequence of SEQ ID NO: 18; and
(6) a heavy chain fragment consisting of an amino acid sequence from amino acid positions 1 to 217 of SEQ ID NO: 8, and a light chain consisting of the amino acid sequence of SEQ ID NO: 20.

The anti-MerTK bispecific antibody of the present invention is not limited by the structures described above and can be a bispecific antibody containing a monovalent or multivalent anti-MerTK antibody or antigen binding fragment thereof and a monovalent or multivalent antibody or antigen binding fragment thereof against an antigen other than MerTK. Also, a multispecific antibody such as a trispecific antibody or a tetraspecific antibody can be provided by further combining the anti-MerTK bispecific antibody of the present invention with antibodies against third and fourth antigens.

The type and length of a linker for use in the bispecific antibody of the present invention are not particularly limited and may be appropriately selected by those skilled in the art. For example, when the anti-MerTK antibody of the present invention or another antibody thereof is a scFv, the length of the peptide linker that links the heavy chain variable region and the light chain variable region of the antibody is preferably 5 or more amino acids (the upper limit is not particularly limited and is usually 30 or less amino acids, preferably 20 or less amino acids), particularly preferably 15 amino acids. For example, a glycine-serine linker (GS linker) or a glycine-lysine-proline-glycine-serine linker (GKPGS linker) can be used as the peptide linker. Examples of such a linker include the following:
Ser,
Gly-Ser,
Gly-Gly-Ser,
Ser-Gly-Gly,
Gly-Gly-Gly-Ser (SEQ ID NO: 46),
Ser-Gly-Gly-Gly (SEQ ID NO: 47),
Gly-Gly-Gly-Gly-Ser (SEQ ID NO: 48),
Ser-Gly-Gly-Gly-Gly (SEQ ID NO: 49),
Gly-Gly-Gly-Gly-Gly-Ser (SEQ ID NO: 50),
Ser-Gly-Gly-Gly-Gly-Gly (SEQ ID NO: 51),
Gly-Gly-Gly-Gly-Gly-Gly-Ser (SEQ ID NO: 52),
Ser-Gly-Gly-Gly-Gly-Gly-Gly (SEQ ID NO: 53),
(Gly-Gly-Gly-Gly-Ser)n (in the case of n = 3, SEQ ID NO: 54),
(Ser-Gly-Gly-Gly-Gly)n,
Gly-Lys-Pro-Gly-Ser (SEQ ID NO: 55), and
(Gly-Lys-Pro-Gly-Ser)n.

n described above represents an integer of 1 or larger. The length or the sequence of the peptide linker can be appropriately selected by those skilled in the art depending on a purpose.

In one embodiment, when the anti-MerTK bispecific antibody of the present invention contains scFv of another antibody, the peptide linker that links the heavy chain variable region and the light chain variable region of another antibody has the amino acid sequence set forth in SEQ ID NO: 48. In one embodiment, the peptide linker that links the heavy chain variable region and the light chain variable region of another antibody has an amino acid sequence (Gly-Gly-Gly-Gly-Ser)n. In one embodiment, the peptide linker that links the heavy chain variable region and the light chain variable region of another antibody has the amino acid sequence set forth in SEQ ID NO: 54.

When the anti-MerTK bispecific antibody of the present invention contains the Fc region, the Fc region in the bispecific antibody may contain a mutation that reduces antibody-dependent cellular cytotoxicity (ADCC), antibody-dependent cellular phagocytosis (ADCP), or complement-dependent cytotoxicity (CDC). In one embodiment, the anti-MerTK bispecific antibody of the present invention contains an Fc region containing L234A and L235A amino acid mutations (LALA mutations) (wherein positions of the mutations are amino acid positions that are numbered according to the EU index in a human Igγ1 constant region). In one embodiment, the anti-MerTK bispecific antibody of the present invention contains an Fc region containing a P329A mutation or a P331G mutation (wherein positions of the mutations are amino acid positions that are numbered according to the EU index in a human Igγ1 constant region). In one embodiment, the anti-MerTK bispecific antibody of the present invention contains an Fc region containing L234A and L235A amino acid mutations (LALA mutations) and a P331G mutation (wherein positions of the mutations are amino acid positions that are numbered according to the EU index in a human Igγ1 constant region). In one embodiment, the anti-MerTK bispecific antibody of the present invention contains an Fc region containing knobs-into-holes mutations. In one embodiment, the anti-MerTK bispecific antibody of the present invention contains an Fc region containing LALA mutations, a P331G mutation, and knobs-into-holes mutations. In one embodiment, the knobs-into-holes mutations contained in the anti-MerTK bispecific antibody of the present invention are a T366W mutation in one of the Fc polypeptides constituting the Fc region, and T366S, L368A, and Y407V mutations in the other Fc polypeptide constituting the Fc region (wherein positions of the mutations are amino acid positions that are numbered according to the EU index in a human Igγ1 constant region). The knobs-into-holes mutations contained in the anti-MerTK bispecific antibody of the present invention may be a T366W mutation contained in the first Fc polypeptide and T366S, L368A, and Y407V mutations contained in the second Fc polypeptide, or may be T366S, L368A, and Y407V mutations contained in the first Fc polypeptide and a T366W mutation contained in the second Fc polypeptide.

Examples of another antibody or the antigen binding fragment include, but are not particularly limited to, antibodies or antigen binding fragments against misfolded proteins, amyloid, myelin debris, Aβ, immunocytes (e.g., T cells), receptors expressed on immunocytes, or tumor-associated antigens on cancer cells.

In one embodiment, the anti-MerTK bispecific antibody of the present invention is a bispecific antibody that binds to MerTK and a misfolded protein and/or amyloid (hereinafter, also referred to as the "anti-MerTK-anti-misfolded protein bispecific antibody of the present invention"). As used herein, "misfolded" or "have undergone misfolding" means that correct folding has not been made *in vivo*. The "misfolded protein" is a protein that has undergone misfolding and lacks its original function, and the "amyloid" is an aggregate of the misfolded protein.

For example, immunoglobulin light chain (IgL), IgH, SAA, TTR, β2 microglobulin, apolipoprotein AI, apolipoprotein AII, apolipoprotein AIV, apolipoprotein CII, apolipoprotein CIII, gelsolin, lysozyme, LECT2, fibrinogen α chain, cystatin C, ABri (pro)protein, ADan (pro)protein, Aβ proprotein, α synuclein, tau protein, prion protein, TMEM106B, (pro)calcitonin, amylin, ANP, prolactin, (pro)somatostatin, glucagon, PTH, insulin, Enfuvritide, GLP1, IL1RAP, pulmonary surfactant protein, corneodesmosin, MFG-E8, keratoepithelin, lactoferrin, OAAP, semenogelin 1, cathepsin K, EFEMP1, huntingtin, myelin, TDP43, and SOD1 are known as proteins known to become misfolded proteins (Amyloid, 2022, 4: 213-219; J. Neurosci., 2006, 26: 328-332; and Neurobiology of Disease, 2023, 184: 106218). In one embodiment, the protein that becomes the misfolded protein is TTR. In one embodiment, the protein that becomes the misfolded protein is IgL.

The anti-MerTK-anti-misfolded protein bispecific antibody of the present invention contains a heavy chain variable region and a light chain variable region of the anti-MerTK antibody of the present invention, and a heavy chain variable region and a light chain variable region of an antibody that binds to the misfolded protein and/or the amyloid. The anti-MerTK-anti-misfolded protein bispecific antibody of the present invention contains the following heavy chain variable region and light chain variable region of the anti-MerTK antibody:
a heavy chain variable region containing CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
a light chain variable region containing CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6.

In one embodiment, the anti-MerTK-anti-misfolded protein bispecific antibody of the present invention contains a heavy chain variable region and a light chain variable region of the anti-MerTK antibody selected from the group consisting of the following (1) to (6):
(1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
(2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
(3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20.

In one embodiment, the anti-MerTK-anti-misfolded protein bispecific antibody of the present invention contains a heavy chain fragment containing the heavy chain variable region of the anti-MerTK antibody and a light chain containing the light chain variable region of the anti-MerTK antibody. In one embodiment, the anti-MerTK-anti-misfolded protein bispecific antibody of the present invention contains a one-armed anti-MerTK antibody containing a heavy chain fragment containing the heavy chain variable region and a light chain containing the light chain variable region of the anti-MerTK antibody, and one Fc region consisting of a first Fc polypeptide and a second Fc polypeptide. In a further embodiment, the one-armed anti-MerTK antibody contained in the anti-MerTK-anti-misfolded protein bispecific antibody of the present invention contains one heavy chain in which the heavy chain fragment containing the heavy chain variable region of the anti-MerTK antibody is linked via a hinge region to the N terminus of the first Fc polypeptide. In a further embodiment, the heavy chain fragment containing the heavy chain variable region of the anti-MerTK antibody is linked via a hinge region to the N terminus of the first Fc polypeptide. In one embodiment, the anti-MerTK-anti-misfolded protein bispecific antibody of the present invention is a bispecific antibody containing a one-armed anti-MerTK antibody containing a heavy chain fragment containing the heavy chain variable region and a light chain containing the light chain variable region of the anti-MerTK antibody, and one Fc region consisting of a first Fc polypeptide and a second Fc polypeptide, wherein the one-armed anti-MerTK antibody contains one heavy chain in which the heavy chain fragment is linked via a hinge region to the N terminus of the first Fc polypeptide. In one embodiment, the anti-MerTK-anti-misfolded protein bispecific antibody of the present invention is a bispecific antibody containing a one-armed anti-MerTK antibody containing a heavy chain fragment containing the heavy chain variable region and a light chain containing the light chain variable region of the anti-MerTK antibody, and one Fc region consisting of a first Fc polypeptide and a second Fc polypeptide, wherein the heavy chain fragment is linked via a hinge region to the N terminus of the first Fc polypeptide.

In one embodiment, the anti-MerTK-anti-misfolded protein bispecific antibody of the present invention contains a one-armed anti-MerTK antibody containing a heavy chain fragment and a light chain selected from the group consisting of the following (1) to (6):
(1) a heavy chain fragment consisting of an amino acid sequence from amino acid positions 1 to 217 of SEQ ID NO: 8, and a light chain consisting of the amino acid sequence of SEQ ID NO: 10;
(2) a heavy chain fragment consisting of an amino acid sequence from amino acid positions 1 to 217 of SEQ ID NO: 8, and a light chain consisting of the amino acid sequence of SEQ ID NO: 12;
(3) a heavy chain fragment consisting of an amino acid sequence from amino acid positions 1 to 217 of SEQ ID NO: 8, and a light chain consisting of the amino acid sequence of SEQ ID NO: 14;
(4) a heavy chain fragment consisting of an amino acid sequence from amino acid positions 1 to 217 of SEQ ID NO: 8, and a light chain consisting of the amino acid sequence of SEQ ID NO: 16;
(5) a heavy chain fragment consisting of an amino acid sequence from amino acid positions 1 to 217 of SEQ ID NO: 8, and a light chain consisting of the amino acid sequence of SEQ ID NO: 18; and
(6) a heavy chain fragment consisting of an amino acid sequence from amino acid positions 1 to 217 of SEQ ID NO: 8, and a light chain consisting of the amino acid sequence of SEQ ID NO: 20.

In one embodiment, the anti-MerTK-anti-misfolded protein bispecific antibody of the present invention contains the one-armed anti-MerTK antibody, and scFv or a Fab region containing the heavy chain variable region and the light chain variable region of the antibody that binds to the misfolded protein and/or the amyloid. In one embodiment, the anti-MerTK-anti-misfolded protein bispecific antibody of the present invention is a bispecific antibody in which the scFv or the Fab region containing the heavy chain variable region and the light chain variable region of the antibody that binds to the misfolded protein and/or the amyloid is further linked via a hinge region to the N terminus of the second Fc polypeptide of the one-armed anti-MerTK antibody.

In one embodiment, the anti-MerTK-anti-misfolded protein bispecific antibody of the present invention contains an antibody that binds to a misfolded protein, myelin debris, or Aβ of a protein, or amyloid thereof, or an antigen binding fragment thereof. Examples of the protein that forms a misfolded protein, myelin debris, or Aβ, or amyloid thereof include IgL, IgH, SAA, TTR, β2 microglobulin, apolipoprotein AI, apolipoprotein AII, apolipoprotein AIV, apolipoprotein CII, apolipoprotein CIII, gelsolin, lysozyme, LECT2, fibrinogen α chain, cystatin C, ABri (pro)protein, ADan (pro)protein, Aβ, Aβ proprotein, α synuclein, tau protein, prion protein, TMEM106B, (pro)calcitonin, amylin, ANP, prolactin, (pro)somatostatin, glucagon, PTH, insulin, Enfuvritide, GLP1, IL1RAP, pulmonary surfactant protein, corneodesmosin, MFG-E8, keratoepithelin, lactoferrin, OAAP, semenogelin 1, cathepsin K, EFEMP1, huntingtin, myelin debris, TDP43, and SOD1.

In one embodiment, another antibody for use in the anti-MerTK-anti-misfolded protein bispecific antibody of the present invention can be an anti-SAA antibody, an anti-amylin antibody, an anti-Aβ antibody, an anti-huntingtin antibody, an anti-TTR antibody, or an anti-IgL antibody, or an antigen binding fragment thereof. In one embodiment, another antibody for use in the anti-MerTK-anti-misfolded protein bispecific antibody of the present invention can be an anti-TTR antibody that selectively binds to misfolded TTR or an anti-IgL antibody that selectively binds to misfolded IgL.

In one embodiment, the anti-MerTK-anti-misfolded protein bispecific antibody of the present invention is a bispecific antibody that contains the anti-MerTK antibody or the antigen binding fragment thereof of the present invention and an anti-TTR antibody or an antigen binding fragment thereof and has binding activity against MerTK and misfolded TTR (in the present specification, referred to as an "anti-MerTK-anti-TTR bispecific antibody"). In one embodiment, the anti-MerTK-anti-misfolded protein bispecific antibody of the present invention is a bispecific antibody that contains the anti-MerTK antibody or the antigen binding fragment thereof of the present invention and an anti-IgL antibody or an antigen binding fragment thereof and has binding activity against MerTK and misfolded IgL (in the present specification, referred to as an "anti-MerTK-anti-IgL bispecific antibody").

In one embodiment, the anti-MerTK bispecific antibody of the present invention contains an antibody or an antigen binding fragment against T cell receptor.

In one embodiment, the anti-MerTK bispecific antibody of the present invention can be a bispecific antibody containing antibodies or antigen binding fragments that bind to MerTK and tumor-associated antigen (TAA) (hereinafter, referred to as the "anti-MerTK-anti-TAA bispecific antibody of the present invention").

Examples of the antibody or the antigen binding fragment thereof for use in the anti-MerTK-anti-TAA bispecific antibody of the present invention include HER2, HER3, HER4, CD19, CD20, CD22, CD33, CD38, CD40, CD44, CD70, CD123, CD138, CXCR3, CXCR5, CCR3, CCR4, CCR9, CRTH2, PMCH, CD4, CD25, CD200, endoplasmin, BCMA, CD276, TSPAN8, CLDN4, CLDN6, CLDN18.2, ENPP3, SLC34A2, TROP2, Nectin-4, ASGR1, mesothelin, PSMA, LIV-1, MUC1, MUC2, MUC4, MUC16, CDH6, CEACAM1, CEACAM3, CEACAM4, CEACAM5, CEACAM6, CEACAM7, CEACAM16, CEACAM18, CEACAM19, CEACAM20, CEACAM21, URLC10, NY-ESO-1, GAA, OFA, cyclinB1, WT-1, CEF, VEGFR1, VEGFR2, TTK, HPV16, HPV16E7, HPV18, CEAIMA910, KOC1, SL-701, MART-1, gp100, tyrosinase, survivin, MAGE-3.1, MAGE-10.A2, OVA BiP, gp209-2M, melan-A, NA17.A2, KOC1, CO16, DEPDC1, MPHOSPH1, MAGE12, ONT-10, GD2L, GD3L, URLC10, CDCA1, TF, PSA, TERT, STF-II, G17DT, ICT-107, Dex2, hTERT, PAP, TRP2, LRRC15, c-Met, PD-L1, FAP, EGFR, B3-H3, GPC3, and 5T4.

The anti-MerTK bispecific antibody of the present invention may contain a mutation that reduces ADCC, CDC, or ADCP and/or a mutation that promotes the formation of a heterodimer. The mutations are in accordance with the description of the aforementioned section <Anti-MerTK antibody of present invention>.

In one embodiment, the anti-MerTK bispecific antibody of the present invention may be modified post-translationally. The post-translational modification is in accordance with the description of the aforementioned section <Anti-MerTK antibody of present invention>. In one embodiment, the post-translational modification is N-linked or O-linked glycosylation, N-terminal or C-terminal processing, deamidation, aspartic acid isomerization, and/or methionine oxidation.

Those skilled in the art are capable of preparing the anti-MerTK bispecific antibody of the present invention by using a method known in the art on the basis of sequence information or the like on the heavy chain variable region and the light chain variable region of the anti-MerTK antibody of the present invention disclosed in the present specification, and sequence information or the like on known another antibody or antigen binding fragment.

### < Fusion product or complex of anti-MerTK bispecific antibody of present invention, or cell allowed to express anti-MerTK bispecific antibody of present invention on the cell surface>

The anti-MerTK bispecific antibody of the present invention provides a fusion product of the anti-MerTK bispecific antibody of the present invention linked to a protein (including a polypeptide) other than MerTK and the antigen to which another antibody binds, and an antibody (including an antigen binding fragment) other than the anti-MerTK antibody and another antibody. The anti-MerTK bispecific antibody of the present invention also provides a complex of the anti-MerTK bispecific antibody of the present invention bound to a modifier. The anti-MerTK bispecific antibody of the present invention further provides a cell (e.g., Tandem CAR) allowed to express the anti-MerTK bispecific antibody of the present invention on surface of the cell (e.g., an immunocompetent cell or an effector cell) (in the present specification, referred to as a "cell allowed to express the anti-MerTK bispecific antibody of the present invention on the cell surface") (Oncology Reports, 2019, 42: 2183-2195). The fusion product, the complex, and the cell allowed to express the antibody on the cell surface are in accordance with the description of the aforementioned section <Fusion product of present invention, complex of present invention, and cell allowed to express anti-MerTK antibody or antigen binding fragment thereof of present invention on the cell surface>.

### <Polynucleotide of anti-MerTK bispecific antibody of present invention, expression vector, transformed host cell, and production method>

The present invention also provides a polynucleotide for use in the production of the anti-MerTK bispecific antibody of the present invention. Those skilled in the art are capable of implementing the polynucleotide in accordance with the description of the aforementioned section <Polynucleotide of present invention> and the after-mentioned section <Polynucleotide of anti-MerTK-anti-TTR bispecific antibody of present invention>.

The present invention also provides an expression vector containing the polynucleotide of the anti-MerTK bispecific antibody of the present invention and a method for producing the anti-MerTK bispecific antibody of the present invention. Those skilled in the art are capable of implementing these in accordance with the aforementioned sections

### <Expression vector of present invention> and <Method for producing anti-MerTK antibody of present invention>.

The present invention also provides a transformed host cell for the anti-MerTK bispecific antibody of the present invention. Those skilled in the art are capable of implementing the host cell in accordance with the aforementioned section <Transformed host cell of present invention> and the after-mentioned section <Transformed host cell for MerTK-anti-TTR bispecific antibody of present invention>.

### <Pharmaceutical use of anti-MerTK bispecific antibody of present invention>

The present invention also provides a pharmaceutical composition containing the anti-MerTK bispecific antibody of the present invention and a pharmaceutically acceptable excipient. Those skilled in the art are capable of implementing the pharmaceutical composition in accordance with the description of the aforementioned section

### <Pharmaceutical composition of present invention>.

The anti-MerTK bispecific antibody of the present invention or the pharmaceutical composition containing the antibody can be used for the prevention or the treatment of diseases caused by the accumulation of a misfolded protein.

The anti-MerTK bispecific antibody of the present invention or the pharmaceutical composition containing it binds to a phagocyte and a material to be phagocytized, puts the phagocyte and the material to be phagocytized physically close to each other, and activates the phagocyte via the activation of MerTK so that the material to be phagocytized can be efficiently taken up into the phagocyte to be digested.

The phagocyte that is activated by the anti-MerTK bispecific antibody of the present invention is in accordance with the description of the aforementioned section <Pharmaceutical use of anti-MerTK antibody of present invention>. The material to be phagocytized, the clearance of which is enhanced through the activation of the phagocytic clearance function of the phagocyte by the anti-MerTK bispecific antibody of the present invention includes a misfolded protein, amyloid and/or cancer cells. In one embodiment, the material to be phagocytized is a misfolded protein. In one embodiment, the material to be phagocytized is amyloid. In one embodiment, the material to be phagocytized is TTR that has undergone misfolding (hereinafter, referred to as "misfolded TTR"). In one embodiment, the material to be phagocytized is IgL that has undergone misfolding (hereinafter, referred to as "misfolded IgL"). In one embodiment, the material to be phagocytized is myelin debris. In one embodiment, the material to be phagocytized is Aβ.

The anti-MerTK bispecific antibody of the present invention or the pharmaceutical composition containing the antibody can be used for the prevention or the treatment of diseases derived from the accumulation of a misfolded protein, a disease derived from the accumulation of amyloid, or a disease derived from a decline in phagocytic clearance function. The present invention also includes a method for preventing or treating a disease derived from the accumulation of a misfolded protein, a disease derived from the accumulation of amyloid, or a disease derived from a decline in phagocytic clearance function, containing the step of administering a therapeutically effective amount of the anti-MerTK bispecific antibody of the present invention to a subject. The present invention also includes the anti-MerTK bispecific antibody of the present invention for use in the prevention or the treatment of diseases derived from the accumulation of a misfolded protein, a disease derived from the accumulation of amyloid, or a disease derived from a decline in phagocytic clearance function. The present invention also includes use of the anti-MerTK bispecific antibody of the present invention in the production of a pharmaceutical composition for the prevention or the treatment of diseases derived from the accumulation of a misfolded protein, a disease derived from the accumulation of amyloid, or a disease derived from a decline in phagocytic clearance function. Examples of the disease derived from the accumulation of a misfolded protein, the disease derived from the accumulation of amyloid, or the disease derived from a decline in phagocytic clearance function to be prevented or treated according to the present invention include, but are not particularly limited to, various amyloidoses, heart diseases, neuropathic diseases, eye diseases, and endocrine/metabolic diseases. In one embodiment, the disease to be prevented or treated with the anti-MerTK bispecific antibody of the present invention or the pharmaceutical composition containing it is amyloidosis, a heart disease, a neuropathic disease, an eye disease, or an endocrine/metabolic disease.

In one embodiment, the disease to be prevented or treated with the anti-MerTK bispecific antibody of the present invention or the pharmaceutical composition containing the antibody is amyloidosis (e.g., localized amyloidosis or systemic amyloidosis). In one embodiment, the amyloidosis to be prevented or treated with the anti-MerTK bispecific antibody of the present invention or the pharmaceutical composition containing the antibody is localized amyloidosis. The localized amyloidosis to be prevented or treated with the anti-MerTK bispecific antibody of the present invention or the pharmaceutical composition containing the antibody is Alzheimer's disease, cerebral amyloid angiopathy, Creutzfeldt-Jakob disease, hereditary cerebral amyloid angiopathy, familial British dementia, familial Danish dementia, isolated atrial amyloidosis, prolactinoma, insulin amyloidosis, corneal amyloidosis, aortal medial amyloidosis, or localized nodular amyloidosis (Circ. J., 2020, 84: 1610-1671). In one embodiment, the amyloidosis to be prevented or treated with the anti-MerTK bispecific antibody of the present invention or the pharmaceutical composition containing the antibody is systemic amyloidosis (e.g., transthyretin amyloid cardiomyopathy (ATTR-CM) or amyloid polyneuropathy (ATTR-PN), AL amyloidosis, AH amyloidosis, Aβ2M amyloidosis (dialysis-related amyloidosis), AA amyloidosis, AApoAI amyloidosis, AApoAII amyloidosis, AApoAIV amyloidosis, AApoCII amyloidosis, AApoCIII amyloidosis, AGel amyloidosis, ALys amyloidosis, ALECT2 amyloidosis, AFib amyloidosis, ACys amyloidosis, ABri amyloidosis, APrP amyloidosis, or ACal amyloidosis) (Heart Int., 2023, 17: 27-35; Amyloid, 2022, 4: 213-219; and Lancet, 2016, 387: 2641-2654). In one embodiment, the systemic amyloidosis to be prevented or treated with the anti-MerTK bispecific antibody of the present invention or the pharmaceutical composition containing the antibody is ATTR-CM and ATTR-PN. In one embodiment, the systemic amyloidosis to be prevented or treated with the anti-MerTK bispecific antibody of the present invention or the pharmaceutical composition containing the antibody is AL amyloidosis.

In one embodiment, the disease to be prevented or treated with the anti-MerTK bispecific antibody of the present invention or the pharmaceutical composition containing the antibody is a neuropathic disease. In one embodiment, the neuropathic disease to be prevented or treated with the anti-MerTK bispecific antibody of the present invention or the pharmaceutical composition containing the antibody is multiple sclerosis or/and Alzheimer's disease.

The anti-MerTK bispecific antibody of the present invention or the pharmaceutical composition containing the antibody can also be used for the prevention or the treatment of a cancer, and those skilled in the art is capable of implementing it in accordance with the description of the aforementioned section <Pharmaceutical use of present invention>. Examples of the cancer to be prevented or treated with the anti-MerTK bispecific antibody of the present invention or the pharmaceutical composition containing the antibody include the cancers described in the aforementioned section <Pharmaceutical use of present invention>.

### <Anti-MerTK-anti-TTR bispecific antibody of present invention>

The present invention provides an anti-MerTK-anti-TTR bispecific antibody that binds to MerTK and misfolded TTR.

The anti-MerTK-anti-TTR bispecific antibody of the present invention contains a heavy chain variable region and a light chain variable region of an anti-MerTK antibody and a heavy chain variable region and a light chain variable region of an antibody that binds to misfolded TTR. The anti-MerTK-anti-TTR bispecific antibody of the present invention contains the following heavy chain variable region and light chain variable region of the anti-MerTK antibody:
a heavy chain variable region containing CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
a light chain variable region containing CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6.

In one embodiment, the anti-MerTK-anti-TTR bispecific antibody of the present invention contains a heavy chain variable region and a light chain variable region of the anti-MerTK antibody selected from the group consisting of the following (1) to (6):
(1) a heavy chain fragment consisting of an amino acid sequence from amino acid positions 1 to 217 of SEQ ID NO: 8, and a light chain consisting of the amino acid sequence of SEQ ID NO: 10;
(2) a heavy chain fragment consisting of an amino acid sequence from amino acid positions 1 to 217 of SEQ ID NO: 8, and a light chain consisting of the amino acid sequence of SEQ ID NO: 12;
(3) a heavy chain fragment consisting of an amino acid sequence from amino acid positions 1 to 217 of SEQ ID NO: 8, and a light chain consisting of the amino acid sequence of SEQ ID NO: 14;
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20.

In one embodiment, the anti-MerTK-anti-TTR bispecific antibody of the present invention contains a heavy chain variable region and a light chain variable region of the anti-MerTK antibody selected from the group consisting of the following (4) to (6):
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20.

In one embodiment, the anti-MerTK-anti-TTR bispecific antibody of the present invention contains a heavy chain fragment containing the heavy chain variable region of the anti-MerTK antibody, and a light chain containing the light chain variable region of the anti-MerTK antibody. In one embodiment, the anti-MerTK-anti-TTR bispecific antibody of the present invention contains a one-armed anti-MerTK antibody containing a heavy chain fragment containing the heavy chain variable region and a light chain containing the light chain variable region of the anti-MerTK antibody, and one Fc region consisting of a first Fc polypeptide and a second Fc polypeptide.

In one embodiment, the anti-MerTK-anti-TTR bispecific antibody of the present invention contains a one-armed anti-MerTK antibody containing a heavy chain fragment and a light chain selected from the group consisting of the following (1) to (6):
(1) a heavy chain fragment consisting of an amino acid sequence from amino acid positions 1 to 217 of SEQ ID NO: 8, and a light chain consisting of the amino acid sequence of SEQ ID NO: 10;
(2) a heavy chain fragment consisting of an amino acid sequence from amino acid positions 1 to 217 of SEQ ID NO: 8, and a light chain consisting of the amino acid sequence of SEQ ID NO: 12;
(3) a heavy chain fragment consisting of an amino acid sequence from amino acid positions 1 to 217 of SEQ ID NO: 8, and a light chain consisting of the amino acid sequence of SEQ ID NO: 14;
(4) a heavy chain fragment consisting of an amino acid sequence from amino acid positions 1 to 217 of SEQ ID NO: 8, and a light chain consisting of the amino acid sequence of SEQ ID NO: 16;
(5) a heavy chain fragment consisting of an amino acid sequence from amino acid positions 1 to 217 of SEQ ID NO: 8, and a light chain consisting of the amino acid sequence of SEQ ID NO: 18; and
(6) a heavy chain fragment consisting of an amino acid sequence from amino acid positions 1 to 217 of SEQ ID NO: 8, and a light chain consisting of the amino acid sequence of SEQ ID NO: 20.

In one embodiment, the anti-MerTK-anti-TTR bispecific antibody of the present invention contains a one-armed anti-MerTK antibody containing a heavy chain fragment and a light chain selected from the group consisting of the following (4) to (6):
(4) a heavy chain fragment consisting of an amino acid sequence from amino acid positions 1 to 217 of SEQ ID NO: 8, and a light chain consisting of the amino acid sequence of SEQ ID NO: 16;
(5) a heavy chain fragment consisting of an amino acid sequence from amino acid positions 1 to 217 of SEQ ID NO: 8, and a light chain consisting of the amino acid sequence of SEQ ID NO: 18; and
(6) a heavy chain fragment consisting of an amino acid sequence from amino acid positions 1 to 217 of SEQ ID NO: 8, and a light chain consisting of the amino acid sequence of SEQ ID NO: 20.

In one embodiment, the anti-MerTK-anti-TTR bispecific antibody of the present invention contains the one-armed anti-MerTK antibody, and a scFv or a Fab region containing the heavy chain variable region and the light chain variable region of the anti-TTR antibody that binds to misfolded TTR. In one embodiment, the anti-MerTK-anti-TTR bispecific antibody of the present invention is a bispecific antibody in which the scFv or the Fab region containing the heavy chain variable region and the light chain variable region of the anti-TTR antibody that binds to misfolded TTR is linked via a hinge region to the N terminus of the second Fc polypeptide of the one-armed anti-MerTK antibody.

The anti-MerTK-anti-TTR bispecific antibody of the present invention contains a heavy chain variable region and a light chain variable region of an anti-TTR antibody that binds to misfolded TTR. The anti-TTR antibody is not particularly limited as long as the antibody binds to misfolded TTR. For example, a 371M antibody (International Publication No. WO 2015/115332) can be used.

In one embodiment, the anti-MerTK-anti-TTR bispecific antibody of the present invention contains the following heavy chain variable region and light chain variable region of the anti-TTR antibody that binds to misfolded TTR:
a heavy chain variable region containing CDR1 consisting of the amino acid sequence of SEQ ID NO: 27, CDR2 consisting of the amino acid sequence of SEQ ID NO: 28, and CDR3 consisting of the amino acid sequence of SEQ ID NO: 29; and
a light chain variable region containing CDR1 consisting of the amino acid sequence of SEQ ID NO: 30, CDR2 consisting of the amino acid sequence of SEQ ID NO: 31, and CDR3 consisting of the amino acid sequence of SEQ ID NO: 32.

In one embodiment, the anti-MerTK-anti-TTR bispecific antibody of the present invention contains the following heavy chain variable region and light chain variable region of the anti-TTR antibody that binds to misfolded TTR:
a heavy chain variable region consisting of an amino acid sequence from amino acid positions 1 to 119 of SEQ ID NO: 43, and
a light chain variable region consisting of an amino acid sequence from amino acid positions 135 to 245 of SEQ ID NO: 43.

In one embodiment, the anti-MerTK-anti-TTR bispecific antibody of the present invention contains an antigen binding fragment of the anti-TTR antibody that binds to misfolded TTR. In one embodiment, the antigen binding fragment of the anti-TTR antibody that binds to misfolded TTR, contained in the anti-MerTK-anti-TTR bispecific antibody of the present invention can be scFv, a Fab fragment, a Fab' fragment, a F(ab')₂ fragment, or a VHH antibody. In one embodiment, the anti-MerTK-anti-TTR bispecific antibody of the present invention contains scFv containing the heavy chain variable region and the light chain variable region of the anti-TTR antibody that binds to misfolded TTR (in the present specification, referred to as "anti-TTR-scFv").

In one embodiment, the anti-MerTK-anti-TTR bispecific antibody of the present invention contains anti-TTR-scFv consisting of the amino acid sequence of SEQ ID NO: 43.

In one embodiment, the anti-MerTK-anti-TTR bispecific antibody of the present invention is of Fab-scFv-Fc (kih) type. In one embodiment, the anti-MerTK-anti-TTR bispecific antibody of the present invention is a bispecific antibody in which the C terminus of the anti-TTR-scFv is further linked via a hinge region to the N terminus of the second Fc polypeptide of the one-armed anti-MerTK antibody.

In one embodiment, in the anti-TTR-scFv, a peptide linker that links the heavy chain variable region and the light chain variable region of the anti-TTR antibody has the amino acid sequence of SEQ ID NO: 48. In one embodiment, the peptide linker that links the heavy chain variable region and the light chain variable region of the anti-TTR antibody has an amino acid sequence (Gly-Gly-Gly-Gly-Ser)n. In one embodiment, the peptide linker that links the heavy chain variable region and the light chain variable region of the anti-TTR antibody has the amino acid sequence of SEQ ID NO: 54.

When the anti-MerTK-anti-TTR bispecific antibody of the present invention contains the Fc region, the Fc region in the bispecific antibody may contain a mutation that reduces antibody-dependent cellular cytotoxicity (ADCC), antibody-dependent cellular phagocytosis (ADCP), or complement-dependent cytotoxicity (CDC). In one embodiment, the anti-MerTK-anti-TTR bispecific antibody of the present invention contains an Fc region containing L234A and L235A amino acid mutations (LALA mutations) (wherein positions of the mutations are amino acid positions that are numbered according to the EU index in a human Igγ1 constant region). In one embodiment, the anti-MerTK-anti-TTR bispecific antibody of the present invention contains an Fc region containing a P331G mutation or a P329A mutation (wherein positions of the mutations are amino acid positions that are numbered according to the EU index in a human Igγ1 constant region). In one embodiment, the anti-MerTK-anti-TTR bispecific antibody of the present invention contains an Fc region containing one or more mutations among LALA mutations, a P331G mutation, and a P329A mutation. In one embodiment, the anti-MerTK-anti-TTR bispecific antibody of the present invention contains an Fc region containing LALA mutations and a P331G mutation.

In one embodiment, the anti-MerTK-anti-TTR bispecific antibody of the present invention contains an Fc region consisting of a first Fc polypeptide and a second Fc polypeptide. In one embodiment, the anti-MerTK-anti-TTR bispecific antibody of the present invention contains a first Fc polypeptide and a second Fc polypeptide consisting of an amino acid sequence having at least 90% identity to the amino acid sequence of SEQ ID NO: 39 or an amino acid sequence differing from the amino acid sequence of SEQ ID NO: 39 by one to ten substitutions.

In one embodiment, the anti-MerTK-anti-TTR bispecific antibody of the present invention contains an Fc region containing knobs-into-holes mutations. In one embodiment, the anti-MerTK-anti-TTR bispecific antibody of the present invention contains an Fc region containing one or more mutations among LALA mutations, a P331G mutation, a P329A mutation, and knobs-into-holes mutations.

In one embodiment, the knobs-into-holes mutations contained in the anti-MerTK-anti-TTR bispecific antibody of the present invention are a T366W mutation in one of the Fc polypeptides constituting the Fc region, and T366S, L368A, and Y407V mutations in the other Fc polypeptide constituting the Fc region (wherein positions of the mutations are amino acid positions that are numbered according to the EU index in a human Igγ1 constant region).

In one embodiment, the first polypeptide and the second polypeptide contained in the Fc region of the anti-MerTK-anti-TTR bispecific antibody of the present invention contain a polypeptide selected from the following (1) and (2):
(1) a first Fc polypeptide consisting of the amino acid sequence of SEQ ID NO: 40, and a second Fc polypeptide consisting of the amino acid sequence of SEQ ID NO: 41; and
(2) a first Fc polypeptide consisting of the amino acid sequence of SEQ ID NO: 41, and a second Fc polypeptide consisting of the amino acid sequence of SEQ ID NO: 40.

In one embodiment, the anti-MerTK-anti-TTR bispecific antibody of the present invention may contain a hinge region. The hinge region is not particularly limited as long as it is a sequence that may generally be used by those skilled in the art. In one embodiment, the anti-MerTK-anti-TTR bispecific antibody of the present invention contains a hinge region consisting of the amino acid sequence represented by SEQ ID NO: 42 and/or a hinge region consisting of the amino acid sequence of SEQ ID NO: 70.

Those skilled in the art are capable of readily preparing the full-length Fab-scFv-Fc (kih)-type anti-MerTK-anti-TTR bispecific antibody by combining the sequences of the anti-MerTK antibody, the anti-TTR-scFv, the hinge region, the Fc region, and the like described in the aforementioned section<Anti-MerTK-anti-TTR bispecific antibody of present invention>.

In one embodiment, the anti-MerTK-anti-TTR bispecific antibody of the present invention may be modified post-translationally. The post-translational modification is in accordance with the description of the aforementioned section <Anti-MerTK bispecific antibody of present invention>.

### <Fusion product and complex of anti-MerTK-anti-TTR bispecific antibody of present invention, and cell allowed to express anti-MerTK-anti-TTR bispecific antibody of present invention on the cell surface>

The anti-MerTK-anti-TTR bispecific antibody of the present invention provides a fusion product of the anti-MerTK-anti-TTR bispecific antibody of the present invention linked to a protein (including a polypeptide) other than MerTK and misfolded TTR, and an antibody (including an antigen binding fragment) other than the anti-MerTK antibody and the anti-TTR antibody. The anti-MerTK-anti-TTR bispecific antibody of the present invention also provides a complex of the anti-MerTK-anti-TTR bispecific antibody of the present invention bound to a modifier. The anti-MerTK-anti-TTR bispecific antibody of the present invention further provides a cell (e.g., Tandem CAR) allowed to express the anti-MerTK-anti-TTR bispecific antibody of the invention on surface of the cell (e.g., an immunocompetent cell or an effector cell) (in the present specification, referred to as a "cell allowed to express the anti-MerTK-anti-TTR bispecific antibody of the present invention on the cell surface") (Oncology Reports, 2019, 42: 2183-2195). The fusion product, the complex, and the cell allowed to express the antibody on the cell surface may be implemented in accordance with the description of the aforementioned section <Fusion product of present invention, complex of present invention, and cell allowed to express anti-MerTK antibody or antigen binding fragment thereof of present invention on the cell surface>.

### <Polynucleotide of anti-MerTK-anti-TTR bispecific antibody of present invention>

The present invention also provides a polynucleotide for use in the production of the anti-MerTK-anti-TTR bispecific antibody. Those skilled in the art are capable of implementing it in accordance with the description of the aforementioned sections

### <Polynucleotide of present invention> and <Polynucleotide of anti-MerTK bispecific antibody of present invention>.

In one embodiment, the polynucleotide for use in the production of the anti-MerTK-anti-TTR bispecific antibody of the present invention is a polynucleotide selected from the group consisting of the following (1) to (9):
(1) a polynucleotide containing a nucleotide sequence encoding an anti-MerTK antibody heavy chain variable region consisting of an amino acid sequence from amino acid positions 1 to 119 of SEQ ID NO: 8;
(2) a polynucleotide containing a nucleotide sequence encoding an anti-MerTK antibody light chain variable region consisting of an amino acid sequence from amino acid positions 1 to 109 of SEQ ID NO: 10;
(3) a polynucleotide containing a nucleotide sequence encoding an anti-MerTK antibody light chain variable region consisting of an amino acid sequence from amino acid positions 1 to 109 of SEQ ID NO: 12;
(4) a polynucleotide containing a nucleotide sequence encoding an anti-MerTK antibody light chain variable region consisting of an amino acid sequence from amino acid positions 1 to 109 of SEQ ID NO: 14;
(5) a polynucleotide containing a nucleotide sequence encoding an anti-MerTK antibody light chain variable region consisting of an amino acid sequence from amino acid positions 1 to 110 of SEQ ID NO: 16;
(6) a polynucleotide containing a nucleotide sequence encoding an anti-MerTK antibody light chain variable region consisting of an amino acid sequence from amino acid positions 1 to 110 of SEQ ID NO: 18;
(7) a polynucleotide containing a nucleotide sequence encoding an anti-MerTK antibody light chain variable region consisting of an amino acid sequence from amino acid positions 1 to 110 of SEQ ID NO: 20;
(8) a polynucleotide containing a nucleotide sequence encoding an anti-TTR antibody heavy chain variable region consisting of an amino acid sequence from amino acid positions 1 to 119 of SEQ ID NO: 43; and
(9) a polynucleotide containing a nucleotide sequence encoding an anti-TTR antibody light chain variable region consisting of an amino acid sequence from amino acid positions 135 to 245 of SEQ ID NO: 43.

Those skilled in the art are capable of preparing the polynucleotide described in this section by using a method known in the art on the basis of the nucleotide sequence thereof.

### <Expression vector for anti-MerTK-anti-TTR bispecific antibody of present invention, and production method>

The present invention also provides an expression vector and a production method for use in the production of the anti-MerTK-anti-TTR bispecific antibody of the present invention. Those skilled in the art are capable of implementing the vector and the method in accordance with the description of the aforementioned sections <Polynucleotide of present invention>, <Expression vector of present invention>, <Method for producing anti-MerTK antibody of present invention>, and <Polynucleotide of anti-MerTK bispecific antibody of present invention, expression vector, transformed host cell, and production method>.

### <Host cell transformed with anti-MerTK-anti-TTR bispecific antibody of present invention>

The present invention also provides a transformed host cell for the anti-MerTK-anti-TTR bispecific antibody of the present invention (hereinafter, referred to as a "host cell of the anti-MerTK-anti-TTR bispecific antibody of the present invention"). Those skilled in the art are capable of implementing elements other than the polynucleotide contained in the host cell of the anti-MerTK-anti-TTR bispecific antibody of the present invention in accordance with the description of the aforementioned section <Transformed host cell of present invention>.

In one embodiment, the host cell of the anti-MerTK-anti-TTR bispecific antibody of the present invention contains polynucleotides selected from the group consisting of the following (1) to (3):
(1) a polynucleotide containing a nucleotide sequence encoding an anti-MerTK antibody heavy chain variable region consisting of an amino acid sequence from amino acid positions 1 to 119 of SEQ ID NO: 8, a polynucleotide containing a nucleotide sequence encoding an anti-MerTK antibody light chain variable region consisting of an amino acid sequence from amino acid positions 1 to 110 of SEQ ID NO: 16, a polynucleotide containing a nucleotide sequence encoding an anti-TTR antibody heavy chain variable region consisting of an amino acid sequence from amino acid positions 1 to 119 of SEQ ID NO: 43, and a polynucleotide containing a nucleotide sequence encoding an anti-TTR antibody light chain variable region consisting of an amino acid sequence from amino acid positions 135 to 245 of SEQ ID NO: 43;
(2) a polynucleotide containing a nucleotide sequence encoding an anti-MerTK antibody heavy chain variable region consisting of an amino acid sequence from amino acid positions 1 to 119 of SEQ ID NO: 8, a polynucleotide containing a nucleotide sequence encoding an anti-MerTK antibody light chain variable region consisting of an amino acid sequence from amino acid positions 1 to 110 of SEQ ID NO: 18, a polynucleotide containing a nucleotide sequence encoding an anti-TTR antibody heavy chain variable region consisting of an amino acid sequence from amino acid positions 1 to 119 of SEQ ID NO: 43, and a polynucleotide containing a nucleotide sequence encoding an anti-TTR antibody light chain variable region consisting of an amino acid sequence from amino acid positions 135 to 245 of SEQ ID NO: 43; and
(3) a polynucleotide containing a nucleotide sequence encoding an anti-MerTK antibody heavy chain variable region consisting of an amino acid sequence from amino acid positions 1 to 119 of SEQ ID NO: 8, a polynucleotide containing a nucleotide sequence encoding an anti-MerTK antibody light chain variable region consisting of an amino acid sequence from amino acid positions 1 to 110 of SEQ ID NO: 20, a polynucleotide containing a nucleotide sequence encoding an anti-TTR antibody heavy chain variable region consisting of an amino acid sequence from amino acid positions 1 to 119 of SEQ ID NO: 43, and a polynucleotide containing a nucleotide sequence encoding an anti-TTR antibody light chain variable region consisting of an amino acid sequence from amino acid positions 135 to 245 of SEQ ID NO: 43.

### <Pharmaceutical use of anti-MerTK-anti-TTR bispecific antibody of present invention>

The present invention also provides a pharmaceutical composition containing the anti-MerTK-anti-TTR bispecific antibody of the present invention and a pharmaceutically acceptable excipient (hereinafter, referred to as the "anti-MerTK-anti-TTR bispecific antibody or pharmaceutical composition containing it of the present invention"). The pharmaceutical composition may be implemented in accordance with the description of the aforementioned section <Pharmaceutical use of anti-MerTK bispecific antibody of present invention>.

The anti-MerTK-anti-TTR bispecific antibody or the pharmaceutical composition containing it of the present invention can be used for the prevention and/or the treatment of diseases derived from the accumulation of a misfolded protein.

In one embodiment, the disease to be prevented or treated with the anti-MerTK-anti-TTR bispecific antibody or the pharmaceutical composition containing it of the present invention is a neuropathic disease, a heart disease, an eye disease, or an endocrine/metabolic disease. In one embodiment, the disease to be prevented or treated with the anti-MerTK-anti-TTR bispecific antibody or the pharmaceutical composition containing it of the present invention is amyloidosis (e.g., localized amyloidosis or systemic amyloidosis). In one embodiment, the amyloidosis to be prevented or treated with the anti-MerTK-anti-TTR bispecific antibody or the pharmaceutical composition containing it of the present invention is systemic amyloidosis. In one embodiment, the systemic amyloidosis to be prevented or treated with the anti-MerTK-anti-TTR bispecific antibody or the pharmaceutical composition containing it of the present invention is ATTR-CM and ATTR-PN.

### <Anti-MerTK-anti-IgL bispecific antibody of present invention>

The present invention provides an anti-MerTK-anti-IgL bispecific antibody that binds to MerTK and misfolded IgL.

IgL is a protein with a molecular weight of 25 kDa composed of 210 amino acids. It is produced from plasma cells formed from a kind of leukocyte in the bone marrow. Usually, it forms a heterodimer with an immunoglobulin heavy chain (IgH) also produced from the plasma cells, and is responsible for humoral immunity of the acquired immune system. However, if aberrant plasma cells overproduce misfolded IgL, IgL-derived amyloid causes deposition in multiple organs such as the heart, the kidney, the digestive tract, and the nerve (Drugs, 2023, 83: 203-216). Misfolded IgL is a protein causative of AL amyloidosis, a kind of systemic amyloidosis. The possibility has been suggested that an antibody against misfolded IgL inhibits the deposition of the misfolded IgL and improves functions (Br. J. Haematol., 2020, 189: 228-238). Nonetheless, this antibody, as in a monoclonal anti- TTR IgG1 antibody, activates the phagocytosis of phagocytes via an Fc region and might therefore induce the onset of cytokine release syndrome.

The anti-MerTK-anti-IgL bispecific antibody of the present invention contains a heavy chain variable region and a light chain variable region of an anti-MerTK antibody and a heavy chain variable region and a light chain variable region of an antibody that binds to misfolded IgL. The anti-MerTK-anti-IgL bispecific antibody of the present invention contains the following heavy chain variable region and light chain variable region of the anti-MerTK antibody:
a heavy chain variable region containing CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
a light chain variable region containing CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6.

In one embodiment, the anti-MerTK-anti-IgL bispecific antibody of the present invention contains a heavy chain variable region and a light chain variable region of the anti-MerTK antibody selected from the group consisting of the following (1) to (6):
(1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
(2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
(3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20.

In one embodiment, the anti-MerTK-anti-IgL bispecific antibody of the present invention contains a heavy chain fragment containing the heavy chain variable region of the anti-MerTK antibody, and a light chain containing the light chain variable region of the anti-MerTK antibody. In one embodiment, the anti-MerTK-anti-IgL bispecific antibody of the present invention contains a one-armed anti-MerTK antibody containing a heavy chain fragment containing the heavy chain variable region and a light chain containing the light chain variable region of the anti-MerTK antibody, and one Fc region consisting of a first Fc polypeptide and a second Fc polypeptide.

In one embodiment, the anti-MerTK-anti-IgL bispecific antibody of the present invention contains a one-armed anti-MerTK antibody containing a heavy chain fragment and a light chain selected from the group consisting of the following (1) to (6):
(1) a heavy chain fragment consisting of an amino acid sequence from amino acid positions 1 to 217 of SEQ ID NO: 8, and a light chain consisting of the amino acid sequence of SEQ ID NO: 10;
(2) a heavy chain fragment consisting of an amino acid sequence from amino acid positions 1 to 217 of SEQ ID NO: 8, and a light chain consisting of the amino acid sequence of SEQ ID NO: 12;
(3) a heavy chain fragment consisting of an amino acid sequence from amino acid positions 1 to 217 of SEQ ID NO: 8, and a light chain consisting of the amino acid sequence of SEQ ID NO: 14;
(4) a heavy chain fragment consisting of an amino acid sequence from amino acid positions 1 to 217 of SEQ ID NO: 8, and a light chain consisting of the amino acid sequence of SEQ ID NO: 16;
(5) a heavy chain fragment consisting of an amino acid sequence from amino acid positions 1 to 217 of SEQ ID NO: 8, and a light chain consisting of the amino acid sequence of SEQ ID NO: 18; and
(6) a heavy chain fragment consisting of an amino acid sequence from amino acid positions 1 to 217 of SEQ ID NO: 8, and a light chain consisting of the amino acid sequence of SEQ ID NO: 20.

In one embodiment, the anti-MerTK-anti-IgL bispecific antibody of the present invention contains the one-armed anti-MerTK antibody, and a scFv or a Fab region containing the heavy chain variable region and the light chain variable region of the anti-IgL antibody that binds to misfolded IgL. In one embodiment, the anti-MerTK-anti-IgL bispecific antibody of the present invention is a bispecific antibody in which the scFv or the Fab region containing the heavy chain variable region and the light chain variable region of the anti-IgL antibody that binds to misfolded IgL is linked via a hinge region to the N terminus of the second Fc polypeptide of the one-armed anti-MerTK antibody.

The anti-MerTK-anti-IgL bispecific antibody of the present invention contains a heavy chain variable region and a light chain variable region of an anti-IgL antibody that binds to misfolded IgL. The anti-IgL antibody is not particularly limited as long as the antibody binds to misfolded IgL. For example, a 2A4 antibody (Japanese Patent No. 5730020) can be used.

In one embodiment, the anti-MerTK-anti-IgL bispecific antibody of the present invention contains the following heavy chain variable region and light chain variable region of the anti-IgL antibody that binds to misfolded IgL:
a heavy chain variable region containing CDR1 consisting of an amino acid sequence from amino acid positions 26 to 35 of SEQ ID NO: 57, CDR2 consisting of an amino acid sequence from amino acid positions 50 to 68 of SEQ ID NO: 57, and CDR3 consisting of an amino acid sequence from amino acid positions 101 to 108 of SEQ ID NO: 57; and
a light chain variable region containing CDR1 consisting of an amino acid sequence from amino acid positions 24 to 39 of SEQ ID NO: 59, CDR2 consisting of an amino acid sequence from amino acid positions 55 to 61 of SEQ ID NO: 59, and CDR3 consisting of an amino acid sequence from amino acid positions 94 to 103 of SEQ ID NO: 59.

In one embodiment, the anti-MerTK-anti-IgL bispecific antibody of the present invention contains the following heavy chain variable region and light chain variable region of the anti-IgL antibody that binds to misfolded IgL:
a heavy chain variable region consisting of an amino acid sequence from amino acid positions 1 to 119 of SEQ ID NO: 57, and
a light chain variable region consisting of an amino acid sequence from amino acid positions 1 to 112 of SEQ ID NO: 59.

In one embodiment, the anti-MerTK-anti-IgL bispecific antibody of the present invention contains an antigen binding fragment of the anti-IgL antibody that binds to misfolded IgL. In one embodiment, the antigen binding fragment of the anti-IgL antibody that binds to misfolded IgL, contained in the anti-MerTK-anti-IgL bispecific antibody of the present invention can be a scFv, a Fab fragment, a Fab' fragment, a F(ab')₂ fragment, or a VHH antibody. In one embodiment, the anti-MerTK-anti-IgL bispecific antibody of the present invention contains a Fab fragment containing the heavy chain variable region and the light chain variable region of the anti-IgL antibody that binds to misfolded IgL (in the present specification, referred to as "anti-IgL-Fab").

In one embodiment, the anti-MerTK-anti-IgL bispecific antibody of the present invention is of CrossMab (IgG-kih) type. In one embodiment, the anti-MerTK-anti-IgL bispecific antibody of the present invention is a bispecific antibody in which the C terminus of the anti-IgL-Fab is further linked via a hinge region to the N terminus of the second Fc polypeptide of the one-armed anti-MerTK antibody.

In one embodiment, the anti-MerTK-anti-IgL bispecific antibody of the present invention contains anti-IgL-Fab containing a fragment that consists of a heavy chain variable region consisting of an amino acid sequence from amino acid positions 1 to 226 of SEQ ID NO: 57 and a light chain constant region, and a fragment that consists of a light chain variable region consisting of SEQ ID NO: 59 and a CH1 domain.

When the anti-MerTK-anti-IgL bispecific antibody of the present invention contains the Fc region, the Fc region in the bispecific antibody may contain a mutation that reduces ADCC, CDC, or ADCP and/or a mutation that promotes the formation of a heterodimer. The mutation is in accordance with the description of the aforementioned section <Anti-MerTK-anti-TTR bispecific antibody of present invention>.

In one embodiment, the anti-MerTK-anti-IgL bispecific antibody of the present invention contains an Fc region consisting of a first Fc polypeptide and a second Fc polypeptide. In one embodiment, the anti-MerTK-anti-IgL bispecific antibody of the present invention contains a first Fc polypeptide and a second Fc polypeptide consisting of an amino acid sequence having at least 90% identity to the amino acid sequence of SEQ ID NO: 39 or an amino acid sequence differing from the amino acid sequence of SEQ ID NO: 39 by one to ten substitutions.

In one embodiment, the anti-MerTK-anti-IgL bispecific antibody of the present invention contains an Fc region containing knobs-into-holes mutations. In one embodiment, the anti-MerTK-anti-IgL bispecific antibody of the present invention contains an Fc region containing one or more mutations among LALA mutations, a P331G mutation, a P329A mutation, and knobs-into-holes mutations. The knobs-into-holes mutations are in accordance with the description of the aforementioned section <Anti-MerTK-anti-TTR bispecific antibody of present invention>.

In one embodiment, the first polypeptide and the second polypeptide contained in the Fc region of the anti-MerTK-anti-IgL bispecific antibody of the present invention contain a polypeptide selected from the following (1) and (2):
(1) a first Fc polypeptide consisting of the amino acid sequence of SEQ ID NO: 40, and a second Fc polypeptide consisting of the amino acid sequence of SEQ ID NO: 41; and
(2) a first Fc polypeptide consisting of the amino acid sequence of SEQ ID NO: 41, and a second Fc polypeptide consisting of the amino acid sequence of SEQ ID NO: 40.

In one embodiment, the anti-MerTK-anti-IgL bispecific antibody of the present invention may contain a hinge region. In one embodiment, the anti-MerTK-anti-IgL bispecific antibody of the present invention contains a hinge region consisting of the amino acid sequence of SEQ ID NO: 42.

Those skilled in the art are capable of readily preparing the full-length anti-MerTK-anti-IgL bispecific antibody (e.g., a CrossMab (IgG-kih)-type anti-MerTK-anti-IgL bispecific antibody) by combining the sequences of the anti-MerTK antibody, the anti-IgL-Fab, the hinge region, the Fc region, and the like described in the aforementioned section <Anti-MerTK-anti-IgL bispecific antibody of present invention>.

In one embodiment, the anti-MerTK-anti-IgL bispecific antibody of the present invention may be modified post-translationally. The post-translational modification is in accordance with the description of the aforementioned section <Anti-MerTK bispecific antibody of present invention>.

### <Fusion product and complex of anti-MerTK-anti-IgL bispecific antibody of present invention, and cell allowed to express anti-MerTK-anti-IgL bispecific antibody of present invention on the cell surface>

The anti-MerTK-anti-IgL bispecific antibody of the present invention provides a fusion product of the anti-MerTK-anti-IgL bispecific antibody of the present invention linked to a protein (including a polypeptide) other than MerTK and misfolded IgL, and an antibody (including an antigen binding fragment) other than the anti-MerTK antibody and the anti-IgL antibody. The anti-MerTK-anti-IgL bispecific antibody of the present invention also provides a complex of the anti-MerTK-anti-IgL bispecific antibody of the present invention bound to a modifier. The anti-MerTK-anti-IgL bispecific antibody of the present invention further provides a cell (e.g., Tandem CAR) allowed to express the anti-MerTK-anti-IgL bispecific antibody of the invention on surface of the cell (e.g., an immunocompetent cell or an effector cell) (in the present specification, referred to as a "cell allowed to express the anti-MerTK-anti-IgL bispecific antibody of the present invention on the cell surface") (Oncology Reports, 2019, 42: 2183-2195). The fusion product, the complex, and the cell allowed to express the antibody on the cell surface may be implemented in accordance with the description of the aforementioned section <Fusion product of present invention, complex of present invention, and cell allowed to express anti-MerTK antibody or antigen binding fragment thereof of present invention on the cell surface>.

### <Polynucleotide of anti-MerTK-anti-IgL bispecific antibody of present invention>

The present invention also provides a polynucleotide for use in the production of the anti-MerTK-anti-IgL bispecific antibody. Those skilled in the art are capable of implementing it in accordance with the description of the aforementioned sections

### <Polynucleotide of present invention> and <Polynucleotide of anti-MerTK bispecific antibody of present invention>.

In one embodiment, the polynucleotide for use in the production of the anti-MerTK-anti-IgL bispecific antibody of the present invention is a polynucleotide selected from the group consisting of the following (1) to (9):
(1) a polynucleotide containing a nucleotide sequence encoding an anti-MerTK antibody heavy chain variable region consisting of an amino acid sequence from amino acid positions 1 to 119 of SEQ ID NO: 8;
(2) a polynucleotide containing a nucleotide sequence encoding an anti-MerTK antibody light chain variable region consisting of an amino acid sequence from amino acid positions 1 to 109 of SEQ ID NO: 10;
(3) a polynucleotide containing a nucleotide sequence encoding an anti-MerTK antibody light chain variable region consisting of an amino acid sequence from amino acid positions 1 to 109 of SEQ ID NO: 12;
(4) a polynucleotide containing a nucleotide sequence encoding an anti-MerTK antibody light chain variable region consisting of an amino acid sequence from amino acid positions 1 to 109 of SEQ ID NO: 14;
(5) a polynucleotide containing a nucleotide sequence encoding an anti-MerTK antibody light chain variable region consisting of an amino acid sequence from amino acid positions 1 to 110 of SEQ ID NO: 16;
(6) a polynucleotide containing a nucleotide sequence encoding an anti-MerTK antibody light chain variable region consisting of an amino acid sequence from amino acid positions 1 to 110 of SEQ ID NO: 18;
(7) a polynucleotide containing a nucleotide sequence encoding an anti-MerTK antibody light chain variable region consisting of an amino acid sequence from amino acid positions 1 to 110 of SEQ ID NO: 20;
(8) a polynucleotide containing a nucleotide sequence encoding an anti-IgL antibody heavy chain variable region consisting of an amino acid sequence from amino acid positions 1 to 119 of SEQ ID NO: 57; and
(9) a polynucleotide containing a nucleotide sequence encoding an anti-IgL antibody light chain variable region consisting of an amino acid sequence from amino acid positions 1 to 112 of SEQ ID NO: 59.

Those skilled in the art are capable of preparing the polynucleotide described in this section by using a method known in the art on the basis of the nucleotide sequence thereof.

### <Expression vector for anti-MerTK-anti-IgL bispecific antibody of present invention, and production method>

The present invention also provides an expression vector and a production method for use in the production of the anti-MerTK-anti-IgL bispecific antibody of the present invention. Those skilled in the art are capable of implementing the vector and the method in accordance with the description of the aforementioned sections <Polynucleotide of present invention>, <Expression vector of present invention>, <Method for producing anti-MerTK antibody of present invention>, and <Polynucleotide of anti-MerTK bispecific antibody of present invention, expression vector, transformed host cell, and production method>.

### <Host cell transformed with anti-MerTK-anti-IgL bispecific antibody of present invention>

The present invention also provides a transformed host cell for the anti-MerTK-anti-IgL bispecific antibody of the present invention (hereinafter, referred to as a "host cell of the anti-MerTK-anti-IgL bispecific antibody of the present invention"). Those skilled in the art are capable of implementing elements other than the polynucleotide contained in the host cell of the anti-MerTK-anti-IgL bispecific antibody of the present invention in accordance with the description of the aforementioned section <Transformed host cell of present invention>.

In one embodiment, the host cell of the anti-MerTK-anti-IgL bispecific antibody of the present invention contains polynucleotides selected from the group consisting of the following (1) to (4):
(1) a polynucleotide containing a nucleotide sequence encoding an anti-MerTK antibody heavy chain variable region consisting of an amino acid sequence from amino acid positions 1 to 119 of SEQ ID NO: 8, a polynucleotide containing a nucleotide sequence encoding an anti-MerTK antibody light chain variable region consisting of an amino acid sequence from amino acid positions 1 to 109 of SEQ ID NO: 12, a polynucleotide containing a nucleotide sequence encoding an anti-IgL antibody heavy chain variable region consisting of an amino acid sequence from amino acid positions 1 to 119 of SEQ ID NO: 57, and a polynucleotide containing a nucleotide sequence encoding an anti-IgL antibody light chain variable region consisting of an amino acid sequence from amino acid positions 1 to 112 of SEQ ID NO: 59;
(2) a polynucleotide containing a nucleotide sequence encoding an anti-MerTK antibody heavy chain variable region consisting of an amino acid sequence from amino acid positions 1 to 119 of SEQ ID NO: 8, a polynucleotide containing a nucleotide sequence encoding an anti-MerTK antibody light chain variable region consisting of an amino acid sequence from amino acid positions 1 to 110 of SEQ ID NO: 16, a polynucleotide containing a nucleotide sequence encoding an anti-IgL antibody heavy chain variable region consisting of an amino acid sequence from amino acid positions 1 to 119 of SEQ ID NO: 57, and a polynucleotide containing a nucleotide sequence encoding an anti-IgL antibody light chain variable region consisting of an amino acid sequence from amino acid positions 1 to 112 of SEQ ID NO: 59;
(3) a polynucleotide containing a nucleotide sequence encoding an anti-MerTK antibody heavy chain variable region consisting of an amino acid sequence from amino acid positions 1 to 119 of SEQ ID NO: 8, a polynucleotide containing a nucleotide sequence encoding an anti-MerTK antibody light chain variable region consisting of an amino acid sequence from amino acid positions 1 to 110 of SEQ ID NO: 18, a polynucleotide containing a nucleotide sequence encoding an anti-IgL antibody heavy chain variable region consisting of an amino acid sequence from amino acid positions 1 to 119 of SEQ ID NO: 57, and a polynucleotide containing a nucleotide sequence encoding an anti-IgL antibody light chain variable region consisting of an amino acid sequence from amino acid positions 1 to 112 of SEQ ID NO: 59; and
(4) a polynucleotide containing a nucleotide sequence encoding an anti-MerTK antibody heavy chain variable region consisting of an amino acid sequence from amino acid positions 1 to 119 of SEQ ID NO: 8, a polynucleotide containing a nucleotide sequence encoding an anti-MerTK antibody light chain variable region consisting of an amino acid sequence from amino acid positions 1 to 110 of SEQ ID NO: 20, a polynucleotide containing a nucleotide sequence encoding an anti-IgL antibody heavy chain variable region consisting of an amino acid sequence from amino acid positions 1 to 119 of SEQ ID NO: 57, and a polynucleotide containing a nucleotide sequence encoding an anti-IgL antibody light chain variable region consisting of an amino acid sequence from amino acid positions 1 to 112 of SEQ ID NO: 59.

### <Pharmaceutical use of anti-MerTK-anti-IgL bispecific antibody of present invention>

The present invention also provides a pharmaceutical composition containing the anti-MerTK-anti-IgL bispecific antibody of the present invention and a pharmaceutically acceptable excipient (hereinafter, referred to as the "anti-MerTK-anti-IgL bispecific antibody or pharmaceutical composition containing it of the present invention"). The pharmaceutical composition may be implemented in accordance with the description of the aforementioned section <Pharmaceutical use of anti-MerTK bispecific antibody of present invention>.

The anti-MerTK-anti-IgL bispecific antibody or the pharmaceutical composition containing it of the present invention can be used for the prevention and/or the treatment of diseases derived from the accumulation of a misfolded protein.

In one embodiment, the disease to be prevented or treated with the anti-MerTK-anti-IgL bispecific antibody or the pharmaceutical composition containing it of the present invention is a kidney disease. In one embodiment, the disease to be prevented or treated with the anti-MerTK-anti-IgL bispecific antibody or the pharmaceutical composition containing it of the present invention is amyloidosis (e.g., localized amyloidosis or systemic amyloidosis). In one embodiment, the amyloidosis to be prevented or treated with the anti-MerTK-anti-IgL bispecific antibody or the pharmaceutical composition containing it of the present invention is systemic amyloidosis. In one embodiment, the systemic amyloidosis to be prevented or treated with the anti-MerTK-anti-IgL bispecific antibody or the pharmaceutical composition containing it of the present invention is AL amyloidosis.

Particular Examples will now be provided for reference in order to gain further understanding of the present invention. However, these are given for illustrative purposes and do not limit the present invention.

### EXAMPLES

### [Example 1: Preparation of anti-MerTK antibody]

### [Example 1-1: Obtainment of anti-MerTK antibody]

### (1) Preparation of MerTK-Fc fusion protein and MerTK-His protein

Each polynucleotide in which a polynucleotide encoding a human MerTK extracellular region (consisting of an amino acid sequence from amino acid positions 21 to 505 of UniProt: Q12866) or a polynucleotide encoding a mouse MerTK extracellular region (consisting of an amino acid sequence from amino acid positions 19 to 497 of UniProt: Q60805) was linked to a polynucleotide encoding an amino acid sequence cleavable with Factor Xa Protease (New England Biolabs, Inc., P8010L), and was inserted into pFUSE-hIgG1-Fc1 vector (InvivoGen, pfuse-hg1fc1) to prepare a vector for expression encoding each of a protein of human MerTK fused with human Fc (hereinafter, referred to as "human MerTK-human Fc fusion protein") and a protein of mouse MerTK fused with human Fc (hereinafter, referred to as "mouse MerTK-human Fc fusion protein"). Further, the polynucleotide encoding the human MerTK extracellular region mentioned above is linked in tandem to a polynucleotide (SEQ ID NO: 21) encoding His tag and inserted into pcDNA3.4 TOPO(R) vector (Thermo Fisher Scientific Inc.) to prepare a vector for expression encoding a protein of human MerTK fused with His tag (hereinafter, referred to as "human MerTK-His protein"). ExpiCHO-S cells (Thermo Fisher Scientific Inc., A29127) were transfected with each of the prepared expression vectors, and recombinant proteins were obtained in accordance with a routine method.

### (2) Obtainment of fully human anti-MerTK agonist antibody

AlivaMab mice (Ablexis, LLC, U.S. Patent No. 9346873) were immunized with several doses of Human MerTK/Mer Protein, His Tag (ACRO Biosystems, MEK-H52H6), and mouse MerTK protein obtained by the cleavage of the mouse MerTK-human Fc fusion protein with Factor Xa Protease and purification. Lymphocytes were recovered from the lymph nodes of the immunized mice in accordance with a routine method. B cells binding to fluorescently labeled human MerTK-His protein and mouse MerTK-human Fc fusion protein were sorted out of the lymphocytes recovered from the mice using a cell sorter (Becton Dickinson and Company, FACSMelody) on the basis of the description of the paper (BMC Biol., 2012, 10: 80). Nucleic acids encoding the heavy chain variable region and the light chain variable region of an antibody extracted from the sorted-out B cells were cloned in accordance with a routine method. From the sequence information, a monoclonal antibody of the antibody was prepared in accordance with a routine method. The prepared antibody was evaluated by using, as indicators, binding activity against human and mouse MerTK recombinant proteins, binding activity against MerTK on mouse bone marrow-derived macrophage cell membrane, the ability to induce MerTK internalization into cells, and the activity of inducing AKT phosphorylation as downstream signals of MerTK. As a result, a fully human anti-MerTK agonist antibody was obtained which had binding activity against human and mouse MerTK recombinant proteins, binding activity against MerTK on mouse bone marrow-derived macrophage cell membrane, the ability to induce MerTK internalization into cells, and the activity of inducing AKT phosphorylation. The sequence of the heavy chain variable region of the anti-MerTK agonist antibody corresponds to amino acid positions 1 to 119 of SEQ ID NO: 8, and the sequence of the light chain variable region corresponds to amino acid positions 1 to 110 of SEQ ID NO: 16.

### (3) Preparation and obtainment of fully human anti-MerTK antibody having introduced Fc mutation

The amino acid sequences of a heavy chain and a light chain were designed on the basis of the amino acid sequences of the heavy chain variable region and the light chain variable region of the fully human anti-MerTK agonist antibody obtained in (2). Specifically, a polypeptide was designed such that the amino acid sequence of a human λ chain constant region was linked to the C-terminal side of the light chain variable region, and a polypeptide was designed such that the amino acid sequence of a human Igγ1 chain constant region was linked to the C-terminal side of the heavy chain variable region. L234A, L235A, and P331G amino acid mutations were further introduced to the heavy chain constant region. In this way, tA3-20 was designed as a fully human anti-MerTK antibody consisting of the heavy chain of SEQ ID NO: 8 and the light chain of SEQ ID NO: 10.

In general, N-linked glycosylation in an antibody variable region might contribute to instable quality in antibody production. Hence, a mutation was introduced to the amino acid sequence of tA3-20 for the purpose of preventing glycosylation that might occur on the frameworks of the light chain variable region of tA3-20. Specifically, a light chain (SEQ ID NO: 12) in which asparagine at position 18 in the framework of SEQ ID NO: 10 was substituted by threonine and a light chain (SEQ ID NO: 14) in which asparagine at position 18 of SEQ ID NO: 10 was substituted by serine were designed as to tA3-20. Light chains of SEQ ID NO: 16, SEQ ID NO: 18, and SEQ ID NO: 20 with asparagine added to the N terminus of the light chains of SEQ ID NO: 10, SEQ ID NO: 12, and SEQ ID NO: 14 were further designed. In this way, a human anti-MerTK antibody consisting of the heavy chain of SEQ ID NO: 8 and the light chain of SEQ ID NO: 12, 14, 16, 18, or 20 was designed. The fully human anti-MerTK antibodies consisting of the heavy chain of SEQ ID NO: 8 and the light chain of SEQ ID NO: 12, 14, 16, 18, or 20 were designated as tA3-20.1, tA3-20.2, tA3-20.3, tA3-20.4, and tA3-20.5, respectively. Table 1 shows information on the amino acid sequences of the heavy chain variable regions and the light chain variable regions of the fully human anti-MerTK antibodies.

**[Table 1]**

| Fully human anti-MerTK antibody | Amino acid sequence of heavy chain variable region | Amino acid sequence of light chain variable region |
|---|---|---|
| tA3-20 | Amino acid positions 1 to 119 of SEQ ID NO: 8 | Amino acid positions 1 to 109 of SEQ ID NO: 10 |
| tA3-20.1 | Amino acid positions 1 to 119 of SEQ ID NO: 8 | Amino acid positions 1 to 109 of SEQ ID NO: 12 |
| tA3-20.2 | Amino acid positions 1 to 119 of SEQ ID NO: 8 | Amino acid positions 1 to 109 of SEQ ID NO: 14 |
| tA3-20.3 | Amino acid positions 1 to 119 of SEQ ID NO: 8 | Amino acid positions 1 to 110 of SEQ ID NO: 16 |
| tA3-20.4 | Amino acid positions 1 to 119 of SEQ ID NO: 8 | Amino acid positions 1 to 110 of SEQ ID NO: 18 |
| tA3-20.5 | Amino acid positions 1 to 119 of SEQ ID NO: 8 | Amino acid positions 1 to 110 of SEQ ID NO: 20 |

The amino acid sequences of CDR1 to CDR3 in SEQ ID NO: 8 are shown in SEQ ID NOs: 1 to 3, respectively, and the amino acid sequences of CDR1 to CDR3 in SEQ ID NO: 10, 12, 14, 16, 18, or 20 are shown in SEQ ID NOs: 4 to 6, respectively.

A mouse-human chimeric anti-MerTK antibody (hereinafter, referred to as "MTK201") was designed in the same manner as in (3) on the basis of the amino acid sequences of the heavy chain variable region of SEQ ID NO: 5 and the light chain variable region of SEQ ID NO: 34 of PTL 4. A mouse-human chimeric anti-MerTK antibody (hereinafter, referred to as "M6") was designed in the same manner as in (3) on the basis of the amino acid sequences of the heavy chain variable region of SEQ ID NO: 49 and the light chain variable region of SEQ ID NO: 50 of International Publication No. WO 2016/106221.

Polynucleotides encoding the heavy chains or the light chains of tA3-20, tA3-20.1, tA3-20.2, tA3-20.3, tA3-20.4, tA3-20.5, MTK201, and M6 were prepared, and each polynucleotide was inserted into pcDNA3.4 TOPO vector in accordance with a routine method to prepare the respective heavy chain expression vectors and light chain expression vectors of tA3-20, tA3-20.1, tA3-20.2, tA3-20.3, tA3-20.4, tA3-20.5, MTK201, or M6 . Table 2 shows the combinations of the polynucleotide sequences encoding the respective heavy chains and light chains of the antibodies tA3-20, tA3-20.1, tA3-20.2, tA3-20.3, tA3-20.4, tA3-20.5, MTK201, and M6. Equal amounts of the heavy chain and light chain expression vectors were introduced to ExpiCHO-S cells using a transfection kit (ExpiFectamine CHO Transfection Kit, Thermo Fisher Scientific Inc., A29129), and the culture was started at 37°C in a 5% CO₂ incubator. On the next day, reagents (ExpiCHO Enhancer and ExpiCHO Feed) attached to the kit were added in accordance with the protocol, and an antibody was secreted into the culture supernatant by the culture.

**[Table 2]**

| Fully human anti-MerTK antibody | Polynucleotide sequence encoding heavy chain | Polynucleotide sequence encoding light chain |
|---|---|---|
| tA3-20 | SEQ ID NO: 7 | SEQ ID NO: 9 |
| tA3-20.1 | SEQ ID NO: 7 | SEQ ID NO: 11 |
| tA3-20.2 | SEQ ID NO: 7 | SEQ ID NO: 13 |
| tA3-20.3 | SEQ ID NO: 7 | SEQ ID NO: 15 |
| tA3-20.4 | SEQ ID NO: 7 | SEQ ID NO: 17 |
| tA3-20.5 | SEQ ID NO: 7 | SEQ ID NO: 19 |
| MTK201 | SEQ ID NO: 23 | SEQ ID NO: 25 |
| M6 | SEQ ID NO: 66 | SEQ ID NO: 68 |

From the obtained culture supernatant, the antibody was purified by an affinity purification method using MabSelect SuRe (Cytiva/Global Life Sciences Technologies Japan K.K., 175438) or a combined use of an affinity purification method using MabSelect SuRe pcc (Cytiva/Global Life Sciences Technologies Japan K.K., 17549102) and an anion exchange chromatography purification method using Sartobind(R)Lab Q100 (Sartorius Stedim Biotech S.A., 93IEXQ42BC-12). The purified antibody was examined for the presence or absence of protein expression and the degree of purification by size-exclusion chromatography using SDS-PAGE or ACQUITY UPLC Protein BEH SEC Column (Waters Corp., 186005225) under reducing and non-reducing conditions.

### [Example 1-2: Evaluation of binding activity of anti-MerTK antibody against human MerTK]

tA3-20, tA3-20.1, and tA3-20.2 were evaluated for their binding activity against human MerTK by ELISA.

The human MerTK-His protein prepared in Example 1-1(1) was prepared to 1 µg/mL with PBS, added at 20 µL per well to a well plate (Clear Flat-Bottom Immuno Nonsterile 384-Well Plate, Thermo Fisher Scientific Inc., 464718), and left standing overnight at 4°C so that the protein was immobilized on the plate. Then, the human MerTK-His protein was removed. A blocking solution was prepared by the addition of 20% (final concentration) Blocking One (Nacalai Tesque, Inc., 03953-95) to TBS containing 0.05% (final concentration) Tween-20 (0.5% Tween-20-containing TBS, prepared by 10-fold dilution of Nippon Gene Co., Ltd., 310-07375; hereinafter, referred to as "TBS-T"), added at 50 µL per well, and left standing for 1 hour for blocking. Then, the washing operation of the wells was performed once with TBS-T. Each of the anti-MerTK antibodies and an anti-chicken egg-white lysozyme antibody (LYS_1c3-m1_h1i, prepared in-house) as an isotype control antibody was serially diluted from 10,000 ng/mL to 0.006 ng/mL. TBS-T containing 5% Blocking One was used in the antibody dilution. 20 µL of each diluted antibody was added to each well and reacted at room temperature for 1 hour. After removal of each antibody, the wells were washed three times with TBS-T, and 15 µL of a secondary antibody (prepared by 4,000-fold dilution of Goat Anti-Human IgG Fc, Multi-Species SP ads-HRP (SouthernBiotech, 2014-05) with TBS-T containing 5% Blocking One) was added to each well and reacted at room temperature for 1 hour. The washing operation of the wells was performed three times with TBS-T, and 15 µL of TMB-Plus Substrate-Chromogen (Dako, S1599) was then added to each well and left standing at room temperature for 5 minutes. The reaction was terminated by the addition of 1 mol/L sulfuric acid (FUJIFILM Wako Pure Chemical Corp., 198-09595), and absorbance was measured at 450 nm and 570 nm using Infinite M200 PRO (Tecan Trading AG). The binding activity of tA3-20.3, tA3-20.4, and tA3-20.5 against human MerTK was evaluated by the same method as above.

As a result of measurement, as shown in Fig. 1, all of tA3-20, tA3-20.1, and tA3-20.2 had concentration-dependent binding activity against human MerTK, and the binding activity was equivalent among them. Also, tA3-20.3, tA3-20.4, and tA3-20.5 were confirmed to have binding activity equivalent to tA3-20 against human MerTK.

### [Example 1-3: Analysis of species cross-reactivity of anti-MerTK agonist antibody tA3-20]

tA3-20 was evaluated for its binding activity against MerTK proteins of various animals (human, mouse, rat, and monkey) by ELISA. Each of the human MerTK-human Fc fusion protein and the mouse MerTK-human Fc fusion protein obtained in Example 1-1, Recombinant Rat MERTK protein (His tag) (Abcam plc, ab267966), and Recombinant Cynomolgus Monkey Mer Fc Chimera Protein, CF (R&D Systems, Inc., 10576-MR-050) was diluted into 1 µg/mL with PBS. The dilution was added at 20 µL per well to a well plate (Clear Flat-Bottom Immuno Nonsterile 384-Well Plate), and the protein was immobilized on the plate overnight at 4°C. On the next day, the plate for immobilization was washed three times with TBS-T (Thermo Fisher Scientific Inc., 28360). Blocking One was added at 100 µL per well, and blocking was performed at room temperature for 1 hour. The plate thus blocked was washed three times with TBS-T. tA3-20 was serially diluted at a 4-fold common ratio from the highest concentration of 20 µg/mL using TBS-T containing 10% Blocking One (hereinafter, referred to as a "10% diluting liquid") and added at 20 µL per well. The plate supplemented with the antibody was incubated at room temperature for 1 hour and then washed three times with TBS-T. HRP-labeled anti-human IgG F(ab')₂ (Abcam plc, ab98535) diluted 10,000-fold with a 10% diluting liquid was added as a secondary antibody at 20 µL per well. After incubation at room temperature for 1 hour, washing was performed three times with TBS-T, and 1-Step Ultra TMB-ELISA Substrate Solution (Thermo Fisher Scientific Inc., 34028) was added at 20 µL per well. After incubation at room temperature for 15 minutes, the reaction was terminated by the addition of 1 mol/L sulfuric acid, and absorbance was measured at 450 nm using SpectraMax (Molecular Devices, LLC).

As a result of measurement, tA3-20 was confirmed to have concentration-dependent binding activity against all of human, mouse, rat, and monkey MerTK proteins. tA3-20.1, tA3-20.2, tA3-20.3, tA3-20.4, and tA3-20.5 having the same CDR sequences as those of tA3-20 are considered to have binding activity against not only human MerTK but all of mouse, rat, and monkey MerTK proteins, as in tA3-20.

### [Example 2: Confirmation of activity of tA3-20 against phagocytosis by human retinal pigment epithelium (RPE) cell]

### [Example 2-1: Confirmation of activity of tA3-20 against phagocytosis of apoptotic cell by human RPE cell]

tA3-20 was evaluated for its activation of the phagocytic clearance function of phagocytes by using, as an indicator, the activation of phagocytosis of apoptotic cells by RPE cells, one of the phagocytes.

### The human RPE cells used were iCell(R) Retinal Pigment Epithelium Cells-

01279 (FUJIFILM Wako Pure Chemical Corp., C1046) which are iPS cell-derived RPE cells. The medium used was MEMα (Thermo Fisher Scientific Inc., 12571-063) supplemented with 25 µg/mL Gentamicin (Thermo Fisher Scientific Inc., 15750-060), 1 × concentration of N-2 supplement (Thermo Fisher Scientific Inc., 17502-048), 1 × concentration of B-27 supplement (Thermo Fisher Scientific Inc., 17504-044), 55 nM hydrocortisone (Merck KGaA, H0888-1G), 0.25 mg/mL taurine (Merck KGaA, T8691-25G), and 14 pg/mL 3,3',5-triiodo-L-thyronine sodium salt (Merck KGaA, T5516-1MG) (all were final concentrations). One vial of cells of iCell(R) Retinal Pigment Epithelium Cells-01279 was evenly inoculated to 96-wells of CellCarrier-96 (PerkinElmer, Inc., 6055300) and cultured at 37°C for 5 weeks under 5% CO₂, and the resultant was used as an iPS-RPE cell culture plate in the subsequent studies. Jurkat cells (ATCC, TIB-152) were prepared to a concentration of 1 × 10⁶ cells/mL with a medium for culture of RPMI 1640 (Merck KGaA, R8758-500ML) supplemented with 10% FBS (Cytiva/Global Life Sciences Technologies Japan K.K., SH30084.03) and 1% penicillin-streptomycin (Merck KGaA, P4458-100ML) (all were final concentrations) (hereinafter, referred to as a "medium for culture"). The Jurkat cells prepared at the concentration were treated with 100 ng/mL (final concentration) SUPERFASLIGAND(R) Protein (soluble) (human), (recombinant) (Enzo Life Sciences Inc., ALX-522-020-3005) at 37°C for 3 hours to induce apoptosis. The apoptotically induced Jurkat cells (hereinafter, referred to as "apoptotic Jurkat cells") were prepared to a concentration of 2 × 10⁶ cells/mL with 20 mM CHES/PBS buffer, pH 9.0 (prepared in-house) and then labeled with 400 ng/mL (final concentration) pHrodo Red, SE (Thermo Fisher Scientific Inc., P36600) at room temperature for 20 minutes. The labeled apoptotic Jurkat cells were prepared to a concentration of 6 × 10⁶ cells/mL using a medium for culture.

The test antibodies used were tA3-20, MTK201, and LYS_1c3-m1_h1i (isotype control). Each of these antibodies was prepared to a concentration of 10 µg/mL using a medium for culture.

These test antibodies and the pHrodo-labeled apoptotic Jurkat cells were each added at 50 µL per well to the iPS-RPE cell culture plate washed once with RPMI 1640. The iPS-RPE cell culture plate was loaded in Incucyte S3 Live Cell Analysis Instrument (Sartorius Japan K.K.) placed in an incubator of 5% CO₂ and 37°C. Image measurement was carried out at 1-hour intervals for 24 hours using a bright field and red laser. The measured images were analyzed using an analysis tool and an algorithm loaded onto Incucyte System software, and red calibrated unit [RCU] × µm²/image was used as an indicator for the amount of phagocytosis.

The results of evaluating the activation of phagocytosis are shown in Fig. 2-1. As a result of analysis, tA3-20 was confirmed to markedly activate phagocytosis because fluorescence intensity elevated with a peak on approximately 16 hours after the start of evaluation. This result indicates that tA3-20 activates the phagocytosis of RPE cells via the binding of MerTK. Furthermore, the activation of phagocytosis by tA3-20 was much higher than that of MTK201. This result suggested that tA3-20 has a strong activating effect on a phagocytic clearance function as compared with MTK201. Owing to the reports suggesting that an anti-MerTK agonist antibody can activate the phagocytic clearance function of phagocytes such as macrophages (PTL 4 and NPL 1), tA3-20 is expected to be capable of activating the phagocytic clearance function of not only RPE cells but other phagocytes (e.g., macrophages).

### [Example 2-2: Confirmation of activity of tA3-20 against phagocytosis of POS by human RPE cell]

tA3-20 was evaluated for its activation of the phagocytic clearance function of RPE cells by using the activation of phagocytosis of pig POS as an indicator.

Pig POS was prepared in accordance with the method of S. Alemedawar et al. (Stem Cell Reports, 2020, 14: 374-389). The prepared POS was prepared to a concentration of 3 ×10⁷ POS/mL with 100 mM sodium bicarbonate (pH 8.2, prepared in-house) and then labeled with 1 µM (final concentration) pHrodo Red, SE with stirring at room temperature for 1 hour. The labeled POS was prepared to a concentration of 6 × 10⁶ POS/mL using a medium for culture.

The test antibodies used were tA3-20, MTK201, M6, and LYS_1c3-m1_h1i (isotype control). Each of these antibodies was prepared to a concentration of 10 µg/mL using a medium for culture.

These test antibodies and the pHrodo-labeled POS were each added at 50 µL per well to the iPS-RPE cell culture plate washed once with DMEM. The iPS-RPE cell culture plate was loaded in Incucyte S3 Live Cell Analysis Instrument placed in an incubator of 5% CO₂ and 37°C. Image measurement was carried out at 1-hour intervals for 60 hours using a bright field and red laser. The measured images were analyzed using an analysis tool and an algorithm loaded onto Incucyte System software, and red calibrated unit [RCU] × µm²/image was calculated and used as an indicator for the amount of phagocytosis.

The results of evaluating the activation of POS phagocytosis are shown in Fig. 2-2. As a result of analysis, tA3-20 also exhibited the activation of phagocytosis for POS. This result suggested that it also has an activating effect on the phagocytic clearance function for POS.

From the results of Example 2-2, tA3-20 was confirmed to strongly activate phagocytosis as compared with MTK201 or M6, suggesting that it has a strong activating effect on a phagocytic clearance function as compared with MTK201 or M6.

### [Example 3: Confirmation of inducing effect on MerTK and AKT phosphorylation in RPE by intravitreally administered tA3-20]

tA3-20 was evaluated using mice for its ability to also act as an agonist against MerTK on RPE *in vivo* by using the activation of an MerTK downstream signaling pathway as an indicator.

### [Example 3-1: Intravitreal administration of test antibody and collection of RPE]

After mydriasis by the ocular instillation of Mydrin(R) P Ophthalmic Solution (Santen Pharmaceutical Co., Ltd.), eight-week-old male C57BL6/J mice (The Jackson Laboratory Japan, Inc.) were systemically anesthetized by the intraperitoneal administration of a three drug-mixed anesthetic (Domitor, Nippon Zenyaku Kogyo Co., Ltd.; Dormicum(R) Injection Solution 10 mg, Maruishi Pharmaceutical Co., Ltd.; Butorphanol(R) 5 mg, Meiji Animal Health Co., Ltd.). Subsequently, local anesthesia was performed by the ocular instillation of Xylocaine(R) Ophthalmic Solution 4% (Sandoz AG). While the fundus of the eye was observed under a microscope (Zeiss OPMI LUMERA 300), 1 µL of a test antibody was intravitreally administered using a microsyringe (Ito Corp., MS-N05) and a 37 G needle (Ito Corp.). The test antibodies (PBS was used as a vehicle) used were tA3-20 (0.1447 mg/mL, 1.447 mg/mL, and 14.47 mg/mL) and LYS_1c3-m1_h1i (isotype control, 14.47 mg/mL). After the completion of intravitreal administration, Cravit(R) Ophthalmic Solution 0.5% (Santen Pharmaceutical Co., Ltd.) was instilled to the recipient eye, to which Tarivid(R) Eye Ointment 0.3% (Santen Pharmaceutical Co., Ltd.) was then applied. Antisedan(R) (Nippon Zenyaku Kogyo Co., Ltd.) was subcutaneously injected for awakening.

The mice were euthanized 1 hour after the intravitreal administration of the test antibody. The eyeballs were excised, and RPE was collected and cryopreserved until use. This experiment was conducted at N = 4/group.

### [Example 3-2: Preparation of sample for SDS-PAGE electrophoresis]

The collected mouse RPE was homogenized with 10 µL of RIPA Buffer (Merck KGaA, R0278) containing Halt Protease and Phosphatase Inhibitor Cocktail, EDTA-free (100×) (Thermo Fisher Scientific Inc., 78441). The prepared homogenate was centrifuged at 9,200 × g for 1 minute, and the supernatant was recovered into a sampling tube. 7.8 µL of the recovered supernatant was mixed with 3 µL of NuPAGE LDS Sample Buffer (4×) (Thermo Fisher Scientific Inc., NP0007) and 1.2 µL of NuPAGE Sample Reducing Agent (10×) (Thermo Fisher Scientific Inc., NP0004), heat-treated at 70°C for 10 minutes, and then used as a sample for SDS-PAGE electrophoresis.

4 µL of the sample for SDS-PAGE electrophoresis was subjected to SDS-PAGE using NuPAGE 4-12% Bis-Tris Gel (Thermo Fisher Scientific Inc., NP0329BOX). Then, the gel was transferred to a PVDF membrane (Thermo Fisher Scientific Inc., IB24002) using iBlot2 Gel Transfer Device (Thermo Fisher Scientific Inc., IB21001). The PVDF membrane after transfer (hereinafter, referred to as a "membrane") was blocked using a blocking reagent (PVDF Blocking Reagent for Can Get Signal, Toyobo Co., Ltd., NYPBR01) at room temperature for 1 hour or longer. Then, the resultant was reacted overnight at 4°C with an anti-phosphorylated MerTK antibody (Merck KGaA, SAB4504621-100UG) or an anti-phosphorylated AKT antibody (Cell Signaling Technology, Inc., #4060) diluted 1,000-fold with Can Get Signal Solution 1 (Toyobo Co., Ltd., NKB-201). The membrane reacted with the antibody was subjected to a washing operation with TBS-T containing 1% BlockAce (MEGMILK SNOW BRAND Co., Ltd., UKB40) (hereinafter, referred to as a "washing buffer") and then reacted at room temperature for 1 hour with an HRP-labeled anti-rabbit antibody (Cytiva/Global Life Sciences Technologies Japan K.K., NA9340V) diluted 10,000-fold with Can Get Signal Solution 2 (Toyobo Co., Ltd., NKB-301). The membrane reacted with the antibody was washed with a washing buffer. The membrane was dipped into ECL Select (Cytiva/Global Life Sciences Technologies Japan K.K., RPN2235) solution, and phosphorylated MerTK or phosphorylated AKT was detected using ChemiDoc Imaging System (Bio-Rad Laboratories, Inc.). In order to detect total MerTK or total AKT in the same membrane as above, the antibody was detached from the membrane after detection using a stripping buffer (Restore PLUS Western Blot Stripping Buffer, Thermo Fisher Scientific Inc., 46430), and blocking was performed at room temperature for 1 hour using a blocking reagent (PVDF Blocking Reagent for Can Get Signal). Then, the resultant was reacted overnight at 4°C with an anti-MerTK antibody (Cell Signaling Technology, Inc., #38102) or an anti-AKT antibody (Cell Signaling Technology, Inc., #4691) diluted 3,000-fold with Can Get Signal Solution 1. The detection of total MerTK or total AKT was carried out by the same method as above using ECL Prime (Cytiva/Global Life Sciences Technologies Japan K.K., RPN2236).

### [Example 3-3: Analysis]

Detected bands were converted into numeric values using Image Lab software version 6.0.0 build 25 (Bio-Rad Laboratories, Inc.) software. The numeric value of each phosphorylated protein band was divided by the numeric value of each total protein band to give a normalized value.

The results about MerTK phosphorylation are shown in Fig. 3-1. tA3-20 exhibited the activity of enhancing MerTK phosphorylation from administration at 0.1447 mg/mL (1 µL/eye) relative to the dose of 14.47 mg/mL (1 µL/eye) of the isotype control antibody LYS_1c3-m1_h1i.

The results about AKT phosphorylation are shown in Fig. 3-2. As in the results about MerTK phosphorylation, tA3-20 exhibited the activity of enhancing AKT phosphorylation from administration at 0.1447 mg/mL (1 µL/eye) relative to the dose of 14.47 mg/mL (1 µL/eye) of LYS_1c3-m1_h1i, and the activity was dose-dependent. tA3-20 was found to have the activity of enhancing the phosphorylation of MerTK expressed on RPE and AKT downstream of MerTK under *in vivo* conditions.

From the results of Example 2 and Example 3, tA3-20 was expected to enhance the phagocytic clearance function of RPE via the MerTK downstream signaling pathway in vivo, suggesting that it is useful for the prevention or the treatment of diseases associated with a decline in phagocytic clearance function of RPE.

### [Example 4: Preparation of mouse-type anti-MerTK-anti-TTR bispecific antibody, mouse-type anti-TTR antibody, and TTR protein]

A mouse-type anti-MerTK-anti-TTR bispecific antibody, a mouse-type anti-TTR antibody, and misfolded recombinant human TTR V30M protein were prepared.

### [Example 4-1: Preparation of recombinant human TTR V30M protein]

Purified recombinant human TTR V30M protein was prepared with reference to the method of Matsubara et al. (Protein Expr. Purif., 2003, 30: 55-61). A gene sequence encoding human TTR V30M (mutant consisting of a mature peptide region of human TTR with valine at position 30 changed to methionine) was inserted into an expression vector pQE-30 (Qiagen N.V., N-Terminus pQE Vector Set, 32915) to obtain pQE30_GOI_02. *E.coli* JM109 Competent Cells (Takara Bio Inc., 9052) were transformed by the introduction of pQE30_GOI_02. 1 mM (final concentration) isopropyl-β-D-thiogalactopyranoside (Nacalai Tesque, Inc., 19742-94) was added to LB liquid medium (prepared in-house), and the liquid culture of the transformed *E. coli* was performed at 37°C for approximately 16 hours. The *E. coli* recovered after culture was lysed using xTractor Buffer Kit (Clontech Laboratories, Inc., 635623). The lysate was centrifuged at 10,000 × g for 10 minutes, and only the supernatant was recovered.

From the recovered supernatant, His-labeled human TTR V30M protein was purified using TALON(R) Metal Affinity Resin (Clontech Laboratories, Inc., 635502) and His Buffer Kit (Cytiva/Global Life Sciences Technologies Japan K.K., 11-0034-00). An eluted fraction containing the recombinant human TTR V30M protein was buffer-replaced with a 20 mM phosphate buffer (pH 7.0, prepared in-house) using Amicon Ultra-4 (Merck KGaA, C7719) or PD-10 Desalting Column (Cytiva/Global Life Sciences Technologies Japan K.K., 17-0851-01) to obtain purified recombinant human TTR V30M protein. Some samples of the purified recombinant human TTR V30M protein for use in administration to mice were further subjected to endotoxin removal using Pierce High Capacity Endotoxin Removal Spin Columns (Thermo Fisher Scientific Inc., 88276) in accordance with the attached manual.

The concentration of the purified recombinant human TTR V30M protein subjected to the buffer replacement and/or endotoxin removal operation was measured using Pierce BCA Protein Assay Kit (Thermo Fisher Scientific Inc., 23225) or NanoDrop A280 Measurement Method (Thermo Fisher Scientific Inc.).

### [Example 4-2: Preparation of misfolded recombinant human TTR V30M protein]

In order to prepare recombinant human TTR V30M protein that underwent misfolding (in the present specification, referred to as "misfolded recombinant human TTR V30M protein"), 200 µL of the purified recombinant human TTR V30M protein (10 mg/mL) was mixed with 800 µL of a 50 mM phosphate buffer (pH 2.7, prepared in-house) and incubated at 37°C for 4 days or longer (hereinafter, referred to as "misfolding treatment") to prepare a misfolding-treated recombinant human TTR V30M protein (in the present specification, referred to as "misTTR") solution. An aliquot of the prepared misTTR solution was used, and a mixture of the misTTR solution and 4 M sodium hydroxide at a ratio of 100:1 (hereinafter, referred to as "neutralized misTTR") was prepared.

In order to confirm that both of misTTR and neutralized misTTR formed misfolding, assay of an amyloid-specific fluorescent dye thioflavin T was conducted by using the purified recombinant human TTR V30M protein before misfolding treatment as a control. They were mixed at a protein:thioflavin T (prepared in-house) ratio of 1:5 and incubated at room temperature for 15 minutes, followed by fluorescent measurement under a condition of excitation/emission: 430/480 nm. As a result, markedly enhanced fluorescence intensity was confirmed for misTTR and neutralized misTTR, indicating that these TTRs formed misfolding. The experiment described above led to the used of misTTR and neutralized misTTR as misfolded TTR in the subsequent studies.

### [Example 4-3: Labeling of misfolded recombinant human TTR V30M protein]

50 µL of neutralized misTTR (2 mg/mL), 950 µL of 100 mM sodium bicarbonate (pH 8.2, prepared in-house), and 5 µL of 10 mM pHrodo were mixed and incubated at 37°C for 15 minutes to prepare pHrodo-labeled misTTR (in the present specification, referred to as "pHrodo-misTTR"). After removal of a supernatant containing an excess of pHrodo by centrifugation, the resultant was suspended in PBS and centrifuged again to remove a supernatant. Precipitated pHrodo-misTTR was resuspended in PBS and prepared to a predetermined concentration.

250 µL of neutralized misTTR (2 mg/mL), 250 µL of 50 mM borate (pH 8.5, Thermo Fisher Scientific Inc., 28341), and 16.75 µL of 10 mM DyLight800 NHS Ester (Thermo Fisher Scientific Inc., 46421) were mixed and incubated at 25°C for 1 hour to prepare DyLight800-labeled misTTR (in the present specification, referred to as "DL800-misTTR"). After removal of a supernatant containing an excess of DyLight800 by centrifugation, the resultant was suspended in PBS and centrifuged again to remove a supernatant. Precipitated DL800-misTTR was resuspended in PBS and prepared to a predetermined concentration.

### [Example 4-4: Preparation of mouse-type anti-MerTK-anti-TTR bispecific antibody]

A bispecific antibody that consisted of a heavy chain consisting of a heavy chain fragment containing the heavy chain variable region of an anti-MerTK antibody, a hinge region, and a mouse first Fc polypeptide, a light chain containing the light chain variable region of an anti-MerTK antibody, and a polypeptide that a mouse second Fc polypeptide linked to anti-TTR-scFv and a hinge region (hereinafter, referred to as a "mouse-type anti-MerTK-anti-TTR bispecific antibody") was prepared by the following method.

### (1) Preparation of vectors encoding heavy chain and light chain of mouse-type anti-MerTK antibody

The amino acid sequences of the heavy chain and the light chain of a mouse-type anti-MerTK antibody were designed. A heavy chain (SEQ ID NO: 34) and a light chain (SEQ ID NO: 36) were designed by linking the heavy chain variable region of tA3-20, a hinge region, and a mouse heavy chain IgG2a constant region (EU index: 215 to 447) and by linking the light chain variable region of tA3-20.3 to a mouse light chain lambda chain constant region (EU index: 108 to 213), respectively. The heavy chain mentioned above was further designed so as to have an amino acid sequence containing (i) LALA mutations (L234A and L235A) in which amino acids at amino acid positions 236 and 237 (EU index: 234 and 235) were each changed from leucine (L) to alanine (A), (ii) electrostatic steering mutations and mutations for stabilizing heterodimeric antibody molecules (mAbs, 2019, 12: 1-12) in which amino acids at amino acid positions 358, 366, 370, 372, 401, and 413 (EU index: 356, 364, 368, 370, 399, and 411) were changed from glutamic acid (E) to lysine (K), from threonine (T) to serine (S), from methionine (M) to leucine (L), from threonine (T) to lysine (K), from aspartic acid (D) to lysine (K), and from arginine (R) to threonine (T), respectively, and (iii) a mutation in which an amino acid at amino acid position 331 (EU index: 329) was changed from proline (P) to alanine (A). Nucleotide sequences (SEQ ID NOs: 33 and 35) encoding the amino acid sequences of the heavy chain and the light chain of the designed mouse-type anti-MerTK antibody were artificially synthesized, and the resulting genes were inserted into pcDNA3.4 TOPO vectors to prepare a vector MHC and a vector MLC.

### (2) Preparation of vector encoding mouse-type anti-TTR-scFv-Fc

A polypeptide of a hinge region and anti-TTR-scFv linked to the N terminus of a mouse second Fc polypeptide (hereinafter, referred to as "mouse-type anti-TTR-scFv-Fc") (SEQ ID NO: 38) was designed so as to consist of an amino acid sequence in which a 371M antibody (anti-TTR antibody that binds to misfolded TTR) heavy chain variable region of SEQ ID NO: 13 of International Publication No. WO 2015/115332, a GS linker (SEQ ID NO: 54), a 371M antibody light chain variable region of SEQ ID NO: 14 of International Publication No. WO 2015/115332, a hinge region, and a mouse heavy chain IgG2a constant region (EU index: 215 to 447) were linked in this order. The mouse-type anti-TTR-scFv-Fc was further designed so as to have an amino acid sequence (SEQ ID NO: 38) containing (i) mutations for the purpose of stabilizing anti-TTR-scFv positioned on the N-terminal side (Protein Engineering, Design and Selection, 2019, 25: 321-329) in which glycine (G) at amino acid position 44 (EU index: 44) of SEQ ID NO: 38 was substituted by cysteine (C), and glycine (G) at amino acid position 237 (EU index: 100) was substituted by cysteine (C), (ii) LALA mutations (L234A and L235A) in which amino acids at amino acid positions 265 and 266 (EU index: 234 and 235) of SEQ ID NO: 38 were each changed from leucine (L) to alanine (A), (iii) electrostatic steering mutations and mutations for stabilizing heterodimeric antibody molecules in which amino acids at amino acid positions 395, 399, 401, 440, 442, and 470 (EU index: 364, 368, 370, 409, 411, and 439) of SEQ ID NO: 38 were changed from threonine (T) to serine (S), from methionine (M) to leucine (L), from threonine (T) to lysine (K), from lysine (K) to glutamic acid (E), from arginine (R) to threonine (T), and from lysine (K) to aspartic acid (D), respectively, and (iv) a mutation in which an amino acid at amino acid position 360 (EU index: 329) of SEQ ID NO: 38 was changed from proline (P) to alanine (A). A nucleotide sequence encoding the amino acid sequence of the designed mouse-type anti-TTR-scFv-Fc was artificially synthesized, and the resulting gene was inserted into pcDNA3.4 TOPO vector to prepare a vector TscFvFc.

### (3) Preparation of mouse-type anti-MerTK-anti-TTR bispecific antibody

A mouse-type anti-MerTK-anti-TTR bispecific antibody was prepared using the vectors prepared in (1) and (2) described above. Specifically, ExpiCHO-S cells were cotransfected with the vector MHC, the vector MLC, and the vector TscFvFc using ExpiFectamine CHO Transfection Kit, and the mouse-type anti-MerTK-anti-TTR bispecific antibody was secreted into a culture supernatant in accordance with a routine method. From the obtained culture supernatant, the antibody was purified by combined use of an affinity purification method using MabSelect SuRe pcc and an ion exchange chromatography purification method using HiTrap SP HP (Cytiva/Global Life Sciences Technologies Japan K.K., 17115201) or a size-exclusion chromatography purification method using Superdex 200 Increase 10/300 GL (Cytiva/Global Life Sciences Technologies Japan K.K., 28990944).

Hereinafter, the prepared mouse-type anti-MerTK-anti-TTR bispecific antibody is referred to as tA-009.

### [Example 4-5: Preparation of mouse-type anti-TTR antibody]

A 371M antibody (in the present specification, referred to as "mouse-type 371M") was prepared as an anti-TTR antibody having human-derived anti-TTR antibody variable regions and mouse-derived constant regions by the following method.

A heavy chain was designed (SEQ ID NO: 44) so as to have an amino acid sequence in which a mouse heavy chain IgG2a constant region (EU index: 118 to 447) was linked to a 371M antibody (anti-TTR antibody) heavy chain variable region of SEQ ID NO: 13 of International Publication No. WO 2015/115332. A light chain was designed (SEQ ID NO: 45) so as to have an amino acid sequence in which a mouse light chain lambda 1 (EU index: 108 to 213) was linked to a 371M antibody light chain variable region of SEQ ID NO: 14 of International Publication No. WO 2015/115332. Nucleotide sequences encoding the amino acid sequences thus designed were artificially synthesized, and the resulting genes were inserted into pcDNA3.4 TOPO vectors. Mouse-type 371M was prepared using the prepared vectors. Specifically, the vectors were introduced to ExpiCHO-S cells using ExpiFectamine CHO Transfection Kit, and the antibody was secreted into a culture supernatant in accordance with a routine method. From the obtained culture supernatant, the antibody was purified by combined use of an affinity purification method using MabSelect SuRe pcc and a size-exclusion chromatography purification method using HiLoad 26/600 superdex(R) 200 pg (Cytiva/Global Life Sciences Technologies Japan K.K., 28-9893-36).

### [Example 5: In vitro activity evaluation of tA-009]

### [Example 5-1: Evaluation of binding activity of tA-009 against human MerTK and misTTR]

tA-009 was evaluated for its binding activity against human MerTK and misTTR by ELISA.

The human MerTK-Fc protein prepared in Example 1-1(1) and the misTTR prepared in Example 4-2 were prepared to 1 µg/mL and 2 µg/mL, respectively, with PBS, added at 20 µL per well to Clear Flat-Bottom Immuno Nonsterile 384-Well Plate, and left standing at room temperature for 1 hour for immobilization onto the plate. Then, the human MerTK-Fc protein and the misTTR were each removed. A blocking solution was prepared by the addition of 20% (final concentration) Blocking One to TBS-T, added at 50 µL per well, and left standing for 1 hour for blocking. Then, the washing operation of the wells was performed once with TBS-T. Each of tA-009 and an anti-keyhole limpet hemocyanin (KLH) antibody (KLH_173A1_ma0, prepared in-house) as an isotype control antibody was serially diluted from 2 × 10⁴ to 1 × 10⁻² ng/mL. TBS-T containing 5% Blocking One was used in the antibody dilution. 15 µL of each diluted antibody was added to each well and reacted at room temperature for 1 hour. After removal of each antibody, the wells were washed three times with TBS-T, and 15 µL of a secondary antibody (prepared by 4,000-fold dilution of Goat Anti-Mouse IgG, Human ads-HRP (SouthernBiotech, 1030-05) with TBS-T containing 5% Blocking One) was then added to each well and reacted at room temperature for 1 hour. The washing operation of the wells was performed three times with TBS-T, and 15 µL of TMB-Plus Substrate-Chromogen was then added to each well and left standing at room temperature for 5 minutes. The reaction was terminated by the addition of 1 mol/L sulfuric acid, and absorbance was measured at 450 nm and 570 nm using Infinite M200 PRO.

As a result of measurement, tA-009 was found to have concentration-dependent binding activity against human MerTK-Fc protein and misTTR (Figs. 4-1 and 4-2).

### [Example 5-2: Activation of phagocytic clearance function of mouse bone marrow-derived macrophage cells by tA-009]

tA-009 was evaluated for phagocytosis of phagocytes by using, as an indicator, the phagocytosis of misTTR by mouse bone marrow-derived macrophage cells, one of the phagocytes.

Mouse bone marrow-derived macrophage cells were prepared by the following method. Myeloid cells were collected from the femur and the tibia of male C57BL6/J mice (The Jackson Laboratory Japan, Inc.) and cultured for 3 days under conditions of 37°C and 5% CO₂ in RPMI-1640 medium (Sigma-Aldrich Co., LLC, R8758-500ML) containing 10% FBS (Cytiva/Global Life Sciences Technologies Japan K.K., SH30070.03) and 40 ng/ml Recombinant Mouse M-CSF Protein (R&D Systems, Inc., 416-ML-010) (hereinafter, referred to as "RPMI-1640 containing FBS, etc."). After subsequent replacement with RPMI-1640 containing FBS, etc. and containing 0.1 µM dexamethasone (Sigma-Aldrich Co., LLC, D4902-100MG), the cells were cultured at a density of 1 × 10⁴ cells/well for 3 days under conditions of 37°C and 5% CO₂ and washed once with RPMI-1640 medium to prepare a mouse bone marrow-derived macrophage cell culture plate. The test antibody used was tA-009, and the control antibody used was tA3-20.3. RPMI-1640 medium containing 5 µg/mL (final concentration) pHrodo-misTTR and 0.7 µmol/L (final concentration) tA-009 or control antibody tA3-20.3 (SEQ ID NO: 8, 16) was added at 50 µL per well to the mouse bone marrow-derived macrophage cell culture plate. The mouse bone marrow-derived macrophage cell culture plate was loaded in Incucyte S3 Live Cell Analysis Instrument placed in an incubator of 5% CO₂ and 37°C. Measurement and analysis were conducted in the same manner as in Example 2.

As a result of analysis, the anti-MerTK-anti-TTR bispecific antibody tA-009 was shown to activate the phagocytosis of misTTR by the mouse bone marrow-derived macrophage cells more markedly than the anti-MerTK antibody tA3-20.3. This result suggested that tA-009 efficiently activates the phagocytic clearance function of the mouse bone marrow-derived macrophage cells for misTTR by putting misTTR and the mouse bone marrow-derived macrophage cells in proximity.

### [Example 6: Activation of phagocytic clearance function of phagocyte for misTTR by tA-009 in mouse in vivo]

tA-009 was evaluated for its ability to activate the phagocytosis of misTTR by phagocytes, by subcutaneously administering pHrodo-misTTR to mice and measuring of the time course of fluorescence intensity by an *in vivo* imaging method. pHrodo-misTTR emits fluorescence when phagocytized by phagocytes. In order to confirm that the activation of phagocytosis by tA-009 was MerTK-dependent, a test was conducted by combined use with an MerTK inhibitor.

A solution in which pHrodo-misTTR and neutralized misTTR prepared in accordance with Example 4-3 were mixed at a protein quantitative ratio of 1:4 was subcutaneously administered in a volume of 40 µL to the back of 13-week-old female BALB/c-nu(nu/nu) mice (The Jackson Laboratory Japan, Inc.). At 1, 6, 12, 24, 48, 72, 120, 168, 240, and 336 hours after administration, the mice were anesthetized with isoflurane (Viatris Inc.), and the amount of phagocytosis of pHrodo-misTTR by accumulated phagocytes was quantified by measuring fluorescence intensity (photons/second, [p/s]) at and around the administration site using IVIS Imaging System (Lumina II; PerkinElmer, Inc.). A cumulative value of the fluorescence intensity values measured at the points in time was calculated as the total amount of phagocytosis ([p/s] h) for the observation period (14 days) to evaluate phagocytosis. The test antibody tA-009 (100 mg/kg) or a control vehicle (PBS) was intraperitoneally administered 1 hour before subcutaneous administration of pHrodo-misTTR. An MerTK inhibitor ONO7475 (10 mg/kg, MedChemExpress, HY-114358) was orally administered once a day a total of six times from day 0 to day 5. On the start date of the test (day 0), ONO7475 was orally administered 0.5 hours before administration of tA-009. This test was conducted at N = 5/group. The calculation of mean and standard error and a significant difference test were carried out for the total amount of phagocytosis of pHrodo-misTTR using GraphPad Prism (GraphPad Software Inc., ver 8.0.2). The comparison between the vehicle group and the tA-009 group and the comparison between the tA-009 group and the tA-009 + ONO7475 group were performed using Student's t-test, and statistical significance was confirmed when a P value was less than 0.05.

tA-009 was evaluated for its MerTK-dependent phagocytosis in the mice *in vivo* by using the fluorescence intensity of pHrodo-misTTR as an indicator. As a result, significant increase in the fluorescence intensity of pHrodo-misTTR was confirmed in the tA-009 administration group compared with the vehicle administration group (Fig. 5). This result indicates that the activation of phagocytosis of pHrodo-misTTR by phagocytes was confirmed in the tA-009 administration group. On the other hand, the activation of phagocytosis by the administration of tA-009 for pHrodo-misTTR was almost completely suppressed by the MerTK inhibitor ONO7475. These results suggested that tA-009 exhibits MerTK-dependent activation of a phagocytic clearance function.

### [Example 7: Activation of phagocytic clearance function of phagocyte for misTTR by tA-009 or mouse-type 371M in senile mouse in vivo]

TTR amyloidosis caused by the deposition of misfolded TTR in organs throughout the body is well known as an age-related disease (Front. Cardiovasc. Med., 2022, 9: 863179). It has also been reported that a decline in phagocytic clearance function of phagocytes such as macrophages and dysfunction thereof due to aging are partly responsible for the onset of various diseases (Immunol. Lett., 2021, 230: 1-10). In order to evaluate whether either MerTK-dependent activation of a phagocytic clearance function or Fcγ receptor-dependent activation of a phagocytic clearance function was effective for the clearance of misfolded TTR under conditions where the phagocytic clearance function declined due to aging, phagocytic clearance activity against DL800-misTTR in senile mice was used as an indicator for evaluation.

### [Example 7-1: Dose finding of tA-009 or mouse-type 371M using young mouse]

In order to compare a phagocytic clearance function for misTTR between tA-009 which exhibited MerTK-dependent activation of the phagocytic clearance function of phagocytes and mouse-type 371M (having Fc from wild-type mouse IgG2a (Immunol Rev., 2015, 268: 25-51)) which exhibited Fcγ receptor-dependent activation, DL800-misTTR was subcutaneously administered to young mice having a normal phagocytic clearance function, and change in the time course of fluorescence intensity was measured by an *in vivo* imaging method. The fluorescence intensity of DL800-misTTR is proportional to its amount of residual one.

A solution in which DL800-misTTR and neutralized misTTR prepared in accordance with Example 4-3 were mixed at a protein quantitative ratio of 1:4 was subcutaneously administered in a volume of 40 µL to the back of 6-week-old (young) female BALB/c-nu(nu/nu) mice (The Jackson Laboratory Japan, Inc.). At 1, 6, 12, 22, 46, 71, 118, 168, 240, and 336 hours after administration, the mice were anesthetized with isoflurane (Viatris Inc.), and the amount of residual misTTR was quantified by measuring fluorescence intensity (photons/second, [p/s]) at and around the administration site using IVIS Imaging System (Lumina II; PerkinElmer, Inc.). A cumulative value of the fluorescence intensity values measured at the points in time was calculated as the total amount of residual misTTR ([p/s] h) for the observation period (14 days) to evaluate a phagocytic clearance function. The test antibody tA-009 (100 mg/kg) or mouse-type 371M (30 mg/kg) or a control vehicle (PBS) was intraperitoneally administered 1 hour before subcutaneous administration of DL800-misTTR. This test was conducted at N = 4/group. The calculation of mean and standard error and a significant difference test were carried out for the total amount of residual DL800-misTTR using GraphPad Prism (GraphPad Software Inc., ver 8.0.2). The comparison between the vehicle group and the test antibody (mouse-type 371M or tA-009) group was performed using Dunnett's test, and statistical significance was confirmed when a P value was less than 0.05.

The results of evaluating tA-009 or mouse-type 371M for the phagocytic clearance function for DL800-misTTR in the mice *in vivo* are shown in Fig. 6-1. As a result, significant and substantially identical decrease in the total amount of residual DL800-misTTR was confirmed in the tA-009 (100 mg/kg) administration group or the mouse-type 371M (30 mg/kg) compared with the vehicle administration group. This result indicates that 100 mg/kg tA-009 and 30 mg/kg mouse-type 371M have a substantially equivalent phagocytic clearance function for DL800-misTTR *in vivo*.

From these results, the subsequent experiments were conducted on the premise that 100 mg/kg tA-009 and 30 mg/kg mouse-type 371M induce a substantially equivalent phagocytic clearance function of phagocytes in young mice that maintain the phagocytic clearance function of phagocytes such as macrophages.

### [Example 7-2: Activation of phagocytic clearance function by tA-009 or mouse-type 371M for DL800-misTTR in senile mouse in vivo]

A decline in phagocytic clearance function of phagocytes such as macrophages due to aging is suggested for senile mice (Clin. Exp. Imunol. 2008, 152 (3): 448-455; and Immunol. Lett. 2021, 230: 1-10). The present inventors hypothesized that a drug that activates the phagocytic clearance function of phagocytes in an MerTK-dependent manner is more useful for the treatment of diseases than a drug that performs Fcγ receptor-dependent activation. A study for evaluating the activation of a phagocytic clearance function was conducted at the doses of tA-009 and mouse-type 371M that exhibited equivalent effect in young mice.

In accordance with the results of Example 7-1, the test antibody tA-009 (100 mg/kg) or mouse-type 371M (30 mg/kg) or a control vehicle (PBS) was intraperitoneally administered to 20-week-old (young) or 103-week-old (senile) male C57BL/6J mice (The Jackson Laboratory Japan, Inc.). Then, a solution in which DL800-misTTR and neutralized misTTR prepared in accordance with Example 4-3 were mixed at a protein quantitative ratio of 1:4 was subcutaneously administered in a volume of 40 µL to the back of the mice mentioned above. At 1, 6, 12, 24, 48, 72, 120 and 168 hours after administration, the mice were anesthetized with isoflurane (Viatris Inc.), and the amount of residual DL800-misTTR was quantified by measuring fluorescence intensity (photons/second, [p/s]) at and around the administration site using IVIS Imaging System (Lumina II; PerkinElmer, Inc.). This test was conducted at N = 5 or 6/group. The calculation of mean and standard error and a significant difference test were carried out for the total amount of residual DL800-misTTR using GraphPad Prism (GraphPad Software Inc., ver 8.0.2). The comparison between the young mouse/vehicle group and the senile mouse/vehicle group was performed using Student's t-test, and the comparison between the senile mouse/vehicle group and the senile mouse/test antibody (mouse-type 371M or tA-009) group was performed using Dunnett's test. Statistical significance was confirmed when each P value was less than 0.05.

The results of evaluating the activation of a phagocytic clearance function by tA-009 and mouse-type 371M for DL800-misTTR in the senile mice *in vivo* are shown in Fig. 6-2. Significant increase in the total amount of residual DL800-misTTR was confirmed in the senile mice compared to the young mice, and the senile mice were confirmed to have a decline in phagocytic clearance function of phagocytes for foreign matter.

In this test using the senile mice, significant decrease in the total amount of residual DL800-misTTR was confirmed in the tA-009 (100 mg/kg) administration group compared to the vehicle administration group. On the other hand, no action was confirmed in the mouse-type 371M (30 mg/kg) administration group.

These results indicate that tA-009 which can activate a phagocytic clearance function in an MerTK-dependent manner can markedly activate the phagocytic clearance function of phagocytes having a decline in phagocytic clearance function due to aging, as compared with mouse-type 371M which can activate the phagocytic clearance function in an Fcγ receptor-dependent manner.

From this study, it was found that the anti-MerTK-anti-TTR bispecific antibody tA-009 which activates a phagocytic clearance function in an MerTK-dependent manner can markedly activate the phagocytic clearance function even in senile mice, as compared with mouse-type 371M which activates the phagocytic clearance function in an Fcγ receptor-dependent manner. This presumably suggests the possibility that the activation of the phagocytic clearance function by the anti-MerTK-anti-misfolded protein bispecific antibody is very useful for age-related diseases caused by the accumulation of a misfolded protein.

### [Example 8: Design of fully human anti-MerTK-anti-TTR bispecific antibody]

### (1) Design of fully human anti-MerTK one-armed antibody

An anti-MerTK-anti-TTR bispecific antibody of a fully human antibody (hereinafter, referred to as a "fully human anti-MerTK-anti-TTR bispecific antibody") was designed with reference to the sequence of the mouse-type anti-MerTK-anti-TTR bispecific antibody obtained in Example 4. First, an anti-MerTK one-armed antibody of a fully human antibody (hereinafter, referred to as a "fully human MerTK one-armed antibody") was designed. An amino acid sequence from amino acid positions 1 to 217 of SEQ ID NO: 8 was used in the heavy chain fragment of the fully human anti-MerTK one-armed antibody. Also, LALA mutations and a P331G amino acid mutation were introduced (SEQ ID NO: 39) to the first or second Fc polypeptide of the fully human anti-MerTK one-armed antibody. Knobs-into-holes mutations were introduced to the first Fc polypeptide and the second Fc polypeptide on the basis of the prior patent literature (International Publication No. WO 1998/050431). Specifically, a sequence (SEQ ID NO: 40) with an amino acid at amino acid position 136 (EU index: 366) of SEQ ID NO: 39 changed from threonine (T) to tryptophan (W) was designed in order to form knobs in one of the first and second Fc polypeptides, and a sequence (SEQ ID NO: 41) with amino acids at amino acid positions 136, 138, and 177 (EU index: 366, 368, and 407) of SEQ ID NO: 39 changed from threonine (T) to serine (S), from L (leucine) to A (alanine), and from tyrosine (Y) to valine (V), respectively, was designed in order to form holes in the other Fc polypeptide. The amino acid sequence of SEQ ID NO: 16, 18, or 20 was used in the light chain of the fully human anti-MerTK one-armed antibody. The fully human anti-MerTK one-armed antibody was designed by combining the heavy chain fragment of the fully human anti-MerTK one-armed antibody, the light chain of the fully human anti-MerTK one-armed antibody, a hinge region (SEQ ID NO: 42), and the first and second Fc polypeptides (SEQ ID NOs: 40 and 41).

### (2) Design of fully human anti-MerTK-anti-TTR bispecific antibody

A fully human anti-MerTK-anti-TTR bispecific antibody was designed such that the amino acid sequence (SEQ ID NO: 43) of anti-TTR-scFv and a hinge region (SEQ ID NO: 70) were linked to the N terminus of the second Fc polypeptide of the fully human anti-MerTK one-armed antibody designed in (1). Table 3 shows the combination of the amino acid sequences of the parts in each designed fully human anti-MerTK-anti-TTR bispecific antibody.

**[Table 3]**

| Heavy chain fragment of fully human anti-MerTK one-armed antibody | Light chain of fully human anti-MerTK one-armed antibody | First or second Fc polypeptide | Anti-TTR-scFv | Hinge region |
|---|---|---|---|---|
| Amino acid positions 1 to 217 of SEQ ID NO: 8 | SEQ ID NO: 16 | SEQ ID NO: 40 or 41 | SEQ ID NO: 43 | SEQ ID NOs: 42 and 70 |
| | SEQ ID NO: 18 | | | |
| | SEQ ID NO: 20 | | | |

The anti-MerTK antibody of the present invention is expected to be useful for the prevention or the treatment of various diseases caused by a decline in phagocytic function.

### [Example 9: Preparation of anti-MerTK-anti-IgL bispecific antibody, anti-IgL antibody, and misfolded IgL protein]

Misfolded IgL protein, an anti-MerTK-anti-IgL bispecific antibody, and an anti-IgL antibody were prepared.

### [Example 9-1: Preparation of recombinant human IgL protein]

With reference to a gene sequence encoding the amino acid sequence of human IgL Vλ6Wil described in the report of J. Wall et al. (Biochemistry, 1999, 38: 14101-14108, Fig. 1), a gene sequence encoding 6 × histidine (SEQ ID NO: 22) was inserted into an expression vector pcDNA3.4 TOPO vector to obtain Vlambda6Wil vector. The Vlambda6Wil vector was introduced to ExpiCHO-S cells using ExpiFectamine CHO Transfection Kit, and a recombinant protein was secreted into a culture supernatant in accordance with a routine method. From the obtained culture supernatant, His-labeled human IgL protein was purified using TALON(R) Metal Affinity Resin (Clontech Laboratories, Inc., 635502) and His Buffer Kit (Cytiva/Global Life Sciences Technologies Japan K.K., 11-0034-00). An eluted fraction containing the recombinant human IgL protein was buffer-replaced with PBS and concentrated using NAP-25 Column (Cytiva/Global Life Sciences Technologies Japan K.K., 17-0852-01) and Amicon Ultra-15 (Merck KGaA, UFC901096) to obtain purified recombinant human IgL protein.

### [Example 9-2: Preparation of misfolded IgL protein]

In order to prepare recombinant human IgL protein that undergone misfolding (in the present specification, referred to as "misfolded IgL protein"), 200 µL of the purified recombinant human IgL protein (1.3 mg/mL) was mixed with 800 µL of a 50 mM phosphate buffer (pH 2.7, prepared in-house) and incubated at 37°C for 4 days or longer to prepare a misfolding-treated recombinant human IgL protein solution.

### [Example 9-3: Preparation of anti-MerTK-anti-IgL bispecific antibody]

A bispecific antibody that consisted of a heavy chain consisting of a heavy chain fragment containing the heavy chain variable region of an anti-MerTK antibody, a hinge region, and a human first Fc polypeptide, a light chain containing the light chain variable region of the anti-MerTK antibody, a heavy chain consisting of a polypeptide in which a heavy chain fragment containing the heavy chain variable region of an anti-IgL antibody, a hinge region, and a human second Fc polypeptide were linked, and a light chain containing the light chain variable region of the anti-IgL antibody (hereinafter, referred to as an "anti-MerTK-anti-IgL bispecific antibody") was prepared by the following method.

### (1) Preparation of vectors encoding heavy chain and light chain of anti-IgL antibody

Vectors encoding the heavy chain and the light chain of an anti-IgL antibody were prepared on the basis of the description of the paper (mAbs, 2016, 8 (6): 1010-1020). A mouse-human chimeric heavy chain (SEQ ID NO: 57) (hereinafter, referred to as an "anti-IgL antibody heavy chain") was designed which consisted of VH and CL of a 2A4 antibody as a mouse-derived anti-IgL antibody described in Japanese Patent No. 5730020, a hinge region, and a human-derived Fc polypeptide having introduced knobs-into-holes mutations, LALA mutations, and P331G amino acid mutation. A mouse-human chimeric light chain (SEQ ID NO: 59) (hereinafter referred to as an "anti-IgL antibody light chain") having VL of the 2A4 antibody and a human-derived CH1 domain was also designed. The nucleotide sequences (SEQ ID NO: 56 and SEQ ID NO: 58) encoding the amino acid sequences of the designed anti-IgL antibody heavy chain, and anti-IgL antibody light chain were artificially synthesized, and the resulting genes were separately inserted into pcDNA3.4 TOPO vectors to prepare a vector encoding the anti-IgL antibody heavy chain and a vector encoding the anti-IgL antibody light chain.

### (2) Preparation of vector encoding heavy chain and light chain of fully human anti-MerTK antibody

The heavy chain (SEQ ID NO: 61) of a fully human anti-MerTK one-armed antibody was designed such that a human MerTK one-armed antibody heavy chain fragment consisting of an amino acid sequence from amino acid positions 1 to 217 of SEQ ID NO: 8, a hinge region (SEQ ID NO: 42), and a first Fc polypeptide (SEQ ID NO: 41) were linked in this order. A nucleotide sequence encoding the amino acid sequence of the designed heavy chain of the fully human anti-MerTK one-armed antibody was artificially synthesized, and the resulting gene was inserted into pcDNA3.4 TOPO vector to prepare a vector encoding the heavy chain of the fully human anti-MerTK antibody.

A nucleotide sequence encoding the light chain (SEQ ID NO: 12) of tA3-20.1 designed in Example 1-1 was artificially synthesized, and the resulting gene was inserted into pcDNA3.4 TOPO vector to prepare a vector encoding the light chain of the fully human anti-MerTK antibody.

### (3) Preparation of anti-MerTK-anti-IgL bispecific antibody

An anti-MerTK-anti-IgL bispecific antibody was prepared using four types of vectors prepared in (1) and (2) described above. Specifically, ExpiCHO-S cells were cotransfected with the vector encoding the anti-IgL antibody heavy chain, the vector encoding the anti-IgL antibody light chain, the vector encoding the heavy chain of the fully human anti-MerTK antibody, and the vector encoding the light chain of the fully human anti-MerTK antibody using ExpiFectamine CHO Transfection Kit, and the anti-MerTK-anti-IgL bispecific antibody was secreted into a culture supernatant in accordance with a routine method. From the obtained culture supernatant, the antibody was purified by use of affinity purification using MabSelect SuRe. Hereinafter, the prepared anti-MerTK-anti-IgL bispecific antibody is referred to as tA3-20_2A4_h1x.

### [Example 9-4: Preparation of anti-IgL antibody]

An anti-IgL antibody having mouse-derived anti-IgL antibody variable regions of SEQ ID NOs: 152 and 154 of the prior patent literature (Japanese Patent No. 5730020) and human-derived constant regions (in the present specification, referred to as "mouse-human chimeric 2A4") was prepared by the following method.

A heavy chain described in SEQ ID NO: 63 and a light chain described in SEQ ID NO: 65 were designed with reference to the sequence of the 2A4 antibody described in Japanese Patent No. 5730020. Nucleotide sequences (SEQ ID NOs: 62 and 64) encoding the two amino acid sequences designed were artificially synthesized, and from the resulting genes, mouse-human chimeric 2A4 was prepared in accordance with a routine method by use of the same method as in Example 1-1.

### [Example 10: Evaluation of binding activity of anti-MerTK-anti-IgL bispecific antibody against human MerTK and misfolded IgL protein]

tA3-20_2A4_h1x was evaluated for its binding activity against human MerTK and misfolded IgL protein by ELISA.

The misfolded IgL protein prepared in Example 9-2 was prepared to 2.5 µg/mL with PBS, added at 50 µL per well to Ni-NTA HisSorb Plates (Qiagen N.V., 35061), and left standing overnight at 4°C so that the protein was immobilized on the plate. Then, after removal of the misfolded IgL protein, the washing operation of the wells was performed three times with PBST. The evaluation antibody used was tA3-20_2A4_h1x, and the control antibodies used were tA3-20.1, mouse-human chimeric 2A4, and LYS_1c3-m1_h1i (isotype control). They were serially diluted from 20 µg/mL to 6.4 ng/mL using PBS containing 0.05% Tween-20 (prepared by 20-fold dilution of Thermo Fisher Scientific Inc., 28352; hereinafter, referred to as "PBST") and containing 0.5% Blocking One, and 50 µL of each dilution was added to each well, and then reacted at room temperature for 1 hour. Then, the washing operation of the wells was performed three times with PBST. The MerTK-Fc fusion protein prepared in Example 1 was biotinylated using Biotin Labeling Kit (Dojindo Laboratories, LK03), then prepared to 2 µg/mL with PBST containing 0.5% Blocking One, and 50 µL of each dilution was added to each well, and then reacted at room temperature for 1 hour. The washing operation of the wells was performed three times with PBST, and 50 µL of Streptavidin-HRP (R&D Systems, Inc., DY998) diluted 200-fold was then added to each well and reacted at room temperature for 1 hour. The washing operation of the wells was performed three times with PBST, and 50 µL of TMB-Plus Substrate-Chromogen was then added to each well and left standing at room temperature for 5 minutes. The reaction was terminated by the addition of 50 µL of 1 mol/L sulfuric acid to each well, and absorbance was measured at 450 nm and 570 nm using Infinite M200 PRO.

As a result, it was found that tA3-20_2A4_h1x can bind to human MerTK and misfolded IgL protein at the same time and its activity is antibody concentration-dependent (Fig. 7).

Phagocytosis by mouse bone marrow-derived macrophages was further confirmed by the same method as in Example 5-2 using misfolded IgL protein labeled with pHrodo by the same method as in Example 4-3. As a result, tA3-20_2A4_h1x was found to activate phagocytosis in an MerTK-dependent manner. This result suggested that tA3-20_2A4_h1x activates the phagocytic clearance function of mouse bone marrow-derived macrophage cells for misfolded IgL protein.

### SEQUENCE LISTING FREE TEXT

SEQ ID NOs: 1 to 3 represent the amino acid sequences of heavy chain CDR1 to CDR3, respectively, of tA3-20 to tA3-20.5, and SEQ ID NOs: 4 to 6 represent the amino acid sequences of light chain CDR1 to CDR3, respectively, of tA3-20 to tA3-20.5. SEQ ID NO: 8 represents the amino acid sequence of the heavy chain of tA3-20 to tA3-20.5, and SEQ ID NO: 7 represents a nucleotide sequence encoding the amino acid sequence represented by SEQ ID NO: 8 of the heavy chain of tA3-20 to tA3-20.5. SEQ ID NO: 10 represents the amino acid sequence of the light chain of tA3-20, and SEQ ID NO: 9 represents a nucleotide sequence encoding the amino acid sequence represented by SEQ ID NO: 10 of the light chain of tA3-20. SEQ ID NO: 12 represents the amino acid sequence of the light chain of tA3-20.1, and SEQ ID NO: 11 represents a nucleotide sequence encoding the amino acid sequence represented by SEQ ID NO: 12 of the light chain of tA3-20.1. SEQ ID NO: 14 represents the amino acid sequence of the light chain of tA3-20.2, and SEQ ID NO: 13 represents a nucleotide sequence encoding the amino acid sequence represented by SEQ ID NO: 14 of the light chain of tA3-20.2. SEQ ID NO: 16 represents the amino acid sequence of the light chain of tA3-20.3, and SEQ ID NO: 15 represents a nucleotide sequence encoding the amino acid sequence represented by SEQ ID NO: 16 of the light chain of tA3-20.3. SEQ ID NO: 18 represents the amino acid sequence of the light chain of tA3-20.4, and SEQ ID NO: 17 represents a nucleotide sequence encoding the amino acid sequence represented by SEQ ID NO: 18 of the light chain of tA3-20.4. SEQ ID NO: 20 represents the amino acid sequence of the light chain of tA3-20.5, and SEQ ID NO: 19 represents a nucleotide sequence encoding the amino acid sequence represented by SEQ ID NO: 20 of the light chain of tA3-20.5. SEQ ID NO: 22 represents the amino acid sequence of a histidine tag, and SEQ ID NO: 21 represents a nucleotide sequence encoding the amino acid sequence of the histidine tag represented by SEQ ID NO: 22. SEQ ID NO: 24 represents the amino acid sequence of the heavy chain of MTK201, and SEQ ID NO: 23 represents a nucleotide sequence encoding the amino acid sequence represented by SEQ ID NO: 24 of the heavy chain of MTK201. SEQ ID NO: 26 represents the amino acid sequence of the light chain of MTK201, and SEQ ID NO: 25 represents a nucleotide sequence encoding the amino acid sequence represented by SEQ ID NO: 26 of the light chain of MTK201. SEQ ID NOs: 27 to 29 represent the amino acid sequences of heavy chain CDR1 to CDR3, respectively, of a 371M antibody, and SEQ ID NOs: 30 to 32 represent the amino acid sequences of light chain CDR1 to CDR3, respectively, of the 371M antibody. SEQ ID NO: 34 represents the amino acid sequence of the heavy chain of a mouse-type anti-MerTK antibody tA3-20.3, and SEQ ID NO: 33 represents a nucleotide sequence encoding the amino acid sequence represented by SEQ ID NO: 34 of the heavy chain of the mouse-type anti-MerTK antibody tA3-20.3. SEQ ID NO: 36 represents the amino acid sequence of the light chain of the mouse-type anti-MerTK antibody tA3-20.3, and SEQ ID NO: 35 represents a nucleotide sequence encoding the amino acid sequence represented by SEQ ID NO: 36 of the light chain of the mouse-type tA3-20.3. SEQ ID NO: 38 represents the amino acid sequence of a polypeptide containing a mouse second Fc polypeptide and anti-TTR-scFv, and SEQ ID NO: 37 represents a nucleotide sequence encoding the polypeptide containing the mouse second Fc polypeptide and anti-TTR-scFv shown in SEQ ID NO: 38. SEQ ID NO: 39 represents the amino acid sequence of the first Fc polypeptide or the second Fc polypeptide of tA3-20 to tA3-20.5. SEQ ID NO: 40 and/or 41 represents the amino acid sequence of the first Fc polypeptide and/or the second Fc polypeptide of an anti-MerTK-anti-TTR bispecific antibody. SEQ ID NO: 42 represents the amino acid sequence of a hinge region that is linked to anti-MerTK-Fab in tA3-20 to tA3-20.5 and the anti-MerTK-anti-TTR bispecific antibody. SEQ ID NO: 43 represents the amino acid sequence of anti-TTR-scFv. SEQ ID NO: 44 represents the amino acid sequence of the heavy chain of a 371M antibody containing the constant region of a mouse heavy chain IgG2a, and SEQ ID NO: 45 represents the amino acid sequence of the light chain of the 371M antibody containing a mouse light chain Lambda 1. SEQ ID NOs: 46 to 55 represent the amino acid sequences of various linkers. SEQ ID NOs: 57 and 59 represent the amino acid sequences of the heavy chain and the light chain, respectively, of a mouse-human chimeric anti-MerTK-anti-IgL bispecific antibody. SEQ ID NOs: 56 and 58 represent nucleotide sequences encoding the amino acid sequences represented by SEQ ID NOs: 57 and 59 of the heavy chain and the light chain, respectively, of the mouse-human chimeric anti-MerTK-anti-IgL bispecific antibody. SEQ ID NO: 61 represents the amino acid sequence of the heavy chain of a human anti-MerTK one-armed antibody. SEQ ID NO: 60 represents a nucleotide sequence encoding the amino acid sequence represented by SEQ ID NO: 61 of the heavy chain of the human anti-MerTK one-armed antibody. SEQ ID NO: 63 represents the amino acid sequence of the heavy chain of mouse-human chimeric 2A4. SEQ ID NO: 62 represents a nucleotide sequence encoding the amino acid sequence represented by SEQ ID NO: 63 of the heavy chain of the mouse-human chimeric 2A4. SEQ ID NO: 65 represents the amino acid sequence of the light chain of the mouse-human chimeric 2A4. SEQ ID NO: 64 represents a nucleotide sequence encoding the amino acid sequence represented by SEQ ID NO: 65 of the light chain of the mouse-human chimeric 2A4. SEQ ID NOs: 67 and 69 represent the amino acid sequences of the heavy chain and the light chain, respectively, of M6. SEQ ID NOs: 66 and 68 represent nucleotide sequences encoding the amino acid sequences represented by SEQ ID NOs: 67 and 69 of the heavy chain and the light chain, respectively, of M6. SEQ ID NO: 70 represents the amino acid sequence of a hinge region that is linked to anti-TTR-scFv in an anti-MerTK-anti-TTR bispecific antibody.

## Claims

1. An anti-MerTK antibody or an antigen binding fragment thereof comprising:
a heavy chain variable region comprising CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
a light chain variable region comprising CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6
.

2. The anti-MerTK antibody or an antigen binding fragment thereof according to claim 1, comprising a heavy chain variable region and a light chain variable region selected from the group consisting of the following (1) to (6):
(1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
(2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
(3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20
.

3. The anti-MerTK antibody according to claim 1 or 2, comprising a heavy chain comprising the heavy chain variable region and a light chain comprising the light chain variable region.

4. The anti-MerTK antibody according to claim 3, comprising L234A and L235A amino acid mutations (LALA mutations) in the heavy chain (wherein positions of the mutations are amino acid positions that are numbered according to the EU index in a human Igγ1 constant region).

5. The anti-MerTK antibody according to claim 3, comprising a P331G mutation in the heavy chain (wherein a position of the mutation is an amino acid position that is numbered according to the EU index in a human Igγ1 constant region).

6. The anti-MerTK antibody according to claim 3, comprising L234A and L235A amino acid mutations (LALA mutations) and a P331G mutation in the heavy chain (wherein positions of the mutations are amino acid positions that are numbered according to the EU index in a human Igγ1 constant region).

7. The anti-MerTK antibody according to claim 6 selected from the group consisting of the following (1) to (6):
(1) an anti-MerTK antibody comprising a heavy chain consisting of the amino acid sequence of SEQ ID NO: 8, and a light chain consisting of the amino acid sequence of SEQ ID NO: 10;
(2) an anti-MerTK antibody comprising a heavy chain consisting of the amino acid sequence of SEQ ID NO: 8, and a light chain consisting of the amino acid sequence of SEQ ID NO: 12;
(3) an anti-MerTK antibody comprising a heavy chain consisting of the amino acid sequence of SEQ ID NO: 8, and a light chain consisting of the amino acid sequence of SEQ ID NO: 14;
(4) an anti-MerTK antibody comprising a heavy chain consisting of the amino acid sequence of SEQ ID NO: 8, and a light chain consisting of the amino acid sequence of SEQ ID NO: 16;
(5) an anti-MerTK antibody comprising a heavy chain consisting of the amino acid sequence of SEQ ID NO: 8, and a light chain consisting of the amino acid sequence of SEQ ID NO: 18; and
(6) an anti-MerTK antibody comprising a heavy chain consisting of the amino acid sequence of SEQ ID NO: 8, and a light chain consisting of the amino acid sequence of SEQ ID NO: 20.

8. The anti-MerTK antibody according to any one of claims 1 to 7 which is an IgG antibody (anti-MerTK IgG antibody).

9. The anti-MerTK antibody or the antigen binding fragment thereof according to claim 1 or 2, wherein the antigen binding fragment is a single-chain variable fragment (scFv), a Fab fragment, a Fab' fragment, or a F(ab')₂ fragment.

10. The anti-MerTK antibody or the antigen binding fragment thereof according to any one of claims 1 to 9, wherein the anti-MerTK antibody or an antigen-binding fragment thereof is modified post-translationally.

11. A fusion product or a complex of the anti-MerTK antibody or the antigen binding fragment thereof according to any one of claims 1 to 10, or a cell allowed to express the anti-MerTK antibody or the antigen binding fragment thereof according to any one of claims 1 to 10 on the cell surface.

12. A polynucleotide comprising a nucleotide sequence encoding a heavy chain variable region or a light chain variable region of an anti-MerTK antibody or an antigen binding fragment thereof, the polynucleotide being selected from the group consisting of the following (1) to (7):
(1) a polynucleotide comprising a nucleotide sequence encoding a heavy chain variable region consisting of an amino acid sequence from amino acid positions 1 to 119 of SEQ ID NO: 8;
(2) a polynucleotide comprising a nucleotide sequence encoding a light chain variable region consisting of an amino acid sequence from amino acid positions 1 to 109 of SEQ ID NO: 10;
(3) a polynucleotide comprising a nucleotide sequence encoding a light chain variable region consisting of an amino acid sequence from amino acid positions 1 to 109 of SEQ ID NO: 12;
(4) a polynucleotide comprising a nucleotide sequence encoding a light chain variable region consisting of an amino acid sequence from amino acid positions 1 to 109 of SEQ ID NO: 14;
(5) a polynucleotide comprising a nucleotide sequence encoding a light chain variable region consisting of an amino acid sequence from amino acid positions 1 to 110 of SEQ ID NO: 16;
(6) a polynucleotide comprising a nucleotide sequence encoding a light chain variable region consisting of an amino acid sequence from amino acid positions 1 to 110 of SEQ ID NO: 18; and
(7) a polynucleotide comprising a nucleotide sequence encoding a light chain variable region consisting of an amino acid sequence from amino acid positions 1 to 110 of SEQ ID NO: 20.

13. A polynucleotide comprising a nucleotide sequence encoding a heavy chain or a light chain of an anti-MerTK antibody, the polynucleotide being selected from the group consisting of the following (1) to (7):
(1) a polynucleotide comprising a nucleotide sequence encoding a heavy chain consisting of the amino acid sequence of SEQ ID NO: 8;
(2) a polynucleotide comprising a nucleotide sequence encoding a light chain consisting of the amino acid sequence of SEQ ID NO: 10;
(3) a polynucleotide comprising a nucleotide sequence encoding a light chain consisting of the amino acid sequence of SEQ ID NO: 12;
(4) a polynucleotide comprising a nucleotide sequence encoding a light chain consisting of the amino acid sequence of SEQ ID NO: 14;
(5) a polynucleotide comprising a nucleotide sequence encoding a light chain consisting of the amino acid sequence of SEQ ID NO: 16;
(6) a polynucleotide comprising a nucleotide sequence encoding a light chain consisting of the amino acid sequence of SEQ ID NO: 18; and
(7) a polynucleotide comprising a nucleotide sequence encoding a light chain consisting of the amino acid sequence of SEQ ID NO: 20.

14. An expression vector comprising the polynucleotide according to claim 12 or 13.

15. A host cell transformed with the expression vector according to claim 14.

16. A host cell comprising a polynucleotide selected from the group consisting of the following (1) to (13):
(1) a polynucleotide comprising a nucleotide sequence encoding a heavy chain variable region consisting of an amino acid sequence from amino acid positions 1 to 119 of SEQ ID NO: 8;
(2) a polynucleotide comprising a nucleotide sequence encoding a light chain variable region consisting of an amino acid sequence from amino acid positions 1 to 109 of SEQ ID NO: 10;
(3) a polynucleotide comprising a nucleotide sequence encoding a light chain variable region consisting of an amino acid sequence from amino acid positions 1 to 109 of SEQ ID NO: 12;
(4) a polynucleotide comprising a nucleotide sequence encoding a light chain variable region consisting of an amino acid sequence from amino acid positions 1 to 109 of SEQ ID NO: 14;
(5) a polynucleotide comprising a nucleotide sequence encoding a light chain variable region consisting of an amino acid sequence from amino acid positions 1 to 110 of SEQ ID NO: 16;
(6) a polynucleotide comprising a nucleotide sequence encoding a light chain variable region consisting of an amino acid sequence from amino acid positions 1 to 110 of SEQ ID NO: 18;
(7) a polynucleotide comprising a nucleotide sequence encoding a light chain variable region consisting of an amino acid sequence from amino acid positions 1 to 110 of SEQ ID NO: 20;
(8) a polynucleotide comprising a nucleotide sequence encoding a heavy chain variable region consisting of an amino acid sequence from amino acid positions 1 to 119 of SEQ ID NO: 8, and a polynucleotide comprising a nucleotide sequence encoding a light chain variable region consisting of an amino acid sequence from amino acid positions 1 to 109 of SEQ ID NO: 10;
(9) a polynucleotide comprising a nucleotide sequence encoding a heavy chain variable region consisting of an amino acid sequence from amino acid positions 1 to 119 of SEQ ID NO: 8, and a polynucleotide comprising a nucleotide sequence encoding a light chain variable region consisting of an amino acid sequence from amino acid positions 1 to 109 of SEQ ID NO: 12;
(10) a polynucleotide comprising a nucleotide sequence encoding a heavy chain variable region consisting of an amino acid sequence from amino acid positions 1 to 119 of SEQ ID NO: 8, and a polynucleotide comprising a nucleotide sequence encoding a light chain variable region consisting of an amino acid sequence from amino acid positions 1 to 109 of SEQ ID NO: 14;
(11) a polynucleotide comprising a nucleotide sequence encoding a heavy chain variable region consisting of an amino acid sequence from amino acid positions 1 to 119 of SEQ ID NO: 8, and a polynucleotide comprising a nucleotide sequence encoding a light chain variable region consisting of an amino acid sequence from amino acid positions 1 to 110 of SEQ ID NO: 16;
(12) a polynucleotide comprising a nucleotide sequence encoding a heavy chain variable region consisting of an amino acid sequence from amino acid positions 1 to 119 of SEQ ID NO: 8, and a polynucleotide comprising a nucleotide sequence encoding a light chain variable region consisting of an amino acid sequence from amino acid positions 1 to 110 of SEQ ID NO: 18; and
(13) a polynucleotide comprising a nucleotide sequence encoding a heavy chain variable region consisting of an amino acid sequence from amino acid positions 1 to 119 of SEQ ID NO: 8, and a polynucleotide comprising a nucleotide sequence encoding a light chain variable region consisting of an amino acid sequence from amino acid positions 1 to 110 of SEQ ID NO: 20.

17. A method for producing an anti-MerTK antibody or an antigen binding fragment thereof, comprising the step of culturing the host cell according to claim 15 or 16 so that the anti-MerTK antibody or the antigen binding fragment thereof is expressed.

18. A pharmaceutical composition comprising the anti-MerTK antibody or the antigen binding fragment thereof according to any one of claims 1 to 10, and a pharmaceutically acceptable excipient.

19. The pharmaceutical composition according to claim 18 for use in the prevention and/or the treatment of an eye disease.

20. The pharmaceutical composition according to claim 19, wherein the eye disease is a retinal disease.

21. The pharmaceutical composition according to claim 20, wherein the retinal disease is a retinal degenerative disease.

22. The pharmaceutical composition according to claim 21, wherein the retinal degenerative disease is retinitis pigmentosa or age-related macular degeneration.

23. The anti-MerTK antibody or the antigen binding fragment thereof according to any one of claims 1 to 10 for use in the prevention and/or the treatment of an eye disease.

24. A method for preventing and/or treating an eye disease, comprising the step of administering a therapeutically effective amount of the anti-MerTK antibody or the antigen binding fragment thereof according to any one of claims 1 to 10 to a subject.

25. Use of the anti-MerTK antibody or the antigen binding fragment thereof according to any one of claims 1 to 10 in the production of a pharmaceutical composition for the prevention and/or the treatment of an eye disease.

26. A bispecific antibody that binds to MerTK and a misfolded protein and/or amyloid, comprising a heavy chain variable region and a light chain variable region of an anti-MerTK antibody, and a heavy chain variable region and a light chain variable region of an antibody that binds to the misfolded protein and/or the amyloid, wherein
the heavy chain variable region of the anti-MerTK antibody comprises CDR1 consisting of the amino acid sequence of SEQ ID NO: 1, CDR2 consisting of the amino acid sequence of SEQ ID NO: 2, and CDR3 consisting of the amino acid sequence of SEQ ID NO: 3, and
the light chain variable region of the anti-MerTK antibody comprises CDR1 consisting of the amino acid sequence of SEQ ID NO: 4, CDR2 consisting of the amino acid sequence of SEQ ID NO: 5, and CDR3 consisting of the amino acid sequence of SEQ ID NO: 6.

27. The bispecific antibody according to claim 26, wherein the heavy chain variable region and the light chain variable region of the anti-MerTK antibody are selected from the group consisting of the following (1) to (6):
(1) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 10;
(2) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 12;
(3) a heavy chain variable region consisting of an amino acid sequence from amino acid positions 1 to 119 of SEQ ID NO: 8, and a light chain variable region consisting of an amino acid sequence from amino acid positions 1 to 109 of SEQ ID NO: 14;
(4) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 16;
(5) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 18; and
(6) a heavy chain variable region consisting of an amino acid sequence from amino acid positions 1 to 119 of SEQ ID NO: 8, and a light chain variable region consisting of an amino acid sequence from amino acid positions 1 to 110 of SEQ ID NO: 20.

28. The bispecific antibody according to claim 27, comprising a one-armed antibody (one-armed anti-MerTK antibody) comprising a heavy chain fragment comprising the heavy chain variable region of the anti-MerTK antibody and a light chain comprising the light chain variable region of the anti-MerTK antibody, and one Fc region consisting of a first Fc polypeptide and a second Fc polypeptide.

29. The bispecific antibody according to claim 28, wherein the heavy chain fragment and the light chain of the one-armed anti-MerTK antibody are selected from the group consisting of the following (1) to (6):
(1) a heavy chain fragment consisting of an amino acid sequence from amino acid positions 1 to 217 of SEQ ID NO: 8, and a light chain consisting of the amino acid sequence of SEQ ID NO: 10;
(2) a heavy chain fragment consisting of an amino acid sequence from amino acid positions 1 to 217 of SEQ ID NO: 8, and a light chain consisting of the amino acid sequence of SEQ ID NO: 12;
(3) a heavy chain fragment consisting of an amino acid sequence from amino acid positions 1 to 217 of SEQ ID NO: 8, and a light chain consisting of the amino acid sequence of SEQ ID NO: 14;
(4) a heavy chain fragment consisting of an amino acid sequence from amino acid positions 1 to 217 of SEQ ID NO: 8, and a light chain consisting of the amino acid sequence of SEQ ID NO: 16;
(5) a heavy chain fragment consisting of an amino acid sequence from amino acid positions 1 to 217 of SEQ ID NO: 8 and a light chain consisting of the amino acid sequence of SEQ ID NO: 18; and
(6) a heavy chain fragment consisting of an amino acid sequence from amino acid positions 1 to 217 of SEQ ID NO: 8, and a light chain consisting of the amino acid sequence of SEQ ID NO: 20.

30. The bispecific antibody according to claim 29, wherein the C terminus of scFv or a Fab region comprising the heavy chain variable region and the light chain variable region of the antibody that binds to the misfolded protein and/or the amyloid is linked via a hinge region to the N terminus of the second Fc polypeptide of the one-armed anti-MerTK antibody.

31. The bispecific antibody according to any one of claims 26 to 30, wherein the antibody that binds to the misfolded protein and/or the amyloid is an anti-TTR antibody that binds to misfolded TTR.

32. The bispecific antibody according to claim 31, wherein
the heavy chain variable region of the anti-TTR antibody comprises CDR1 consisting of the amino acid sequence of SEQ ID NO: 27, CDR2 consisting of the amino acid sequence of SEQ ID NO: 28, and CDR3 consisting of the amino acid sequence of SEQ ID NO: 29, and
the light chain variable region of the anti-TTR antibody comprises CDR1 consisting of the amino acid sequence of SEQ ID NO: 30, CDR2 consisting of the amino acid sequence of SEQ ID NO: 31, and CDR3 consisting of the amino acid sequence of SEQ ID NO: 32.

33. The bispecific antibody according to claim 32, wherein
the heavy chain variable region of the anti-TTR antibody is a heavy chain variable region consisting of an amino acid sequence from amino acid positions 1 to 119 of SEQ ID NO: 43, and
the light chain variable region of the anti-TTR antibody is a light chain variable region consisting of an amino acid sequence from amino acid positions 135 to 245 of SEQ ID NO: 43.

34. The bispecific antibody according to claim 33, comprising the scFv comprising the heavy chain variable region and the light chain variable region of the anti-TTR antibody (anti-TTR-scFv).

35. The bispecific antibody according to claim 34, wherein the anti-TTR-scFv consists of the amino acid sequence of SEQ ID NO: 43.

36. The bispecific antibody according to claim 34 or 35, comprising a one-armed antibody (one-armed anti-MerTK antibody) comprising a heavy chain fragment comprising the heavy chain variable region of the anti-MerTK antibody and a light chain comprising the light chain variable region of the anti-MerTK antibody, and one Fc region consisting of a first Fc polypeptide and a second Fc polypeptide, wherein the C terminus of the anti-TTR-scFv is linked via a hinge region to the N terminus of the second Fc polypeptide of the one-armed anti-MerTK antibody.

37. The bispecific antibody according to any one of claims 28 to 36, comprising an Fc region comprising L234A and L235A amino acid mutations (LALA mutations) and a P331G mutation (wherein positions of the mutations are amino acid positions that are numbered according to the EU index in a human Igγ1 constant region).

38. The bispecific antibody according to claim 37, wherein the first Fc polypeptide and the second Fc polypeptide comprise an amino acid sequence having at least 90% identity to the amino acid sequence of SEQ ID NO: 39 or an amino acid sequence differing from the amino acid sequence of SEQ ID NO: 39 by one to ten substitutions.

39. The bispecific antibody according to claim 38, comprising an Fc region comprising knobs-into-holes mutations.

40. The bispecific antibody according to claim 38, comprising an Fc region comprising LALA mutations, a P331G mutation, and knobs-into-holes mutations.

41. The bispecific antibody according to claim 39 or 40, wherein the knobs-into-holes mutations are a T366W mutation in one of the Fc polypeptides constituting the Fc region, and T366S, L368A, and Y407V mutations in the other Fc polypeptide constituting the Fc region (wherein positions of the mutations are amino acid positions that are numbered according to the EU index in a human Igγ1 constant region).

42. The bispecific antibody according to claim 38, wherein the sequences of the first Fc polypeptide and the second Fc polypeptide are polypeptides consisting of any sequence of the following (1) or (2):
(1) a first Fc polypeptide consisting of the amino acid sequence of SEQ ID NO: 40, and a second Fc polypeptide consisting of the amino acid sequence of SEQ ID NO: 41; or
(2) a first Fc polypeptide consisting of the amino acid sequence of SEQ ID NO: 41, and a second Fc polypeptide consisting of the amino acid sequence of SEQ ID NO: 40.

43. The bispecific antibody according to claim 42, comprising a hinge region consisting of the amino acid sequence represented by SEQ ID NO: 42 and/or a hinge region consisting of the amino acid sequence represented by SEQ ID NO: 70.

44. The bispecific antibody according to any one of claims 26 to 43, wherein the bispecific antibody is modified post-translationally.

45. A fusion product or a complex of the bispecific antibody according to any one of claims 26 to 44, or a cell allowed to express the bispecific antibody according to any one of claims 26 to 44 on the cell surface.

46. A polynucleotide for use in the production of the bispecific antibody according to any one of claims 26 to 44, the polynucleotide being selected from the group consisting of the following (1) to (9):
(1) a polynucleotide comprising a nucleotide sequence encoding a heavy chain variable region of an anti-MerTK antibody, the heavy chain variable region consisting of an amino acid sequence from amino acid positions 1 to 119 of SEQ ID NO: 8;
(2) a polynucleotide comprising a nucleotide sequence encoding a light chain variable region of an anti-MerTK antibody, the light chain variable region consisting of an amino acid sequence from amino acid positions 1 to 109 of SEQ ID NO: 10;
(3) a polynucleotide comprising a nucleotide sequence encoding a light chain variable region of an anti-MerTK antibody, the light chain variable region consisting of an amino acid sequence from amino acid positions 1 to 109 of SEQ ID NO: 12;
(4) a polynucleotide comprising a nucleotide sequence encoding a light chain variable region of an anti-MerTK antibody, the light chain variable region consisting of an amino acid sequence from amino acid positions 1 to 109 of SEQ ID NO: 14;
(5) a polynucleotide comprising a nucleotide sequence encoding a light chain variable region of an anti-MerTK antibody, the light chain variable region consisting of an amino acid sequence from amino acid positions 1 to 110 of SEQ ID NO: 16;
(6) a polynucleotide comprising a nucleotide sequence encoding a light chain variable region of an anti-MerTK antibody, the light chain variable region consisting of an amino acid sequence from amino acid positions 1 to 110 of SEQ ID NO: 18;
(7) a polynucleotide comprising a nucleotide sequence encoding an a light chain variable region of an anti-MerTK antibody, the light chain variable region consisting of an amino acid sequence from amino acid positions 1 to 110 of SEQ ID NO: 20;
(8) a polynucleotide comprising a nucleotide sequence encoding a heavy chain variable region of an anti-TTR antibody, the heavy chain variable region consisting of an amino acid sequence from amino acid positions 1 to 119 of SEQ ID NO: 43; and
(9) a polynucleotide comprising a nucleotide sequence encoding a light chain variable region of an anti-TTR antibody, the light chain variable region consisting of an amino acid sequence from amino acid positions 135 to 245 of SEQ ID NO: 43.

47. An expression vector comprising the polynucleotide according to claim 46.

48. A host cell transformed with the expression vector according to claim 47.

49. A host cell for use in the production of the bispecific antibody according to any one of claims 27 to 44, the host cell comprising polynucleotides selected from the group consisting of the following (1) to (3):
(1) a polynucleotide comprising a nucleotide sequence encoding a heavy chain variable region of an anti-MerTK antibody, the heavy chain variable region consisting of an amino acid sequence from amino acid positions 1 to 119 of SEQ ID NO: 8, a polynucleotide comprising a nucleotide sequence encoding a light chain variable region of an anti-MerTK antibody, the light chain variable region consisting of an amino acid sequence from amino acid positions 1 to 110 of SEQ ID NO: 16, a polynucleotide comprising a nucleotide sequence encoding a heavy chain variable region of an anti-TTR antibody, the heavy chain variable region consisting of an amino acid sequence from amino acid positions 1 to 119 of SEQ ID NO: 43, and a polynucleotide comprising a nucleotide sequence encoding a light chain variable region of an anti-TTR antibody, the light chain variable region consisting of an amino acid sequence from amino acid positions 135 to 245 of SEQ ID NO: 43;
(2) a polynucleotide comprising a nucleotide sequence encoding a heavy chain variable region of an anti-MerTK antibody, the heavy chain variable region consisting of an amino acid sequence from amino acid positions 1 to 119 of SEQ ID NO: 8, a polynucleotide comprising a nucleotide sequence encoding a light chain variable region of an anti-MerTK antibody, the light chain variable region consisting of an amino acid sequence from amino acid positions 1 to 110 of SEQ ID NO: 18, a polynucleotide comprising a nucleotide sequence encoding a heavy chain variable region of an anti-TTR antibody, the heavy chain variable region consisting of an amino acid sequence from amino acid positions 1 to 119 of SEQ ID NO: 43, and a polynucleotide comprising a nucleotide sequence encoding a light chain variable region of an anti-TTR antibody, the light chain variable region consisting of an amino acid sequence from amino acid positions 135 to 245 of SEQ ID NO: 43; and
(3) a polynucleotide comprising a nucleotide sequence encoding a heavy chain variable region of an anti-MerTK antibody, the heavy chain variable region consisting of an amino acid sequence from amino acid positions 1 to 119 of SEQ ID NO: 8, a polynucleotide comprising a nucleotide sequence encoding a light chain variable region of an anti-MerTK antibody, the light chain variable region consisting of an amino acid sequence from amino acid positions 1 to 110 of SEQ ID NO: 20, a polynucleotide comprising a nucleotide sequence encoding a heavy chain variable region of an anti-TTR antibody, the heavy chain variable region consisting of an amino acid sequence from amino acid positions 1 to 119 of SEQ ID NO: 43, and a polynucleotide comprising a nucleotide sequence encoding a light chain variable region of an anti-TTR antibody, the light chain variable region consisting of an amino acid sequence from amino acid positions 135 to 245 of SEQ ID NO: 43.

50. A method for producing a bispecific antibody that binds to MerTK and TTR, comprising the step of culturing the host cell according to claim 48 or 49 so that the bispecific antibody is expressed.

51. A pharmaceutical composition comprising the bispecific antibody according to any one of claims 26 to 44, and a pharmaceutically acceptable excipient.

52. The pharmaceutical composition according to claim 51 for use in the prevention and/or the treatment of diseases derived from the accumulation of a misfolded protein.

53. The pharmaceutical composition according to claim 52, wherein the disease derived from the accumulation of a misfolded protein is amyloidosis.

54. The pharmaceutical composition according to claim 53, wherein the amyloidosis is systemic amyloidosis.

55. The pharmaceutical composition according to claim 54, wherein the systemic amyloidosis is TTR amyloidosis or amyloid polyneuropathy.

56. The bispecific antibody according to any one of claims 26 to 44 for use in the prevention and/or the treatment of diseases derived from the accumulation of a misfolded protein.

57. A method for preventing and/or treating a disease derived from the accumulation of a misfolded protein, comprising the step of administering a therapeutically effective amount of the bispecific antibody according to any one of claims 26 to 44 to a subject.

58. Use of the bispecific antibody according to any one of claims 26 to 44 in the production of a pharmaceutical composition for the prevention and/or the treatment of diseases derived from the accumulation of a misfolded protein.
